# EUROPEAN PATENT APPLICATION

(11) **EP 4 692 092 A1**
(43) Date of publication of application: **11.02.2026**
(21) Application number: 24781365.2
(22) Date of filing: 21.03.2024
(51) Int. Cl.: C07D 493/04, A61K 31/36, A61K 31/5377, A61K 31/4525, A61K 31/496, A61K 31/381, A61K 31/38, A61P 25/28, C07D 337/12

(54) **NOVEL COMPOUNDS WITH MTORC2-INHIBITORY ACTIVITY AND USES THEREOF**

(30) Priority: 24.03.2023 KR 20230038902
(71) Applicant: Aliad Biopharma, Seoul 02455 (KR)
(72) Inventor: SHIN, Hee Joo, Hwaseong-si, Gyeonggi-do 18500 (KR); KIM, Yunhee, Seoul 06280 (KR); HAN, Young Taek, Cheonan-si, Chungcheongnam-do 31165 (KR)
(74) Representative: Gill Jennings & Every LLP
(86) International application number: PCT/KR2024/095625
(87) International publication number: WO 2024/205377

(57) **Abstract**

The present invention relates to novel compounds with mTORC2-inhibitory activity, a preparation method therefor, and a pharmaceutical composition containing same as an active ingredient. The novel compound of Chemical Formula 1 in the present invention selectively inhibits mTORC2 activity and thus can be effectively used for the prevention, alleviation, or treatment of neurodegenerative diseases caused by overactivated mTORC2.

## Description

### TECHNICAL FIELD

The present invention relates to novel compounds having mTORC2-inhibitory activity, a preparation method thereof, and a pharmaceutical composition comprising the same as an active ingredient.

### BACKGROUND

The mTOR complex exists as mTORC1 (mammalian target of rapamycin complex 1), composed of mTOR, mLST8, DEPTOR, Tti1/Tel2, RAPTOR and PRAS40; and mTORC2 (mammalian target of rapamycin complex 2), composed of mTOR, mLST8, DEPTOR, Tti1/Tel2, RICTOR, mSIN1, and PROTOR1/2, and both mTORC1 and mTORC2 share the catalytic mTOR subunit, a serine/threonine protein kinase that belongs to the phosphatidylinositol 3-kinase-related kinase (PIKK) family. These protein complexes function as signal hubs that regulate cell metabolism, growth, proliferation and survival by integrating intracellular and extracellular signals, and mTORC1 and mTORC2 have distinct characteristics in terms of protein complex composition, substrate specificity, and regulatory mechanism, allowing them to elicit specific cellular responses depending on the types of signals received from upstream.

mTORC1 detects the levels of available intracellular energy and nutrients, regulating anabolism to proceed only when energy and nutrients are sufficient. Specifically, when cellular energy and nutrient levels are sufficiently high, mTORC1 phosphorylates 4E-BP1 (eukaryotic translation initiation factor 4E-binding protein 1), thereby activating cap-dependent protein synthesis, and it also activates SREBP (sterol responsive element binding protein) to facilitate de novo lipid synthesis. In addition, mTORC1 phosphorylates ULK1 and TFEB (transcription factor EB) to inhibit autophagy, thereby suppressing catabolism. Consequently, when intracellular energy and nutrient levels are high, mTORC1 promotes anabolism while inhibiting catabolism. Conversely, when energy and nutrient levels are low, it suppresses anabolism and promotes catabolism, maintaining a balance between anabolism and catabolism in accordance with cellular energy and nutrient status.

In contrast to the function of mTORC1, mTORC2 plays a role in leading cells to survive or proliferate, or interact with each other at the appropriate place and time in responding to signals transmitted from outside the cells. Specifically, when the receptors are activated by insulin or growth factors, phosphoinositide 3-kinase (PI3K) is activated, leading to an increase in PIP3 on the cell membrane, and PIP3 then activates mTORC2. Once activated, mTORC2 phosphorylates and activates AGC kinase family enzymes, including AKT (protein kinase B), SGK (serum and glucocorticoid-inducible kinase), PKC (protein kinase C), and the like. These AGC kinases have been shown to regulate cell survival, division, and metabolism. In addition, mTORC2 modulates the cytoskeleton by phosphorylating proteins such as PKCα, PKCδ, Rho, and Rac1, which control actin filament structures, thereby playing a role in determining cell migration and tissue structure.

Once activated, mTORC2 phosphorylates AKT at Ser473, resulting in activation of AKT. Therefore, the level of AKT Ser473 phosphorylation serves as an indicator of mTORC2 activity. Activated AKT then phosphorylates and inhibits TSC2 (Tuberous sclerosis complex 2). TSC is a negative regulator of mTORC1, and TSC blocks the action of Rheb, which is GTPase that directly binds to mTORC1 and activates it. Therefore, when TSC is inhibited, mTORC1 becomes activated. Activated mTORC1 phosphorylates S6K1 (p70S6 Kinase 1) at Thr389, and S6K1 activated by phosphorylation then promotes cap-dependent translation. Consequently, the level of pS6K1 Thr389 phosphorylation serves as an indicator of an mTORC1 activity.

The process of Aβ production from amyloid precursor protein (APP) in neurons primarily occurs during endocytosis, and Arc protein is required for γ-secretase, an enzyme that cleaves APP, to associate with endosomes. Metabotropic glutamate receptor 1/5-dependent long-term depression (LTD) is mediated by the production of the Arc protein within dendritic spines, and mTORC2 is required for this process. Therefore, hyperactivation of mTORC2 in dendritic spines increases Arc protein production, which in turn promotes Aβ production. Consequently, mTORC2 inhibitors can reduce Arc protein synthesis in dendritic spines, thereby suppressing Aβ generation, making them potentially useful for Alzheimer's disease treatment.

Autophagy is a cellular physiological process that clears damaged or dysfunctional proteins and organelles within cells, and mTORC2 inhibits autophagy through the AKT/FoxO3 signaling pathway and mTORC1 activation. It has been found that autophagic activity is decreased in the brain tissue from Alzheimer's disease patients, and as a result, Aβ aggregates and tau tangles generated within neurons are not properly cleared but accumulate. These accumulated protein aggregates further activate the mTOR signaling pathway, ultimately promoting neuronal death. Therefore, mTORC2 inhibitors could potentially prevent neuronal death by clearing accumulated Aβ and tau aggregates in neurons through an enhancement of autophagic activity. Huntington's disease and spinocerebellar ataxia are caused by abnormal CAG trinucleotide sequence expansion in the huntingtin gene and the ataxin-1 gene, respectively. The expanded polyglutamine repeats in huntingtin and ataxin-1 proteins lead to their aggregation in cells, inducing neuronal cell death (Koyuncu et al. (2017) Int. J. Mol. Sci. 18: 1568). Consequently, mTORC2 inhibitors can clear huntingtin and ataxin-1 protein aggregates by promoting autophagy, making them potentially useful for Huntington's disease and spinocerebellar ataxia treatment.

The increase in mTOR signaling and the phosphorylation of S6K, a downstream target of mTORC1, are found to be proportional to the accumulation of Aβ and neurofibrillary tangles (NFTs) in the brains of Alzheimer's disease patients, and pathological accumulation of hyperphosphorylated tau is associated with the activation of mTOR proteins via phosphorylation at S2481 (Li et al. (2005) FEBS J. 272: 4211-4220). In addition, AKT, a kinase that activates mTORC1, is increased in the temporal lobes of Alzheimer's disease patients, and inhibition of mTOR signaling mitigates physiological and behavioral symptoms in animal models of Aβ accumulation. Reducing Aβ levels through pharmacological or genetic interventions is accompanied by decreased activity of the mTOR pathway, and suppression of mTOR signaling has been found to alleviate symptoms in neurodegenerative diseases such as Parkinson's disease (PD) and Huntington's disease (HD) (Lipton & Sahin (2014) Neuron 84: 275-291).

Based on the above-mentioned research findings, the present invention predicts that novel compounds that inhibit the production of Aβ40 and Aβ42 and promote autophagy will serve as therapeutic agents to target protein for neurodegenerative diseases, including Alzheimer's disease.

### DETAILED DESCRIPTION OF THE INVENTION

### Technical Problem

One object of the present invention is to provide a novel compound represented by the following Chemical Formula 1, or a pharmaceutically acceptable salt thereof.

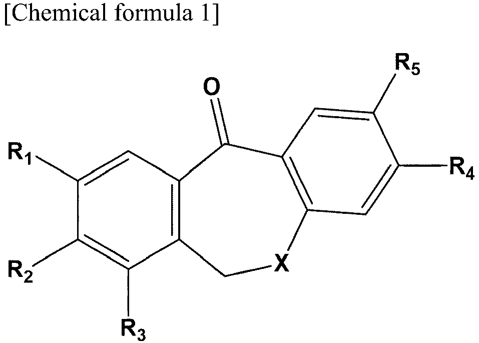

Another object of the present invention is to provide a pharmaceutical composition for prevention or treatment of neurodegenerative diseases, comprising a compound of Chemical Formula 1 or a pharmaceutically acceptable salt thereof as an active ingredient.

Another object of the present invention is to provide a food composition for prevention or improvement of neurodegenerative diseases, comprising a compound of Chemical Formula 1 or a pharmaceutically acceptable salt thereof as an active ingredient.

Yet another object of the present invention is to provide a use for the preparation of a composition for the prevention, improvement, and/or treatment of neurodegenerative diseases, comprising a compound of Chemical Formula 1 or a pharmaceutically acceptable salt thereof as an active ingredient.

Yet another object of the present invention is to provide a method for the prevention, improvement, and/or treatment of neurodegenerative diseases, comprising administering an effective amount of the compound of Chemical Formula 1 or a pharmaceutically acceptable salt thereof to a subject (individual) in need of prevention, improvement, and/or treatment of a neurodegenerative diseases.

Other object of the present invention is to provide a method for preparing the compound of Chemical Formula 1.

### Technical Solution

As a result of their efforts to discover a novel compound capable of inhibiting mTORC2 activity and exhibiting therapeutic effects on neurodegenerative diseases caused by hyperactivation of mTORC2, the present inventors have found that the compound represented by Chemical Formula 1 described herein effectively inhibits mTORC2 activity and thus can exhibit a therapeutic effect on neurodegenerative diseases associated with hyperactivation of mTORC2, thereby completing the present invention.

One embodiment of the present application provides a compound represented by the following Chemical Formula 1, or a pharmaceutically acceptable salt thereof.

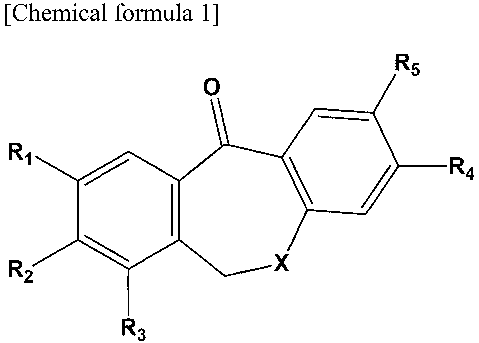

### Definition of Terms

The terms as used herein will be briefly explained below.

As used herein, the term "pharmaceutically acceptable salt" refers to a salt of a compound that does not cause significant irritation to an organism to which the compound is administered, and does not impair the biological activity and properties of the compound, and in the present invention, it collectively refers to any salt that retains the biological effectiveness and properties of the compound of Chemical Formula 1 and is preferred in terms of pharmaceutical, biological, or other properties. The pharmaceutically acceptable salt may include acid addition salts formed by acids capable of forming a non-toxic acid addition salt containing pharmaceutically acceptable anions, for example, inorganic acids such as hydrochloric acid, sulfuric acid, nitric acid, phosphoric acid, hydrobromic acid, hydroiodic acid, etc.; organic carbonic acids such as tartaric acid, formic acid, citric acid, acetic acid, trichloroacetic acid, trifluoroacetic acid, gluconic acid, benzoic acid, lactic acid, fumaric acid, maleic acid, salicylic acid, etc.; or sulfonic acids such as methanesulfonic acid, ethanesulfonic acid, benzenesulfonic acid, p-toluenesulfonic acid, etc. As a specific example thereof, an acid addition salt of the compound of one embodiment may be prepared by reacting free base forms of these compounds with a stoichiometric amount of the appropriate acid. At this time, the reaction may be performed in water, an organic solvent or a mixture thereof, and specifically, in a non-aqueous medium such as ether, ethyl acetate, ethanol, isopropanol, acetonitrile, etc. In addition, according to the form of the pharmaceutically acceptable salt, each form of the salts can be obtained by a General reaction which is apparent to those skilled in the art. Further, the pharmaceutically acceptable salt may be alkaline metal salts or alkaline earth metal salts formed by lithium, sodium, potassium, calcium, magnesium, etc., amino acid salts such as lysine, arginine, guanidine, etc., organic salts such as dicyclohexylamine, N-methyl-D-glucamine, tris(hydroxymethyl)methylamine, diethanolamine, choline, triethylamine, etc.

As used herein, the term "aryl" refers to a carbocyclic (e.g., phenyl) group which has a conjugated pi electron system and at least one ring. This term includes a monocyclic or fused-ring polycyclic (i.e., rings that share adjacent pairs of carbon atoms) group.

As used herein, the term "heteroaryl" refers to a heterocyclic aryl group which has a conjugated pi electron system and at least one ring, and is exemplified by furan, thiophene, pyrrole, imidazole, oxazole, isoxazole, oxadiazole, tetrazole, thiazole, imidazole, pyrazole, isothiazole, triazole, thiadiazole, pyridine, pyridazine, pyrimidine, pyrazine, triazine, etc., but is not limited thereto.

As used herein, the term "alkyl" refers to an aliphatic hydrocarbon group. An alkyl moiety may be a 'saturated alkyl' group containing no alkene or alkyne moiety or an " unsaturated alkyl" group containing at least one alkene or alkyne moiety. The "alkene" moiety refers to a group composed of at least one carbon-carbon double bond, and the "alkyne" moiety refers to a group composed of at least one carbon-carbon triple bond. For example, "alkyl" can be a saturated alkyl or unsaturated alkyl having a linear, branched, or cyclic structure. General alkyl groups include, but are not limited to, methyl, ethyl, propyl, isopropyl, butyl, isobutyl, t-butyl, pentyl, hexyl, ethenyl, propenyl, butenyl, and the like. For example, a C1-C4-alkyl has 1 to 4 carbon atoms in the alkyl chain and is selected from the group consisting of methyl, ethyl, propyl, iso-propyl, n-butyl, iso-butyl, sec-butyl, and t-butyl.

As used herein, the alkyl group may be a C₁₋₁₀ linear or branched alkyl group, and the alkoxy group may be a C₁₋₁₀ linear or branched alkoxy group. The C₁₋₁₀ linear or branched alkyl group, or the C₁₋₁₀ linear or branched alkoxy group may each independently be a group having: 1 to 10 carbon atoms (C1-C10), 1 to 9 carbon atoms (C1-C9), 1 to 8 carbon atoms (C1-C8), 1 to 7 carbon atoms (C1-C7), 1 to 6 carbon atoms (C1-C6), 1 to 5 carbon atoms (C1-C5), 1 to 4 carbon atoms (C1-C4), 1 to 3 carbon atoms (C1-C3), 1 to 2 carbon atoms (C1-C2), 1 carbon atom (C1), 2 to 10 carbon atoms (C2-C10), 2 to 9 carbon atoms (C2-C9), 2 to 8 carbon atoms (C2-C8), 2 to 7 carbon atoms (C2-C7), 2 to 6 carbon atoms (C2-C6), 2 to 5 carbon atoms (C2-C5), 2 to 4 carbon atoms (C2-C4), 2 to 3 carbon atoms (C2-C3), 2 carbon atoms (C2), 3 to 10 carbon atoms (C3-C10), 3 to 9 carbon atoms (C3-C9), 3 to 8 carbon atoms (C3-C8), 3 to 7 carbon atoms (C3-C7), 3 to 6 carbon atoms (C3-C6), 3 to 5 carbon atoms (C3-C5), 3 to 4 carbon atoms (C3-C4), 3 carbon atoms (C3), 4 to 10 carbon atoms (C4-C10), 4 to 9 carbon atoms (C4-C9), 4 to 8 carbon atoms (C4-C8), 4 to 7 carbon atoms (C4-C7), 4 to 6 carbon atoms (C4-C6), 4 to 5 carbon atoms (C4-C5), 4 carbon atoms (C4), 5 to 10 carbon atoms (C5-C10), 5 to 9 carbon atoms (C5-C9), 5 to 8 carbon atoms (C5-C8), 5 to 7 carbon atoms (C5-C7), 5 to 6 carbon atoms (C5-C6), 5 carbon atoms (C5), 6 to 10 carbon atoms (C6-C10), 6 to 9 carbon atoms (C6-C9), 6 to 8 carbon atoms (C6-C8), 6 to 7 carbon atoms (C6-C7), 6 carbon atoms (C6), 7 to 10 carbon atoms (C7-C10), 7 to 9 carbon atoms (C7-C9), 7 to 8 carbon atoms (C7-C8), 7 carbon atoms (C7), 8 to 10 carbon atoms (C8-10), 8 to 9 carbon atoms (C8-C9), 9 carbon atoms (C9), 9 to 10 carbon atoms (C9-C10), or 10 carbon atoms (C10).

As used herein, the term "halo" or "halogen" may be fluoro(-F), chloro (-Cl), bromo (-Br), or iodo(-I).

As used herein, the term 'heterocycle' refers to a group in which a cyclic carbon is replaced by oxygen, nitrogen, sulfur, etc., and the group may contain any double bond. The heterocycle may be exemplified by pyrroline, pyrrolidine, tetrahydrofuran, imidazoline, imidazolidine, pyrazoline, pyrazolidine, pyran, piperidine, piperazine, morpholine, thiomorpholine, etc., but is not limited thereto.

The "each independently substituted" means that when the number of replaceable hydrogen atoms is two or more, each hydrogen atom may be replaced with the same or different substituents from each other.

Terms other than those described above may be construed as meaning commonly understood by those skilled in the art to which the present invention pertains.

Hereinafter, the present application is described in more detail.

### Compound of Chemical Formula 1

One embodiment of the present application provides a compound represented by the following Chemical Formula 1 or a pharmaceutically acceptable salt thereof. 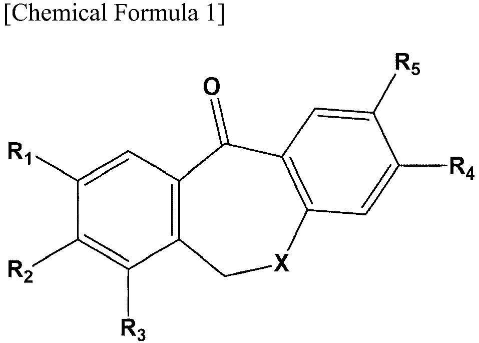
wherein in Chemical Formula 1,
the "X" is oxygen, sulfur, or a methylene group or a substituted amine,
the "R₁" is hydrogen, halo (F, Br, Cl, or I), a substituted or unsubstituted C₆₋₁₀ aryl, or a substituted or unsubstituted 4 to 12-membered heteroaryl containing one or more heteroatoms selected from the group consisting of N, O, and S,
the "R₂" is hydrogen, halo (F, Br, Cl, or I), a substituted or unsubstituted C₆₋₁₀ aryl, a substituted or unsubstituted C₁₋₆ amino, a substituted or unsubstituted 4 to 12-membered heterocycloalkyl, or an unsubstituted benzylamino,
the "R₃" is hydrogen or halo (F, Br, Cl, or I),
the "R₄" and "R₅" are each independently an unsubstituted C₁₋₆ alkoxy, or "R₄" and "R₅" together with two oxygen atoms to which they are attached may form -O(CH)ₙO-(wherein n is 1 or 2),
the substituted amine may be substituted with one or more substituents selected from the group consisting of a substituted or unsubstituted C₁₋₁₀ linear or branched alkyl, a substituted or unsubstituted C₆₋₁₀ aryl, and a substituted or unsubstituted C₆₋₁₀ arylsulfonyl, wherein the substituted linear or branched alkyl, aryl or arylsulfonyl may be substituted with a C₁₋₅ linear or branched alkyl,
the substituted aryl, or the substituted heteroaryl may be substituted with one or more substituents selected from the group consisting of hydroxy(OH), halo(F, Br, Cl, or I), a C₁₋₁₀ linear or branched alkyl which is substituted or unsubstituted with 1 to 3 halo, and an unsubstituted C₁₋₅ linear or branched alkoxy (as used herein, "substituted with one or more" includes when substituted with two or more identical substituents: for example, when substituted with two -OCH₃, etc.),
the substituted amino may be substituted with one or more substituents selected from the group consisting of a C₁₋₅ linear or branched alkyl, an unsubstituted C₃₋₈ cycloalkyl, a C₁₋₅ linear alkyl which is substituted with a phenyl group substituted with a C₁₋₅ linear or branched alkoxy, a C₁₋₅ linear alkyl which is substituted with an unsubstituted thiophenyl group, a C₃₋₈ cycloalkyl and a sulfonyl which is substituted with a C₁₋₅ linear alkyl,
the substituted heterocycloalkyl may be substituted with an unsubstituted C₆₋₁₀ aryl, or an unsubstituted C₆₋₁₀ aryl C₁₋₆ alkyl, and
the compound of Chemical Formula 1 does not include the following compounds:
   a) 2,3-dimethoxydibenz[b,e]oxepin-11(6H)-one; and
   b) 9-bromo[2]benzoxepino[3,4-f]-1,3-benzodioxol-11(6H)-one.

In the Chemical Formula 1 of the present invention, the "X" may be oxygen, sulfur, or a methylene group, and in one embodiment, the "X" may be oxygen.

In the Chemical Formula 1 of the present invention, the "R₁" may be hydrogen, halo(F, Br, Cl, or I), a substituted or unsubstituted C₆₋₁₀ aryl, or a substituted or unsubstituted 4 to 10-membered heteroaryl containing one or more heteroatoms selected from the group consisting of N, O, and S, and specifically, it may be hydrogen(H), fluoro(F), bromo(Br), a substituted or unsubstituted C₆₋₁₀ aryl, or an unsubstituted 4 to 6-membered heteroaryl containing O as a heteroatom.

The substituted aryl of "R₁" may be substituted with 1 to 3 substituents selected from the group consisting of hydroxy(OH), fluoro(F), chloro(Cl), a C₁₋₃ linear alkyl substituted or unsubstituted with 1 to 3 fluoro, and an unsubstituted C₁₋₃ linear alkoxy, and specifically, it may be substituted with one substituent selected from the group consisting of hydroxy(OH), fluoro(F), chloro(Cl), trifluoro(CF₃), methyl(CH₃), and 1 to 3 methoxy(OCH₃) groups.

The heteroaryl of "R₁" may be furanyl.

In the Chemical Formula 1 of the present invention, the "R₂" may be hydrogen, halo(-F, -Br, -Cl, or -I), a substituted or unsubstituted C₆₋₁₀ aryl, a substituted or unsubstituted amino, a substituted or unsubstituted 4 to 6-membered heterocycloalkyl, or an unsubstituted benzylamino, and specifically, it may be hydrogen(H), fluoro(F), a substituted C₆ aryl, a substituted or unsubstituted C₁₋₆ alkylamino, a substituted or unsubstituted 6-membered heterocycloalkyl, or an unsubstituted benzylamino.

The substituted aryl of "R₂" may be substituted with 1 to 3 substituents selected from the group consisting of chloro(Cl), an unsubstituted C₁₋₃ linear alkyl, and an unsubstituted C₁₋₃ linear alkoxy, and specifically, it may be substituted with one substituent selected from the group consisting of chloro(Cl), methyl(CH₃), and 1 to 3 methoxy(OCH₃) groups.

The substituted amino of "R₂" may be substituted with one to three substituents selected from the group consisting of a C₁₋₅ linear or branched alkyl, an unsubstituted C₅₋₇ cycloalkyl, a C₁₋₄ linear alkyl which is substituted with a phenyl group substituted with a C₁₋₄ linear or branched alkoxy, a C₁₋₄ straight alkyl which is substituted with an unsubstituted thiophenyl group, and a sulfonyl which is substituted with a C₁₋₄ linear alkyl, and specifically, it may be substituted with one substituent selected from the group consisting of two methyl (CH₃), cyclohexyl, phenethyl, thiophenylethyl, and methylsulfonyl.

The substituted heterocycloalkyl of "R₂" may be substituted with an unsubstituted phenyl.

The heterocycloalkyl of "R₂" may be morpholine, piperidine, or piperazine, and specifically, it may be an unsubstituted morpholine, an unsubstituted piperidine, or piperazine substituted with an unsubstituted phenyl.

In the Chemical Formula 1 of the present invention, the "R₃" may be hydrogen or halo (F, Br, Cl, or I), and specifically, it may be hydrogen or fluoro.

In the Chemical Formula 1 of the present invention, the "R₄" and "R₅" may each independently be an unsubstituted C₁₋₃ alkoxy, or "R₄" and "R₅" together with two oxygen atoms to which they are attached may form -O(CH)ₙO- (wherein n is 1 or 2). Specifically, "R₄" and "R₅" may each independently be methoxy, or "R₄" and "R₅" together with two oxygen atoms to which they are attached may form -O(CH)₂O-.

More specifically, in the Chemical Formula 1, the "X" may be oxygen, sulfur or methylene group, and
the "R₁" may be one selected from the group consisting of hydrogen(H), fluoro(F), bromo(Br), 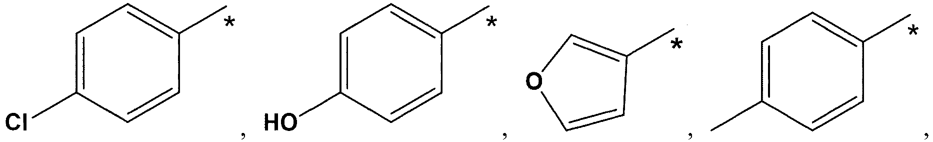 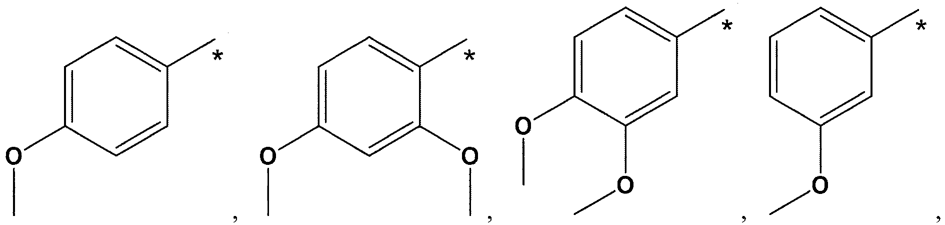 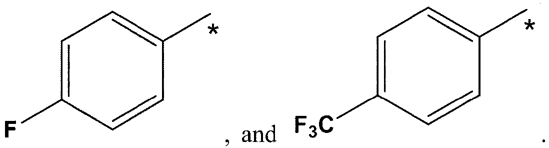 (An asterisk (*) in the exemplified substituents means a portion that bonds to other groups.)
The "R₂" may be one selected from the group consisting of hydrogen(H), fluoro(F), 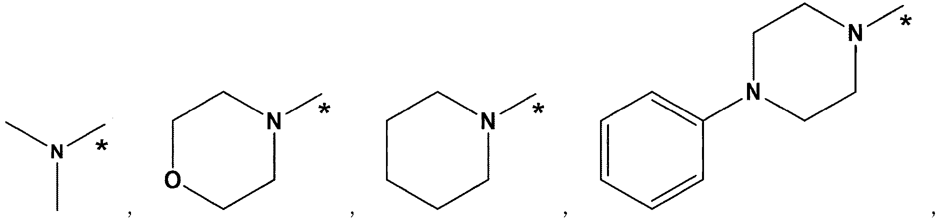 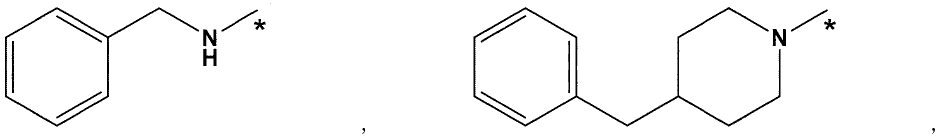 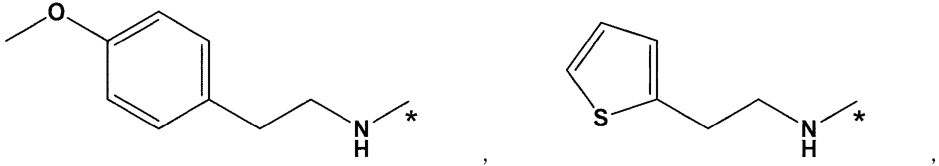 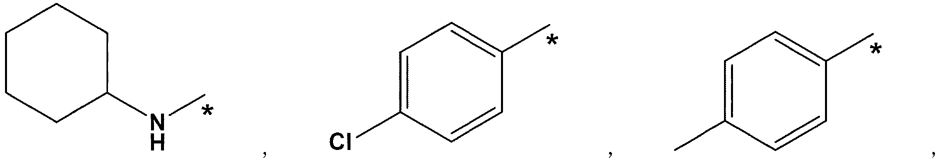 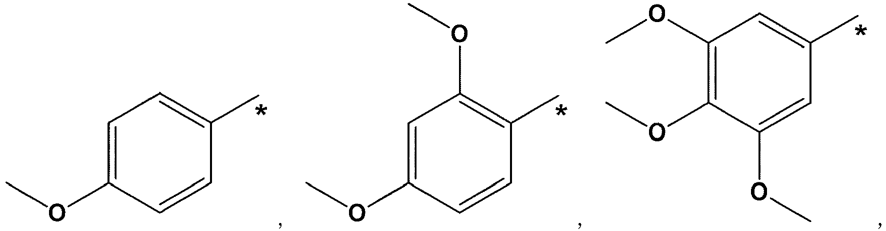 and

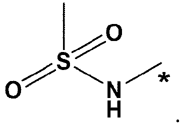

The "R₄" and "R₅" are each independently a substituted or unsubstituted methoxy, or "R₄" and "R₅" together with two oxygen atoms to which they are attached may form dioxolane.

In one embodiment, the compound of Chemical Formula 1 may be one or more kinds selected from the group consisting of the following compounds:
1) 9-fluoro[2]benzoxepino[3,4-f]-1,3-benzodioxol-11(6H)-one **(HN2203);**
2) [2]benzoxepino[3,4-f]-1,3-benzodioxol-11(6H)-one **(HN2204);**
3) 8,9-difluoro[2]benzoxepino[3,4-f]-1,3-benzodioxol-11(6H)-one **(HN2208);**
4) 8-(dimethylamino)-9-fluoro[2]benzoxepino[3,4-f]-1,3-benzodioxol-11(6H)-one **(HN2209);**
5) 8-(morpholino)-9-fluoro[2]benzoxepino[3,4-f]-1,3-benzodioxol-11(6H)-one **(HN2210);**
6) 8-(piperidinyl)-9-fluoro[2]benzoxepino[3,4-f]-1,3-benzodioxol-11(6H)-one **(HN2211);**
7) 8-(phenylpiperazine-1-yl)-9-fluoro[2]benzoxepino[3,4-f]-1,3-benzodioxol-11(6H)-one **(HN2212);**
8) 8-(benzylamino)-9-fluoro[2]benzoxepino[3,4-f]-1,3-benzodioxol-11(6H)-one **(HN2213);**
9) 9-(4-chlorophenyl)[2]benzoxepino[3,4-f]-1,3-benzodioxol-11(6H)-one **(HN2214);**
10) 9-(4-hydroxyphenyl)[2]benzoxepino[3,4-f]-1,3-benzodioxol-11(6H)-one **(HN2215);**
11) 9-(3-furanyl)[2]benzoxepino[3,4-f]-1,3-benzodioxol-11(6H)-one **(HN2216);**
12) 9-(p-tolyl)[2]benzoxepino[3,4-f]-1,3-benzodioxol-11(6H)-one **(HN2217);**
13) 9-(4-methoxyphenyl)[2]benzoxepino[3,4-f]-1,3-benzodioxol-11(6H)-one **(HN2218);**
14) 9-(2,4-dimethoxyphenyl)[2]benzoxepino[3,4-f]-1,3-benzodioxol-11(6H)-one **(HN2219);**
15) 9-(3,4,5-trimethoxyphenyl)[2]benzoxepino[3,4-f]-1,3-benzodioxol-11(6H)-one **(HN2220);**
16) 8-(cyclohexylamino)-9-fluoro[2]benzoxepino[3,4-f]-1,3-benzodioxol-11(6H)-one **(HN2221);**
17) 8-(4-benzylpiperidin-1-yl)-9-fluoro[2]benzoxepino[3,4-f]-1,3-benzodioxol-11(6H)-one **(HN2222);**
18) 8-((4-methoxyphenethyl)amino)-9-fluoro[2]benzoxepino[3,4-f]-1,3-benzodioxol-11(6H)-one **(HN2223);**
19) 8-((2-(thiophen-2-yl)ethyl)amino)-9-fluoro[2]benzoxepino[3,4-f]-1,3-benzodioxol-11(6H)-one **(HN2224);**
20) 7, 8-difluoro[2]benzoxepino[3,4-f]-1,3-benzodioxol-11(6H)-one **(HN2225);**
21) 8-(dimethylamino)-7-fluoro[2]benzoxepino[3,4-f]-1,3-benzodioxol-11(6H)-one **(HN2226);**
22) 8-(morpholino)-7-fluoro[2]benzoxepino[3,4-f]-1,3-benzodioxol-11(6H)-one **(HN2227);**
23) 8-(piperidinyl)-7-fluoro[2]benzoxepino[3,4-f]-1,3-benzodioxol-11(6H)-one **(HN2228);**
24) 8-(phenylpiperazine-1-yl)-7-fluoro[2]benzoxepino[3,4-f]-1,3-benzodioxol-11(6H)-one **(HN2229);**
25) 8-(benzylamino)-7-fluoro[2]benzoxepino[3,4-f]-1,3-benzodioxol-11(6H)-one **(HN2230);**
26) 8-(cyclohexylamino)-7-fluoro[2]benzoxepino[3,4-f]-1,3-benzodioxol-11(6H)-one **(HN2231);**
27) 8-(4-benzylpiperidin-1-yl)-7-fluoro[2]benzoxepino[3,4-f]-1,3-benzodioxol-11(6H)-one **(HN2232);**
28) 8-((4-methoxyphenethyl)amino)-7-fluoro[2]benzoxepino[3,4-f]-1,3-benzodioxol-11(6H)-one **(HN2233);**
29) 8-((2-(thiophen-2-yl)ethyl)amino)-7-fluoro[2]benzoxepino[3,4-f]-1,3-benzodioxol-11(6H)-one **(HN2234);**
30) 9-bromo-2,3-dimethoxydibenz[b,e]oxepin-11(6H)-one **(HN2235);**
31) 9-(2,4-dimethoxyphenyl)-2,3-dimethoxydibenzo[b,e]oxepin-11(6H)-one **(HN2236);**
32) 9-(3,4-dimethoxyphenyl)-2,3-dimethoxydibenzo[b,e]oxepin-11(6H)-one **(HN2237);**
33) 2,3-dimethoxy-9-(3-methoxyphenyl)dibenzo[b,e]oxepin-11(6H)-one **(HN2238);**
34) 9-(4-fluorophenyl)-2,3-dimethoxydibenzo[b,e]oxepin-11(6H)-one **(HN2239);**
35) 8-(4-chlorophenyl)[2]benzoxepino[3,4-f]-1,3-benzodioxol-11(6H)-one **HN2240);**
36) 8-(p-tolyl)[2]benzoxepino[3,4-f]-1,3-benzodioxol-11(6H)-one **(HN2241);**
37) 8-(4-methoxyphenyl)[2]benzoxepino[3,4-f]-1,3-benzodioxol-11(6H)-one **(HN2242);**
38) 8-(2,4-dimethoxyphenyl)[2]benzoxepino[3,4-f]-1,3-benzodioxol-11(6H)-one **(HN2243);**
39) 8-(3,4,5-trimethoxyphenyl)[2]benzoxepino[3,4-f]-1,3-benzodioxol-11(6H)-one **(HN2244);**
40) 2,3-dimethoxy-9-(4-(trifluoromethyl)phenyl)dibenzo[b,e]oxepin-11(6H)-one **(HN2245);**
41) N-(9-fluoro-11-oxo-6,11-dihydro-[1,3]dioxolo[4',5': 4,5]benzo[1,2-b]benzo[e]oxepin-8-yl)methanesulfonamide **(HN2301);**
42) N-(7-fluoro-11-oxo-6,11-dihydro-[1,3]dioxolo[4',5': 4,5]benzo[1,2-b]benzo[e]oxepin-8-yl)methanesulfonamide **(HN2302);**
43) 2,3-dimethoxy-10,11-dihydro-5H-dibenzo[a,d][7]annulen-5-one **(HN2303);**
44) 9-bromo-2,3-dimethoxydibenzo[b,e]thiepin-11(6H)-one **(HN2304);**
45) 2,3-dimethoxy-9-(3,4,5-trimethoxyphenyl)dibenzo[b,e]thiepin-11(6H)-one **(HN2305);**
46) 2,3-dimethoxy-9-(4-methoxyphenyl)dibenzo[b,e]thiepin-11(6H)-one **(HN2306);**
47) 2,3-dimethoxy-9-(p-tolyl)dibenzo[b,e]thiepin-11(6H)-one **(HN2307);**
48) 9-(3-furanyl)-{2,3-dimethoxydibenzo[b,e]thiepin-11(6H)-one **(HN2308);**
49) 9-(4-chlorophenyl)-2,3-dimethoxydibenzo[b,e]thiepin-11(6H)-one **(HN2309);**
50) 10,11-dihydro-SH-benzo[4',5']cyclohepta[1',2':4,5]benzo[1,2-d][1,3]dioxol-5-one **(HN2310);**
51) 2,3-difluoro-7,8-dimethoxy-10,11-dihydro-5H-dibenzo[a,d][7]annulen-5-one **(HN2311);**
52) 8-(cyclopropylamino)-9-fluoro-[1,3]dioxolo[4',5':4,5]benzo[1,2-b]benzo[e]oxepin-11(6H)-one **(HN2312);**
53) 8-(cyclopentylamino)-9-fluoro-[1,3]dioxolo[4',5':4,5]benzo[1,2-b]benzo[e]oxepin-11(6H)-one **(HN2313);**
54) 2-(dimethylamino)-3-fluoro-7,8-dimethoxy-10,11-dihydro-5H-dibenzo[a,d][7]annulen-5-one **(HN2314);**
55) 3-fluoro-7,8-dimethoxy-2-(piperidin-1-yl)-10,11-dihydro-5H-dibenzo[a,d][7]annulen-5-one **(HN2315);**
56) 3-fluoro-7,8-dimethoxy-2-(4-phenylpiperazin-1-yl)-10,11-dihydro-5H-dibenzo[a,d][7]annulen-5-one **(HN2316);**
57) 2-(benzylamino)-3-fluoro-7,8-dimethoxy-10,11-dihydro-5H-dibenzo[a,d][7]annulen-5-one **(HN2317);**
58) 8-(cyclopropylamino)-7-fluoro-[1,3]dioxolo[4',5':4,5]benzo[1,2-b]benzo[e]oxepin-11(6H)-one **(HN2318);**
59) 8-(cyclopentylamino)-7-fluoro-[1,3]dioxolo[4',5':4,5]benzo[1,2-b]benzo[e]oxepin-11(6H)-one **(HN2319);**
60) 2-(cyclopropylamino)-3-fluoro-7,8-dimethoxy-10,11-dihydro-5H-dibenzo[a,d][7]annulen-5-one **(HN2320);**
61) 2-(cyclopentylamino)-3-fluoro-7,8-dimethoxy-10,11-dihydro-5H-dibenzo[a,d][7]annulen-5-one **(HN2321);**
62) 8-((2,4-dichlorophenethyl)amino)-9-fluoro-[1,3]dioxolo[4',5':4,5]benzo[1,2-b]benzo[e]oxepin-11(6H)-one **(HN2322);**
63) 9-fluoro-8-((2-methoxybenzyl)amino)-[1,3]dioxolo[4',5':4,5]benzo[1,2-b]benzo[e]oxepin-11(6H)-one **(HN2323);**
64) 8,9-difluoro-2,3-dimethoxydibenzo[b,e]thiepin-11(6H)-one **(HN2324);**
65) 9-fluoro-8-((3-methoxybenzyl)amino)-[1,3]dioxolo[4',5':4,5]benzo[1,2-b]benzo[e]oxepin-11(6H)-one **(HN2325);**
66) 9-fluoro-8-((4-methoxybenzyl)amino)-[1,3]dioxolo[4',5':4,5]benzo[1,2-b]benzo[e]oxepin-11(6H)-one **(HN2326);**
67) 9-fluoro-8-((3-methoxyphenethyl)amino)-[1,3]dioxolo[4',5':4,5]benzo[1,2-b]benzo[e]oxepin-11(6H)-one **(HN2327);**
68) 8-((2,4-dimethoxybenzyl)amino)-9-fluoro-[1,3]dioxolo[4',5':4,5]benzo[1,2-b]benzo[e]oxepin-11(6H)-one **(HN2328);**
69) 8-((3,4-dimethoxybenzyl)amino)-9-fluoro-[1,3]dioxolo[4',5':4,5]benzo[1,2-b]benzo[e]oxepin-11(6H)-one **(HN2329);**
70) 8-Bromo[2]benzoxepino[3,4-f]-1,3-benzodioxol-11(6H)-one **(HN2336);** and
71) 2,3-dimethoxy-5-tosyl-5,6-dihydro-11H-dibenzo[b,e]azepin-11-one**(HN2337).**

### Activity of the compound of Chemical Formula 1

The compound of Chemical Formula 1 of the present invention can inhibit the activity of mTORC1 (mammalian target of rapamycin complex 1) and/or mTORC2 (mammalian target of rapamycin complex 2), and specifically, it can selectively inhibit the activity of mTORC2, and can have the activity for preventing, improving and/or treating neurodegenerative diseases.

The inhibition of mTORC1 activity can be confirmed by measuring the extent to which p70S6K Thr389 phosphorylation is inhibited in human cells, while the inhibition of mTORC2 activity can be confirmed by measuring the extent to which AKT Ser473 phosphorylation is inhibited in human cells. The measurements can be carried out by Western blot method, but are not limited thereto.

In one embodiment of the present invention, it was confirmed that the compound of Chemical Formula 1 can inhibit both AKT Ser473 phosphorylation and p70S6K Thr389 phosphorylation in human cells, so that the compound of Chemical Formula 1 has mTORC1 and/or mTORC2 inhibitory activity. Furthermore, in one embodiment, it was confirmed that the compound of Chemical Formula 1 has a higher inhibitory effect on AKT Ser473 phosphorylation than on p70S6K Thr389 phosphorylation, indicating that the compound of Chemical Formula 1 can selectively inhibit mTORC2 activity.

The compound of Chemical Formula 1 of the present invention can inhibit the formation of mTORC2 complex.

The neurodegenerative disease refers to a disease occurring in the central nervous system among degenerative diseases, and the neurodegenerative disease may be a disease caused by excessive activity of mTORC2.

The neurodegenerative disease may be at least one selected from the group consisting of Alzheimer's disease, senile dementia, Lewy body dementia, frontotemporal dementia, mild cognitive impairment, Parkinson's disease, Pick's disease, Huntington's disease, spinocerebellar ataxia, multiple systemic atrophy, epilepsy, encephalopathy, stroke, cerebral amyloid angiopathy, Down's syndrome, systemic amyloid disease, Niemann-Pick disease, amyotrophic lateral sclerosis, Tourette's syndrome, Friedrich's ataxia, Machado-Joseph's disease, dystonia, and progressive supranuclear palsy. In one embodiment, the neurodegenerative disease may be Alzheimer's disease.

As used herein, the term "prevention" refers to any act that suppresses or delays the onset of a disease (disorder) through administration of the composition according to one embodiment, the term "treatment" refers to any act that improves or beneficially alters the symptoms of a suspected or affected individual with the disease through administration of the composition according to one embodiment, and the term "improvement" refers to any act that reduces at least one parameter related to the state in which the disease is being treated, for example, the severity of a symptom, through administration of the composition according to one embodiment.

In accordance with the activity of the compound of Chemical Formula 1, the present invention provides a composition for inhibiting mTORC2 activity, comprising the compound of Chemical Formula 1 or a pharmaceutically acceptable salt thereof.

The composition for inhibiting mTORC2 activity may be a pharmaceutical composition or a food composition, but is not limited thereto.

In addition, in accordance with the activity of the compound of Chemical Formula 1, the present invention provides a composition for prevention or treatment of neurodegenerative diseases comprising the compound of Chemical Formula 1 or a pharmaceutically acceptable salt thereof.

The composition may be a pharmaceutical composition or a food composition, but is not limited thereto.

### Pharmaceutical Composition

The present invention provides a composition for prevention or treatment of neurodegenerative diseases, comprising the compound of Chemical Formula 1 or a pharmaceutically acceptable salt thereof as an active ingredient.

The composition may be a pharmaceutical composition or a food composition.

In addition, the present invention provides a method for prevention, improvement, and/or treatment of neurodegenerative diseases, comprising administering an effective amount of the compound of Chemical Formula 1 or a pharmaceutically acceptable salt thereof to a subject in need of prevention, amelioration and/or treatment of a neurodegenerative diseases.

The method may further include a step of identifying a subject in need of prevention, improvement and/or treatment of a neurodegenerative disease prior to the administering step.

Another embodiment provides a use of a composition comprising the compound of Chemical Formula 1 or a pharmaceutically acceptable salt thereof for preparing a composition for prevention, improvement and/or treatment of neurodegenerative diseases.

The composition may be a pharmaceutical composition.

The pharmaceutically acceptable salt of the compound of Chemical Formula 1 may be at least one selected from the group consisting of an acid addition salt, a metal salt, an amino acid salt, and an organic salt of the compound of Chemical Formula 1. The acid addition salt may be acid addition salts formed by, for example, inorganic acids such as hydrochloric acid, sulfuric acid, nitric acid, phosphoric acid, hydrobromic acid, hydroiodic acid, etc.; organic carbonic acids such as tartaric acid, formic acid, citric acid, acetic acid, trichloroacetic acid, trifluoroacetic acid, gluconic acid, benzoic acid, lactic acid, fumaric acid, maleic acid, salicylic acid, etc.; or sulfonic acids such as methanesulfonic acid, ethanesulfonic acid, benzenesulfonic acid, p-toluenesulfonic acid, etc. The metal salt may be alkaline metal salts or alkaline earth metal salts formed by lithium, sodium, potassium, calcium, magnesium, etc. The amino acid salt may be amino acid salts formed by lysine, arginine, guanidine, etc. The organic salt may be organic salts formed by dicyclohexylamine, N-methyl-D-glucamine, tris(hydroxymethyl)methylamine, diethanolamine, choline, triethylamine, etc.

The administration subject of the pharmaceutical composition may be one or more kinds selected from a mammal including primates such as human and monkeys, rodents such as mice and rats, livestock such as dogs, cats, pigs, cattle, horses, sheep and goats, poultry such as chickens, ducks, geese, pheasants, quails and turkeys, and the like, or a cell, tissue or culture thereof derived therefrom. For example, the administration subject may be a subject in need of prevention, improvement and/or treatment of neurodegenerative diseases, and may be, for example, a human.

A pharmaceutical composition comprising the compound of Chemical Formula 1 or the pharmaceutically acceptable salt thereof as an active ingredient may be formulated and used in the form of a General drug preparation. For example, the drug formulation may be prepared in a variety of formulations for oral or parenteral administration, and the type of the formulation may differ, depending on the method of use, administration method, administration purpose, etc.

When prepared into a variety of formulations for oral or parenteral administration, it may be formulated by using one or more kinds selected from the group consisting of general diluents or excipients, such as a filler, an extender, a binder, a wetting agent, a disintegrating agent, and a surfactant.

Solid formulations for oral administration may include tablets, pills, powders, granules, capsules, and the like. Such solid formulations may be prepared by mixing the active ingredient with one or more excipients, e.g., one or more selected from the group consisting of starch, calcium carbonate, sucrose, lactose, gelatin, and the like. In addition to simple excipients, lubricants such as magnesium stearate and talc may also be used. Further, liquid formulations for oral administration may include suspensions, liquids for internal use, emulsions, syrups, and the like. When formulating into the liquid formulation, commonly used simple diluents such as water and/or liquid paraffin may be used, and optionally, one or more selected from the group consisting of various excipients, for example, wetting agents, sweeteners, fragrances, preservatives, and the like may be further included.

The parenteral administration may be performed via an intravenous route, an intramuscular route, a subcutaneous route, an intraperitoneal route, an intranasal route, a transdermal route, an intradermal route, a topical route, an intrapulmonary route. The formulation for parenteral administration may include sterile aqueous solutions, non-aqueous solutions, suspensions, emulsions, lyophilized preparations, suppositories, etc. A non-aqueous solvent for the preparation of the non-aqueous solutions and a suspension solvent for the preparation of the suspensions may include propylene glycol, polyethylene glycol, vegetable oil such as olive oil, injectable ester such as ethyloleate, etc. The base for suppositories may include witepsol, macrogol, tween 61, cacao butter, laurin butter, glycerogelatin, etc. When administered intranasally, the pharmaceutical composition may be diluted and administered via nasal spray, which is absorbed into the nasal cavity via a nebulizer or mist system. For the nasal spray, an aerosol agent, etc. may be used as a nasal spray agent or a respiratory preparation.

The content of the active ingredient of the compound of Chemical Formula 1 or a pharmaceutically acceptable salt thereof contained in the pharmaceutical composition may be 0.01% by weight to 99.9% by weight, 0.01% by weight to 90% by weight, 0.01% by weight to 80% by weight, 0.01% by weight to 70% by weight, 0.01% by weight to 60% by weight, 0.01% by weight to 50% by weight, 0.01% by weight to 40% by weight, 0.01% by weight to 30% by weight, 1% by weight to 99.9% by weight, 1% by weight to 90% by weight, 1% by weight to 80% by weight, 1% by weight to 70% by weight, 1% by weight to 60% by weight, 1% by weight to 50% by weight, 1% by weight to 40% by weight, 1% by weight to 30% by weight, 5% by weight to 99.9% by weight, 5% by weight to 90% by weight, 5% by weight to 80% by weight, 5% by weight to 70% by weight, 5% by weight to 60% by weight, 5% by weight to 50% by weight, 5% by weight to 40% by weight, 5% by weight to 30% by weight, 10% by weight to 99.9% by weight, 10% by weight to 90% by weight, 10% by weight to 80% by weight, 10% by weight to 70% by weight, 10% by weight to 60% by weight, 10% by weight to 50% by weight, 10% by weight to 40% by weight, or 10% by weight to 30% by weight, based on the total weight of the pharmaceutical composition. However, the content is not limited thereto, and can be appropriately adjusted depending on the formulation form, administration method, administration purpose, etc.

The pharmaceutically effective amount of the pharmaceutical composition comprising the compound of Chemical Formula 1 or a pharmaceutically acceptable salt thereof as an active ingredient may be variously prescribed in consideration of factors such as the formulation method, the administration method, and the age, body weight, sex, and pathological condition of the patient, food, administration time, administration interval, administration route, excretion rate, and responsiveness of the patient, and the composition may be administered or taken once or several times a day, but is not limited thereto and can be administered in various dosages and methods. In one embodiment, a single dose of the pharmaceutical composition may be in the range of 0.001mg/kg to 100mg/kg, but is not limited thereto.

As used herein, the term "pharmaceutically effective amount" means an amount of the active ingredient that is enough to obtain a desired pharmaceutical effect, and according to circumstances, it means a concentration or administration dose of the active ingredient in the pharmaceutical composition, which is enough to exhibit the desired pharmaceutical effect.

### Preparation method

In another embodiment, a method for producing the compound of Chemical Formula 1 is provided. The detailed method for preparing the compound of Chemical Formula 1 will be described below in the following aspects.

### Advantageous Effects

The present invention relates to a novel compound having mTORC2 inhibitory activity, a preparation method thereof, and a pharmaceutical composition comprising the same as an active ingredient. A novel compound of Chemical Formula 1 according to the present invention inhibits mTORC2 activity, and therefore can be effectively used for the prevention, improvement or treatment of neurodegenerative diseases (e.g., Alzheimer's disease) caused by overactivated mTORC2.

### BRIEF DESCRIPTION OF THE DRAWINGS

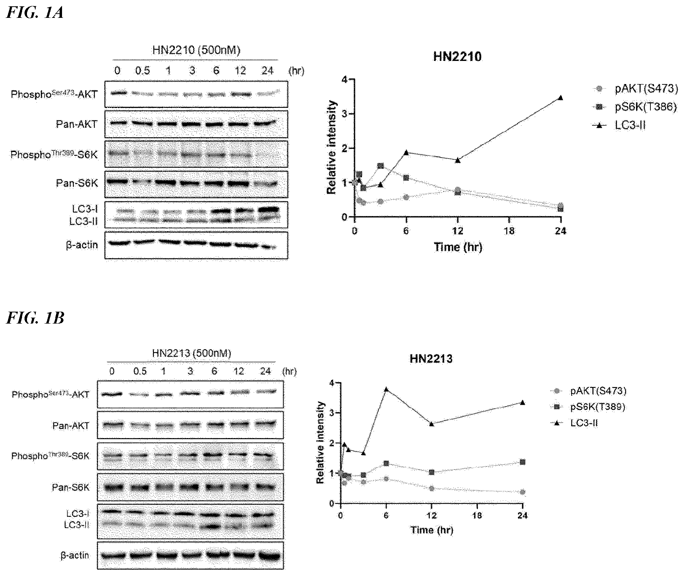

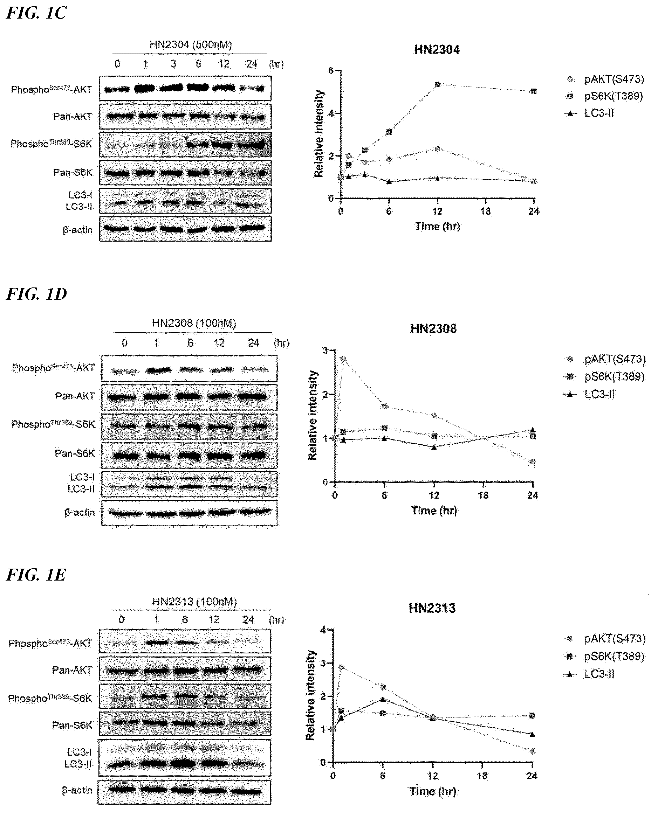

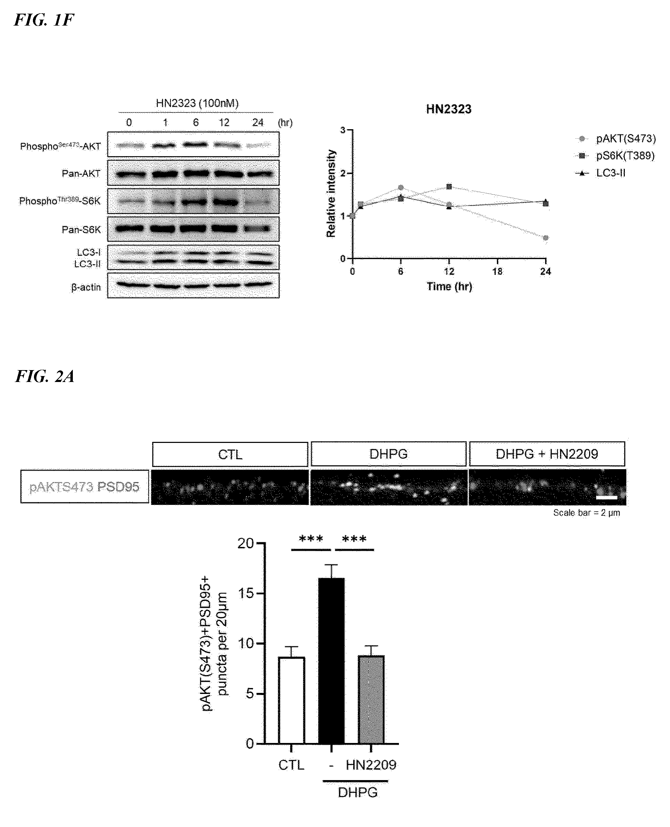

FIG. 1 shows the results of Western blot analysis evaluating the time-dependent inhibitory effect of the compound of the present application on mTORC2, mTORC1, and LC3-II activity in primary cultured cortical neurons (left figure), and the graph illustrating changes in Akt Ser473 phosphorylation, Akt Thr308 phosphorylation, and LC3-II changes (right figure). Specifically, FIG. 1a shows the result for HN2210, FIG. 1b for HN2213, FIG. 1c for HN2304, FIG. 1d for HN2308, FIG. 1e for HN2313, and FIG. 1f for HN2323.

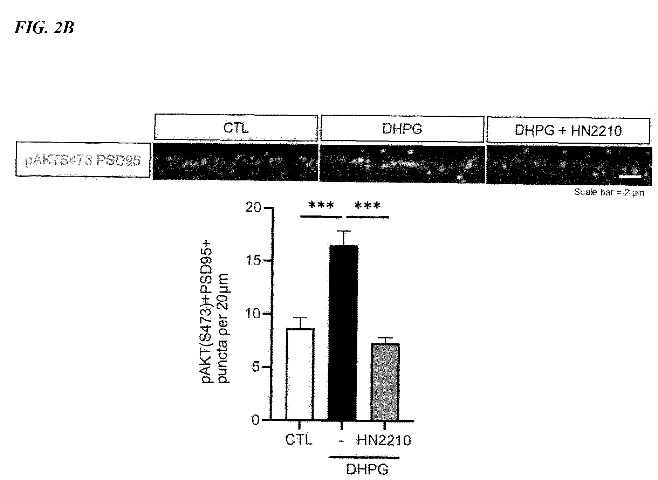

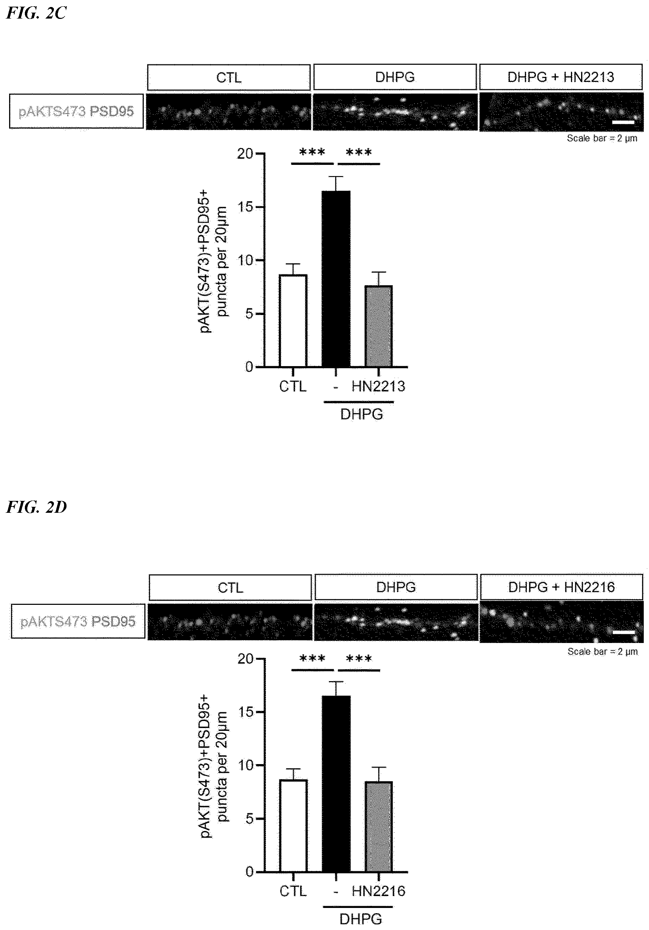

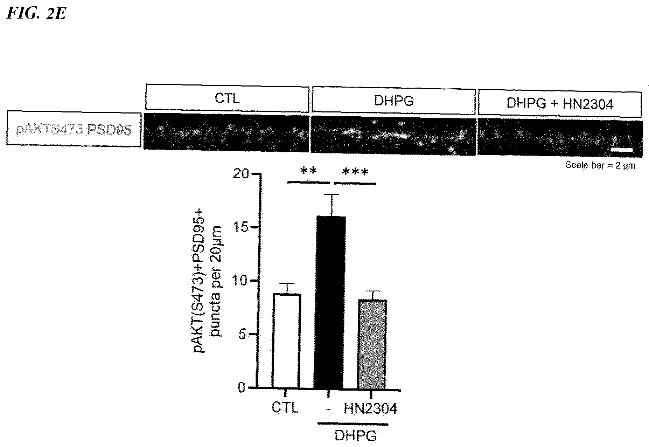

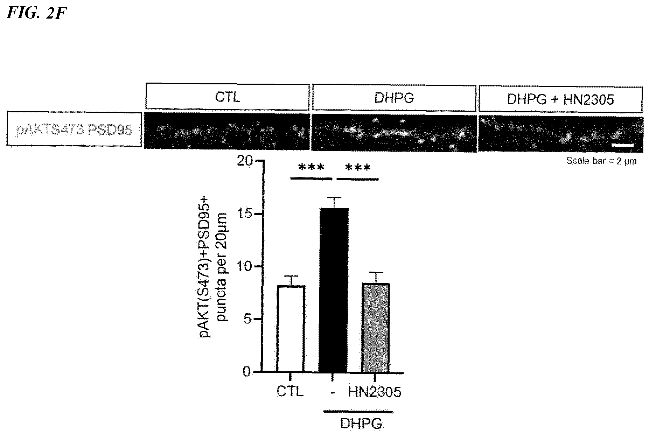

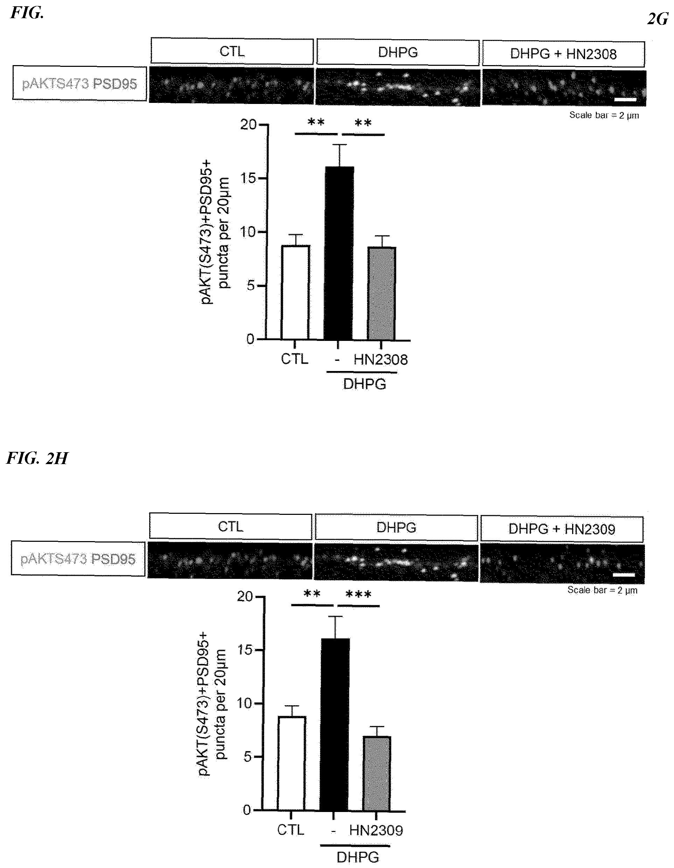

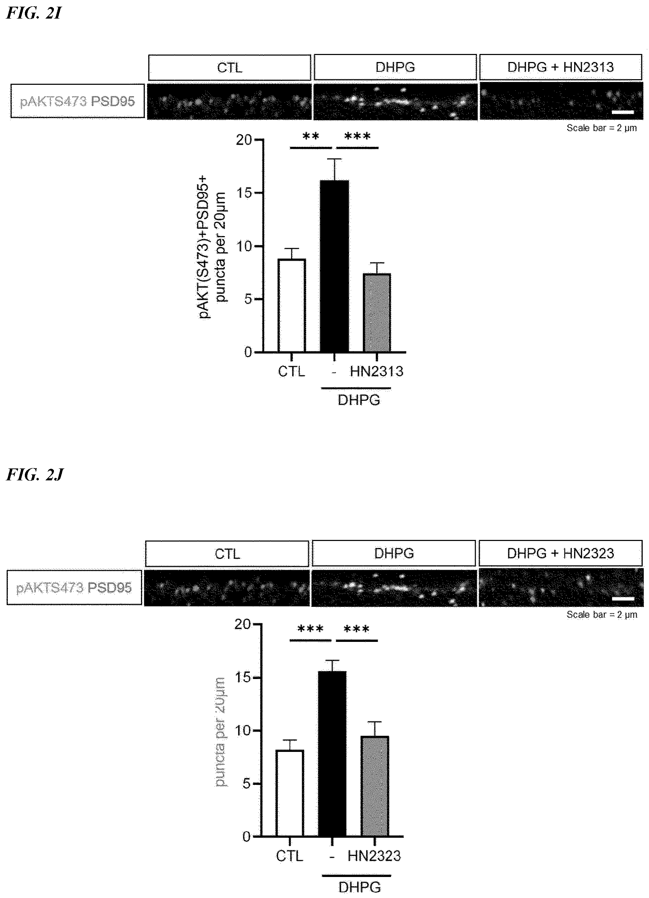

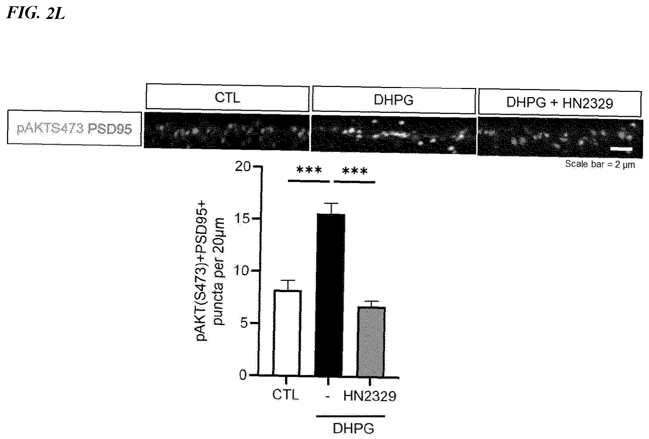

FIG. 2 shows confocal scanning microscope images (top) and graphs (bottom) showing that the compound of the present application inhibits the phosphorylation of AKT-S473 (green dots) induced by mTORC2 activation upon treatment with the mGluR1/5 agonist DHPG in dendritic spines (PSD95+) of primary cultured hippocampal neurons, in which the phosphorylation of AKT-S473 is expressed as the number of green dots per 20µm of dendritic length. Specifically, FIG. 2a shows the results for HN2209, FIG. 2b for HN2210, FIG. 2c for HN2213, FIG. 2d for HN2216, FIG. 2e for HN2304, FIG. 2f for HN2305, FIG. 2g for HN2308, FIG. 2h for HN2309, FIG. 2i for HN2313, FIG. 2j for HN2323, FIG. 2k for HN2325, and FIG. 2l for HN2329.

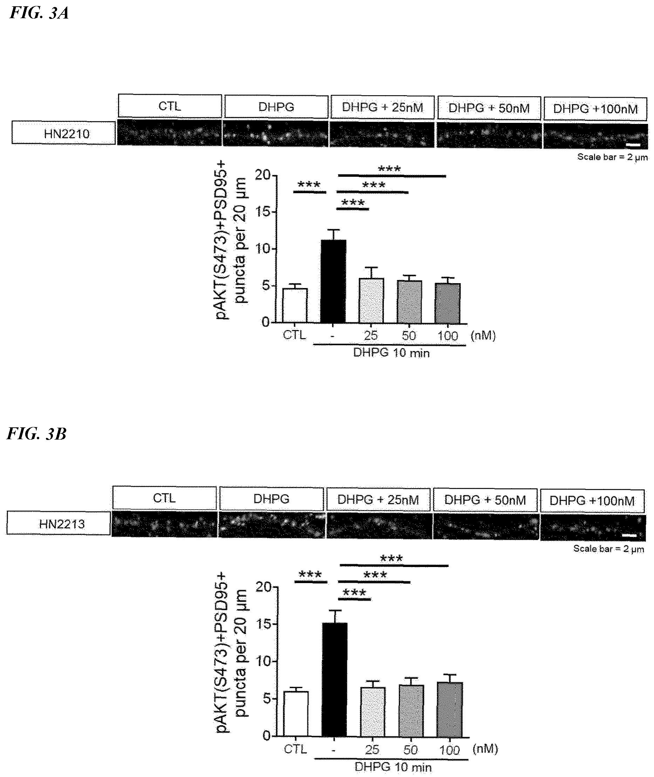

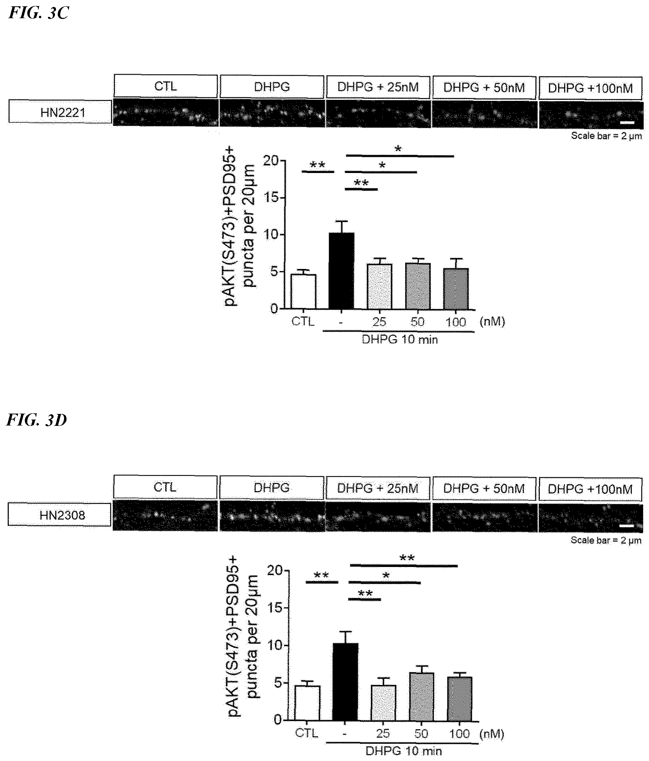

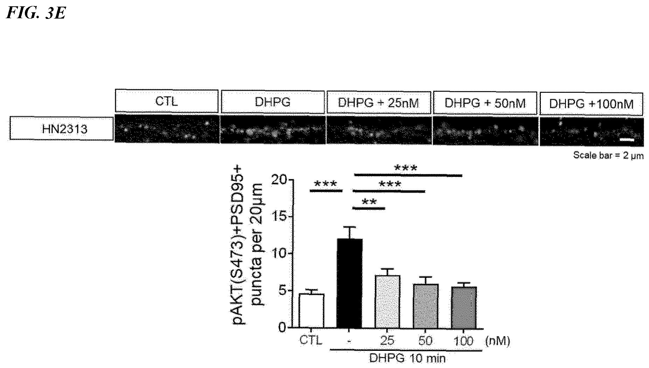

FIG. 3 shows confocal scanning microscope images (top) and the quantification graphs (bottom) evaluating the dose-dependent inhibitory activity of the present compound against mGluR1/5-dependent mTORC2 activation in dendritic spines (PSD95+) of hippocampal neurons at concentrations of 25, 50, and 100 nM. Specifically, FIG. 3a shows the results for HN2210, FIG. 3b for HN2213, FIG. 3c for HN2221, FIG. 3d for HN2308, and FIG. 3e for HN2313.

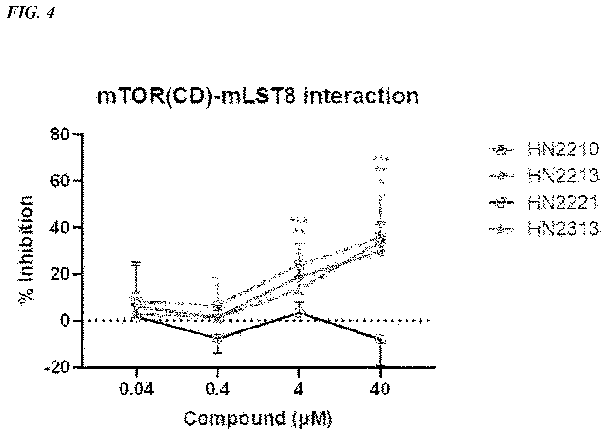

FIG. 4 shows the *in vitro* test tube assay demonstrating that the compound of the present application inhibits the binding between mTOR protein and mLST8 protein, which were expressed and isolated from E. coli and insect cells, respectively.

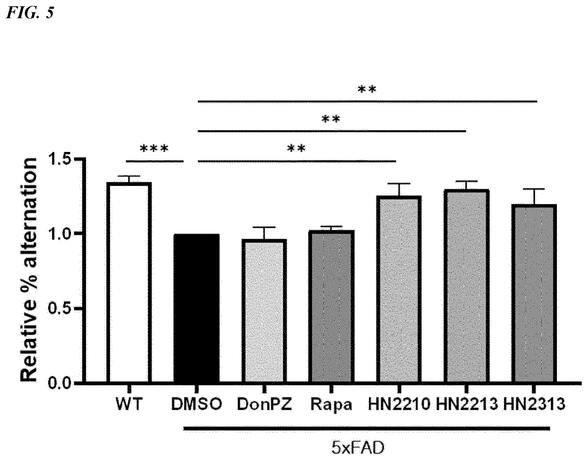

FIG. 5 shows the results of a Y-maze test conducted after administering the compound of the present application (5 mg/kg) to 5xFAD dementia model mice for two weeks. The control groups were treated for two weeks with DMSO, donepezil (1 mg/kg) which is a commercially available dementia therapeutic agent, or rapamycin (5 mg/kg) which inhibits both mTORC1 and mTORC2.

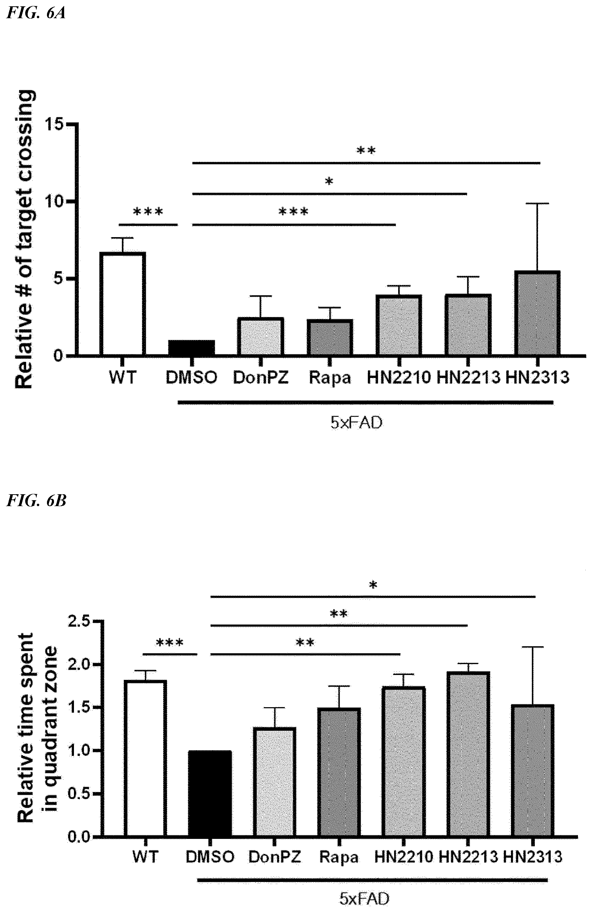

FIG. 6 shows the results of a Morris water maze test conducted after administering the compound of the present application (5 mg/kg) to 5xFAD dementia model mice for two weeks. Specifically, FIG. 6a shows the number of target crossings by the mice, and FIG. 6b shows the time spent in quadrant zone. The control groups were treated for two weeks with DMSO, donepezil (1 mg/kg) which is a commercially available dementia therapeutic agent, or rapamycin (5 mg/kg) which inhibits both mTORC1 and mTORC2.

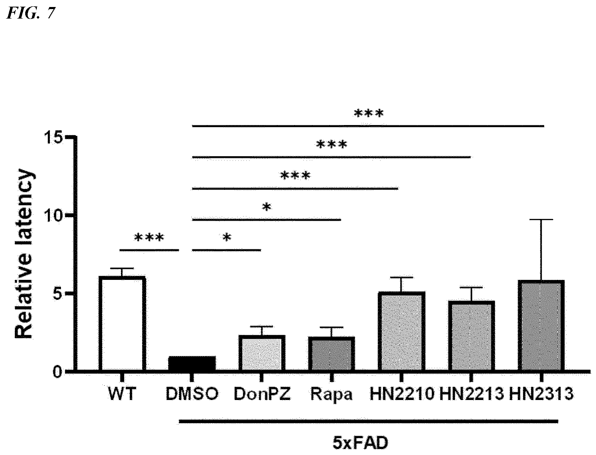

FIG. 7 shows the results of a passive avoidance test conducted after administering the compound of the present application (5 mg/kg) to 5xFAD dementia model mice for two weeks. The control groups were treated for two weeks with DMSO, donepezil (1 mg/kg) which is a commercially available dementia therapeutic agent, or rapamycin (5 mg/kg) which inhibits both mTORC1 and mTORC2.

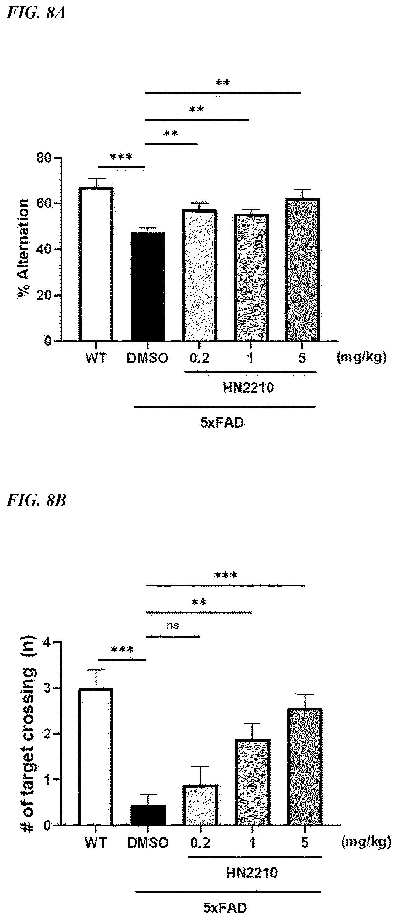

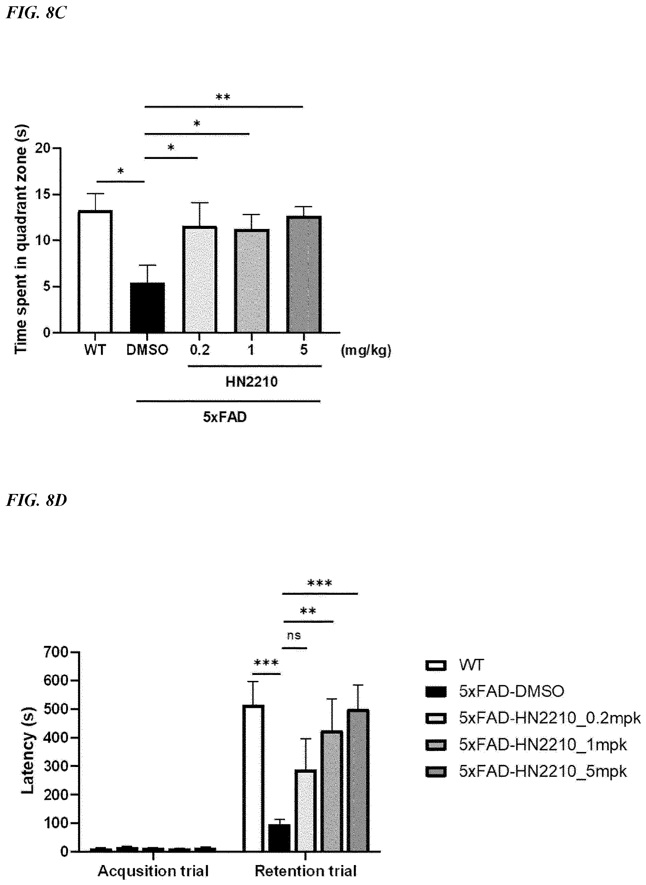

FIG. 8 shows the results of Y-maze, Morris water maze, and passive avoidance tests conducted after administering the compound of the present application to 5xFAD dementia model mice at doses of 0.2 mg/kg, 1 mg/kg, and 5 mg/kg for two weeks. Specifically, FIG. 8a shows the Y-maze test results, FIG. 8b shows the number of target crossings in the Morris water maze, FIG. 8c shows the time spent in quadrant zone during the Morris water maze, and FIG. 8d shows the results of the passive avoidance test.

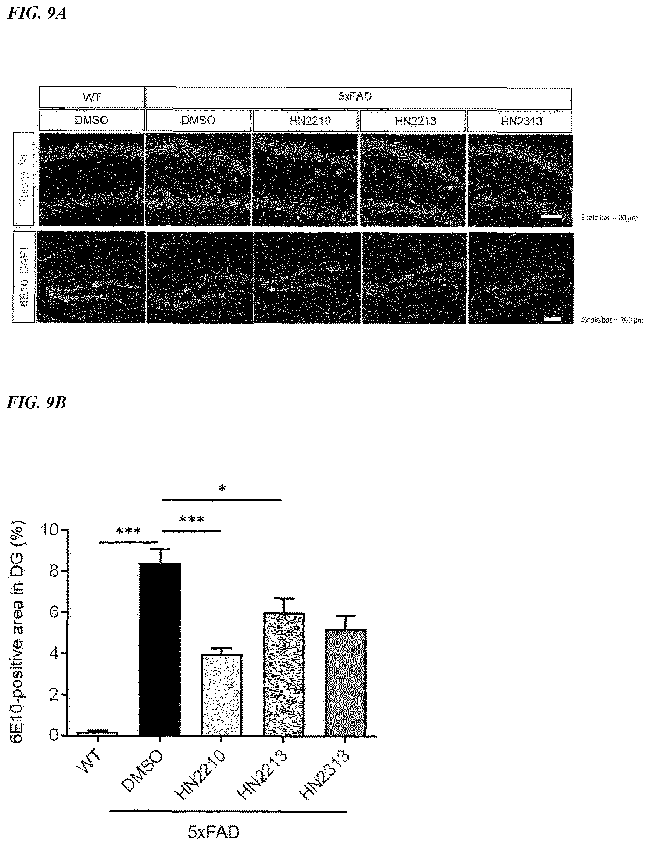

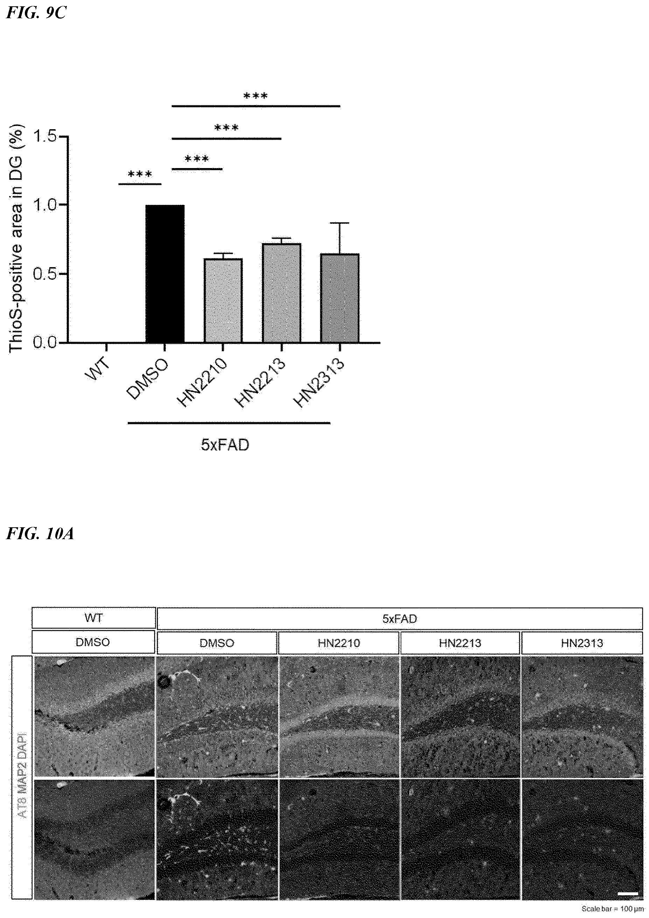

FIG. 9 is a diagram demonstrating the amyloid plaque reduction effect in brain tissues of 5xFAD dementia model mice treated with the compound of the present application (5 mg/kg) for two weeks. Specifically, FIG. 9a displays confocal microscopy images stained with the 6E10 antibody or Thioflavin-S to visualize the hippocampal region with the highest amyloid plaque accumulation, confirming the amyloid plaque clearance effect of the compound of the present application. FIG. 9b is a graph that quantified the 6E10 antibody-positive signal areas in the dentate gyrus (DG), and FIG. 9c is a graph that quantified the Thioflavin-S positive signal areas in the same region.

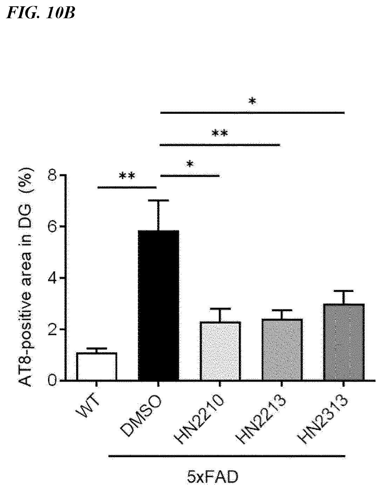

FIG. 10 is a diagram demonstrating the tau aggregate removal effect in brain tissues of 5xFAD dementia model mice treated with the compound of the present application (5 mg/kg) for two weeks. Specifically, FIG. 10a displays a confocal microscope image of the hippocampal region with the highest neurofibrillary tangles (NFT), stained with the hyperphosphorylated Tau antibody (AT8) (phospho-Tau in green, MAP2-positive neuron in red, and DAPI for nuclei), demonstrating the tau aggregation clearance effect of the compound of the present application. FIG. 10b is a graph that quantified the AT8 antibody-positive signal areas in the dentate gyrus region.

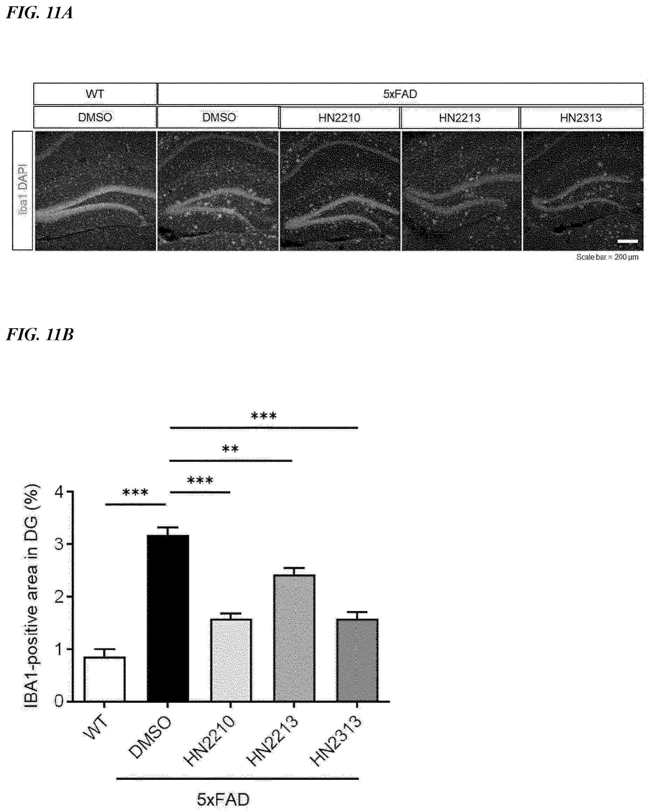

FIG. 11 is a diagram demonstrating the anti-inflammatory effect in brain tissues of 5xFAD dementia model mice treated with the compound of the present application (5 mg/kg) for two weeks. Specifically, FIG. 11a displays confocal microscopy images stained with the IBA-1 antibody, which labels activated microglia, demonstrating the reduction of neuroinflammation induced by the compound of the present application. FIG. 11b is a graph that quantified the IBA-1 positive signal areas in the dentate gyrus region.

### DETAILED DESCRIPTION OF THE EMBODIMENTS

Hereinafter, the present invention will be described in more detail with reference to the examples. However, the following examples are for illustrative purposes only, and are not intended to limit the scope of the present invention.

### <EXAMPLE>

### General Process of Compound Synthesis and NMR Analysis

### 1. Analytical Device

Devices used for confirmation of structures of the products obtained in this experiment are as follows. For nuclear magnetic resonance spectrum (¹H NMR), Bruker Magnet System 500'54 Ascend and JEOL JNM-ECZ500R were used, and as a solvent, CDCl₃, MeOH-d₄, or DMSO-d₆ was used.

### 2. General Process

### General Preparation Method 1: General preparation process of Methyl 2-bromomethyl benzoate derivative (2)

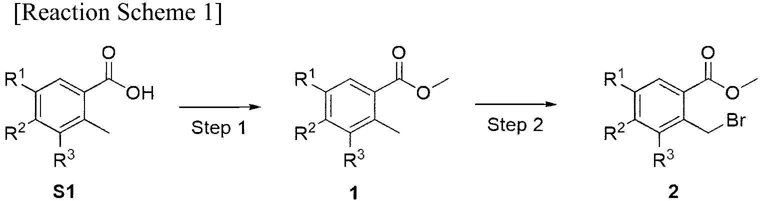

### Step 1: Preparation of Methyl 2-methylbenzoate compound 1

2-Methylbenzoic acid **S1** (1 eq.) was dissolved in DMF, and then K₂CO₃ (1.5 eq.) and MeI (1.5 eq.) were added thereto. The reaction mixture was stirred at room temperature till the reaction is complete, checked by TLC. H₂O was added to the reaction mixture to complete the reaction, and the resultant mixture was extracted with CH₂Cl₂. The organic layer was washed with H₂O and then dried over anhydrous MgSO₄. After removing the solvent under vacuum, the product was purified by column chromatography using 5% EtOAc in n-hexane to obtain the target compound **1.**

### Step 2: Preparation of Methyl 2-bromomethyl benzoate compound 2

Methyl 2-methylbenzoate 1 (1 eq.) prepared in step 1 was dissolved in 1,2-dichloroethane (usually CCl₄ can also be used), and then N-bromosuccinimide (1.1 eq.) was added thereto. The reaction mixture was stirred under reflux conditions till the reaction is complete, checked by TLC. The reaction mixture was cooled and extracted with CH₂Cl₂. The organic layer was washed with H₂O and then dried over anhydrous MgSO₄. After removing the solvent under vacuum, the product was purified by column chromatography using 5% EtOAc in *n*-hexane to obtain the target compound **2.**

### General Preparation Method 2: General preparation process of dibenzo[b,e]oxeoin-11(6H)-one derivative (6)

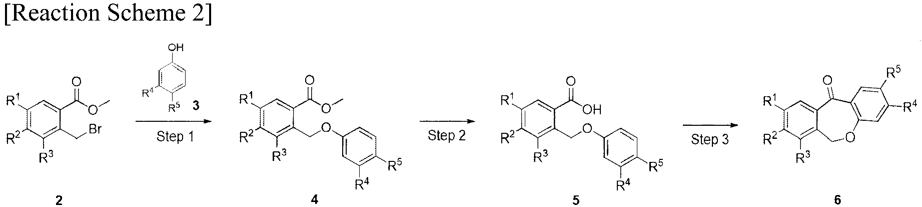

### Step 1: Preparation of Methyl 2-(phenoxymethyl)benzoate compound 4

Methyl 2-bromomethyl benzoate **2** (1 eq.) prepared in General Preparation Method 1 was dissolved in DMF, and then phenol compound **3** (1.5 eq.) and K₂CO₃ (2 eq.) were added thereto. The reaction mixture was stirred at room temperature till the reaction is complete, checked by TLC. H₂O was added to the reaction mixture to complete the reaction, and the resultant mixture was extracted with EtOAc. The organic layer was washed with H₂O and then dried over anhydrous MgSO₄. After removing the solvent under vacuum, the product was purified by column chromatography using 5~10% EtOAc solvent in n-hexane to obtain the target compound **4.**

### Step 2: Preparation of 2-(Phenoxymethyl)benzoic acid compound 5

Methyl 2-(phenoxymethyl)benzoate **4** (1 eq.) prepared in step 1 was dissolved in MeOH: THF: H₂O (1:1:1), and then KOH (4 eq.) was added thereto. The reaction mixture was stirred under reflux conditions till the reaction is complete, checked by TLC. The reaction mixture was cooled and the solvent was removed under vacuum, and then extracted with CH₂Cl₂. The organic layer was washed with H₂O and 1N HCl and then dried over anhydrous MgSO₄. After removing the solvent under vacuum, the product was purified by column chromatography using 5%MeOH solvent in CH₂Cl₂ to obtain the target compound **5.**

### Step 3: Preparation of dibenzo[b,e]oxeoin-11(6H)-one compound 6

2-(Phenoxymethyl)benzoic acid **5** (1 eq.) prepared in step 2 was dissolved in CH₂Cl₂, and then BF₃·OEt₂ (0.1 eq.) and trifluoroacetic anhydride (3 eq.) were added thereto. The reaction mixture was stirred under reflux conditions till the reaction is complete, checked by TLC. The reaction mixture was cooled and then NaHCO₃ was added to complete the reaction, and the resultant mixture was extracted with CH₂Cl₂. The organic layer was washed with H₂O and then dried over anhydrous MgSO₄. After removing the solvent under vacuum, the product was purified by column chromatography to obtain the target compound **6.**

### General Preparation Method 3: General preparation process of dibenzo[b,e]thiepin-11(6H)-one derivative (10)

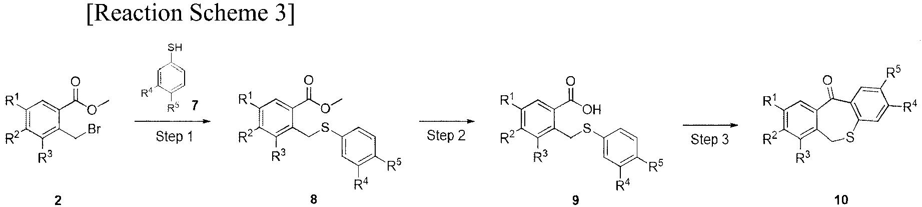

### Step 1: Preparation of methyl 2-((phenylthio)methyl)benzoate compound 8

Methyl 2-bromomethyl benzoate **2** (1 eq.) prepared in General Preparation Method 1 was dissolved in DMF, and then thiol compound 7 (1 eq.) and K₂CO₃ (2 eq.) were added thereto. The reaction mixture was stirred at room temperature till the reaction is complete, checked by TLC. H₂O was added to the reaction mixture to complete the reaction, and the resultant mixture was extracted with EtOAc. The organic layer was washed with water and then dried over anhydrous MgSO₄. After removing the solvent under vacuum, the product was purified by column chromatography using 10~15% EtOAc solvent in n-hexane to obtain the target compound **8.**

### Step 2: Preparation of 2-((Phenylthio)methyl)benzoic acid compound 9

Methyl 2-((phenylthiol)methyl)benzoate **8** (1 eq.) prepared in step 1 was dissolved in MeOH: THF: H₂O (1:1:1), and then KOH (4 eq.) was added thereto. The reaction mixture was stirred under reflux conditions till the reaction is complete, checked by TLC. The reaction mixture was cooled and the solvent was removed under vacuum, and then extracted with CH₂Cl₂. The organic layer was washed with H₂O and 1N HCl and then dried over anhydrous MgSO₄. After removing the solvent under vacuum, the product was purified by column chromatography using 5% MeOH solvent in CH₂Cl₂ to obtain the target compound **9.**

### Step 3: Preparation of dibenzo[b,e]thiepin-11(6H)-one compound 10

2-((Phenylthio)methyl)benzoic acid **9** (1 eq.) prepared in step 2 was dissolved in CH₂Cl₂, and then BF₃·OEt₂ (0.1 eq.) and trifluoroacetic anhydride (3 eq.) were added thereto. The reaction mixture was stirred under reflux conditions till the reaction is complete, checked by TLC. The reaction mixture was cooled and then NaHCO₃ was added to complete the reaction, and the resultant mixture was extracted with CH₂Cl₂. The organic layer was washed with H₂O and then dried over anhydrous MgSO₄. After removing the solvent under vacuum, the product was purified by column chromatography to obtain the target compound **10.**

### General Preparation Method 4: General preparation process of 10,11-dihydro-5H-dibenzo[a,d][7]annulen-5-one derivative

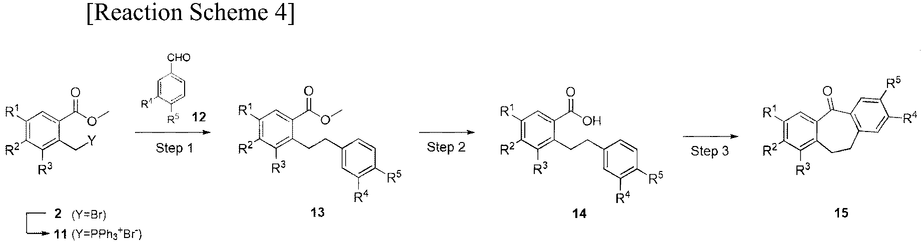

### Step 1: Preparation of methyl 2-phenethylbenzoate compound 13

Methyl 2-bromomethyl benzoate **2** (1 eq.) prepared in General Preparation Method 1 and PPh3 (1.1 eq.) were dissolved in acetone, and stirred overnight under reflux conditions. The reaction mixture was cooled, the precipitate was filtered, and then washed with acetone to obtain benzyltriphenylphosphonium bromide salt **11,** which was used in the next reaction without further purification.

60% NaH (2 eq.) was added to a THF solution of benzyltriphenylphosphonium bromide salt **11** (1.3 eq.) at 0°C. The mixture was stirred for 1 hour, and then benzaldehyde **12** (1 eq.) was added thereto. The reaction mixture was stirred at room temperature till the reaction is complete, checked by TLC. Saturated NH₄Cl aqueous solution was added to complete the reaction, and the resultant mixture was extracted with EtOAc. The organic layer was washed with water and then dried over anhydrous MgSO₄. After removing the solvent under vacuum, the next reaction was carried out without purification. The concentrated mixture was dissolved in MeOH and a catalytic amount of Pd/C was added. The mixture was stirred for 3 hours by purging H₂ gas, and then filtered through Cellite to remove the catalyst. The reaction mixture was concentrated under vacuum to remove the solvent. The product was purified by column chromatography to obtain the desired methyl 2-phenethylbenzoate compound **13.**

### Step 2: Preparation of 2-Phenethylbenzoic acid compound 14

Methyl 2-phenethylbenzoate compound **13** (1 eq.) prepared in step 1 was dissolved in MeOH: THF: H₂O (1:1:1), and then KOH (4 eq.) was added thereto. The reaction mixture was stirred under reflux conditions till the reaction is complete, checked by TLC. The reaction mixture was cooled and the solvent was removed under vacuum, and then extracted with CH₂Cl₂. The organic layer was washed with H₂O and 1N HCl and then dried over anhydrous MgSO₄. After removing the solvent under vacuum, the product was purified by column chromatography using 5% MeOH solvent in CH₂Cl₂ to obtain the target compound **14.**

### Step 3: Preparation of 10,11-Dihydro-5H-dibenzo[a,d][7]annulen-5-one compound 15

2-Phenylethylbenzoic acid **14** (1 eq.) prepared in step 2 was dissolved in CH₂Cl₂, and then BF₃·OEt₂ (0.1 eq.) and trifluoroacetic anhydride (3 eq.) were added thereto. The reaction mixture was stirred under reflux conditions till the reaction is complete, checked by TLC. The reaction mixture was cooled and then NaHCO₃ was added to complete the reaction, and the resultant mixture was extracted with CH₂Cl₂. The organic layer was washed with H₂O and then dried over anhydrous MgSO₄. After removing the solvent under vacuum, the product was purified by column chromatography to obtain the target compound **15.**

### General Preparation Method 5: Nucleophilic aromatic substitution

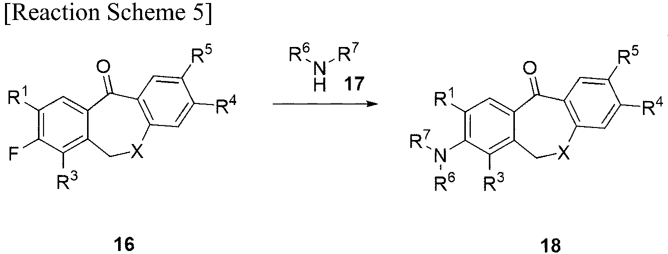

p-Fluorobenzophenone compound of Chemical Formula **16** (1 eq.) prepared in General Preparation Method 2-4, K₂CO₃ (1 eq.), tetrabutylammonium bromide (0.5 eq.), and amine compound of Chemical Formula **17** (3~5 eq.) were added to DMSO. The reaction mixture was stirred at 90°C till the reaction is complete, checked by TLC. The reaction mixture was cooled and then water was added to complete the reaction, and the resultant mixture was extracted with CH₂Cl₂. The organic layer was washed with water and then dried over anhydrous Na₂SO₄. After removing the solvent under vacuum, the product was purified by column chromatography to obtain the target compound **18.**

### General Preparation Method 6: Suzuki cross-coupling reaction

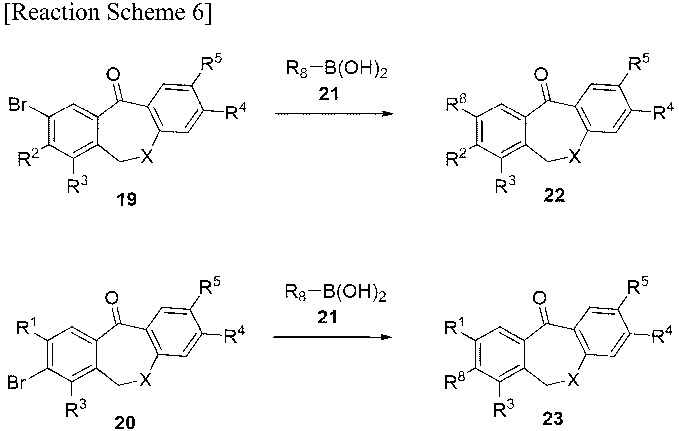

The bromobenzophenone compounds of Chemical Formulas **19~20** (1 eq.) prepared in General Preparation Methods 2~4, boronic acid of Chemical Formula **21** (1.5 eq.), and K₂CO₃ (4 eq.) were dissolved in aq. DMF (H₂O : DMF = 1 : 5), and then Pd(PPh₃)₄ (0.1 eq.) was added thereto. The reaction mixture was stirred at 90°C till the reaction is complete, checked by TLC. The reaction mixture was cooled and then H₂O was added to complete the reaction, and the resultant mixture was extracted with ethyl acetate. The organic layer was washed with H₂O and then dried over anhydrous MgSO₄. After removing the solvent under vacuum, the product was purified by column chromatography to obtain the target compounds **22~23.**

### General Preparation Method 7: General preparation of 5,6-dihydro-11H-dibenzo[b,e]azepin-11-one

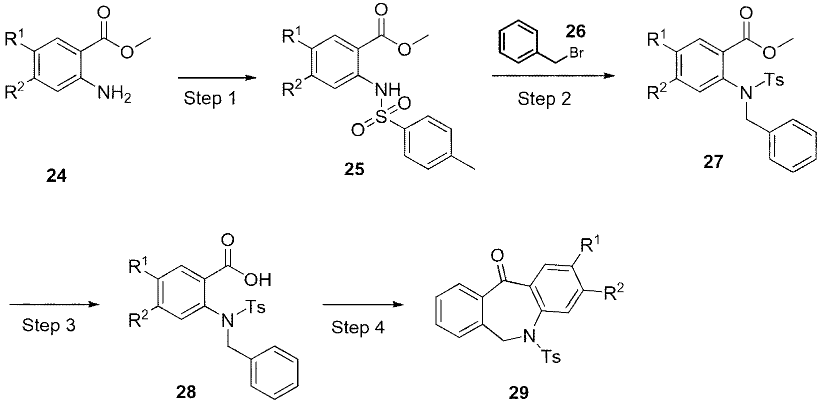

### Step 1: Preparation of methyl 2-((4-methylphenyl)sulfonamido)benzoate compound 25

Methyl 2-aminobenzoate **24** (1 eq.) was dissolved in 1,2-dichloroethane, and then p-TsCl (1.2 eq.) and pyridine (3 eq.) were added thereto. The reaction mixture was stirred at room temperature till the reaction is complete, checked by TLC. H₂O was added to the reaction mixture to complete the reaction, and the resultant mixture was extracted with CH₂Cl₂. The organic layer was washed with H₂O and then dried over anhydrous Na₂SO₄. After removing the solvent under vacuum, the product was purified by column chromatography using 5% EtOAc and 50% methylene chloride solvent in n-hexane to obtain the target compound **25.**

### Step 2: Preparation of methyl 2-((N-benzyl-4-methylphenyl)sulfonamido)benzoate compound 27

In a flame-dried round bottom flask, methyl 2-((4-methylphenyl)sulfonamido)benzoate **25** (1 eq.) prepared in step 1 was dissolved in DMF, 60% NaH (2 eq.) was added at 0°C, and stirred for 30 minutes. The mixture was stirred for 30 minutes, and then benzyl bromide **26** (2 eq.) was added thereto. The reaction mixture was stirred at room temperature till the reaction is complete, checked by TLC. H₂O was added to the reaction mixture to complete the reaction, and the resultant mixture was extracted with EtOAc. The organic layer was washed with H₂O and 1N HCl and then dried over anhydrous Na₂SO₄. After removing the solvent under vacuum, the product was purified by column chromatography using 25% EtOAc solvent in n-hexane to obtain the target compound **27.**

### Step 3: Preparation of 2-((N-benzyl-4-methylphenyl)sulfonamido)benzoic acid compound 28

Methyl 2-((N-benzyl-4-methylphenyl)sulfonamido)benzoate **27** (1 eq.) prepared in step 2 was dissolved in MeOH : THF : H₂O (1 : 1 : 1), and then KOH (4 eq.) was added thereto. The reaction mixture was stirred under reflux conditions till the reaction is complete, checked by TLC. The reaction mixture was cooled and the solvent was removed under vacuum, and then extracted with CH₂Cl₂. The organic layer was washed with H₂O and 1N HCl and then dried over anhydrous Na₂SO₄. After removing the solvent under vacuum, the product was purified by column chromatography using 5% MeOH solvent in CH₂Cl₂ to obtain the target compound **28.**

### Step 4: Preparation of 5,6-dihydro-11H-dibenzo[b,e]azepin-11-one compound 29

2-((N-benzyl-4-methylphenyl)sulfonamido)benzoic acid **28** (1 eq.) prepared in step 3 was dissolved in CH₂Cl₂, and then BF₃·OEt₂ (0.1 eq.), trifluoroacetic anhydride (3 eq.) were added thereto. The reaction mixture was stirred under reflux conditions till the reaction is complete, checked by TLC. The reaction mixture was cooled and then NaHCO₃ was added thereto to complete the reaction, and the resultant mixture was extracted with CH₂Cl₂. The organic layer was washed with H₂O and then dried over anhydrous Na₂SO₄. After removing the solvent under vacuum, the product was purified by column chromatography using 40% EtOAc solvent in n-hexane to obtain the target compound **29.**

### <Example 1> Preparation of 9-fluoro[2]benzoxepino[3,4-f]-1,3-benzodioxol-11(6H)-one (HN2203)

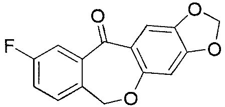

### Step 1: Preparation of methyl 5-fluoro-2-methylbenzoate

According to the preparation method of step 1 of General Preparation Method 1, 5-fluoro-2-methylbenzoic acid (100 mg, 0.648 mmol) and iodomethane (0.05 mL, 0.778 mmol) were allowed to react, and then the reaction mixture was purified by column chromatography using 5% ethyl acetate solvent in n-hexane to obtain methyl 5-fluoro-2-methylbenzoate (92.6 mg, 85%).

### Step 2: Preparation of methyl 2-bromomethyl-5-fluorobenzoate

According to the preparation method of step 2 of General Preparation Method 1, methyl 5-fluoro-2-methylbenzoate (30 mg, 0.178 mmol) prepared in step 1 was reacted with N-bromosuccinimide(34.7 mg, 0.196 mmol), and then the reaction mixture was purified by column chromatography using 5% ethyl acetate solvent in n-hexane to obtain methyl 2-bromomethyl-5-fluorobenzoate (41 mg, 93%).

### Step 3: Preparation of methyl 2-[(1,3-benzodioxol-5-yloxy)methyl]-5-fluorobenzoate

According to the preparation method of step 1 of General Preparation Method 2, methyl 2-bromomethyl-5-fluorobenzoate(123.1 mg, 0.498 mmol) prepared in step 2 and sesamol(103.2 mg, 0.747 mmol) were allowed to react, and then the reaction mixture was purified by column chromatography using 10% ethyl acetate solvent in n-hexane to obtain methyl 2-[(1,3-benzodioxol-5-yloxy)methyl]-5-fluorobenzoate(87.3 mg, 58%).

### Step 4: Preparation of 2-[(1,3-benzodioxol-5-yloxy)methyl]-5-fluorobenzoic acid

According to the preparation method of step 2 of General Preparation Method 2, methyl 2-[(1,3-benzodioxol-5-yloxy)methyl]-5-fluorobenzoate(67 mg, 0.22 mmol) prepared in step 3 was reacted to obtain 2-[(1,3-benzodioxol-5-yloxy)methyl]-5-fluorobenzoic acid(59.5 mg, 93%).

### Step 5: Preparation of 9-fluoro[2]benzoxepino[3,4-f]-1,3-benzodioxol-11(6H)-one

According to the preparation method of step 3 of General Preparation Method 2, 2-[(1,3-benzodioxol-5-yloxy)methyl]-5-fluorobenzoic acid(30 mg, 0.103 mmol) prepared in step 4 was reacted, and then the reaction mixture was purified by column chromatography using 25% ethyl acetate solvent in n-hexane to obtain 9-fluoro[2]benzoxepino[3,4-f]-1,3-benzodioxol-11(6H)-one(26.9 mg, 96%).
¹H NMR (500 MHz, CDCl₃) δ 7.65 (s, 1H), 7.63 (d, *J =* 2.4 Hz, 1H), 7.33 (dd, *J* = 8.2, 5.2 Hz, 1H), 7.22 (td, *J* = 8.1, 2.5 Hz, 1H), 6.49 (s, 1H), 6.02 (s, 2H), 5.13 (s, 2H)

### <Example 2> Preparation of [2]benzoxepino[3,4-f]-1,3-benzodioxol-11(6H)-one (HN2204)

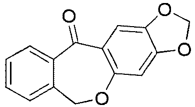

### Step 1: Preparation of methyl 2-[(1,3-benzodioxol-5-yloxy)methyl]benzoate

According to the preparation method of step 2 of General Preparation Method 2, methyl 2-(bromomethyl)benzoate(50 mg, 0.218 mmol) and sesamol(45.3 mg, 0.327 mmol) were allowed to react, and then the reaction mixture was purified by column chromatography using 10% ethyl acetate solvent in n-hexane to obtain methyl 2-[(1,3-benzodioxol-5-yloxy)methyl]benzoate (37.4 mg, 59%).

### Step 2: Preparation of 2-[(1,3-benzodioxol-5-yloxy)methyl]benzoic acid

According to the preparation method of step 1 of General Preparation Method 2, methyl 2-[(1,3-benzodioxol-5-yloxy)methyl]benzoate (75 mg, 0.261 mmol) prepared in step 1 was reacted to obtain 2-[(1,3-benzodioxol-5-yloxy)methyl]benzoic acid (47.4 mg, 67%).

### Step 3: Preparation of [2]benzoxepino[3,4-f]-1,3-benzodioxol-11(6H)-one

According to the preparation method of step 3 of General Preparation Method 2, 2-[(1,3-benzodioxol-5-yloxy)methyl]benzoic acid(29.6 mg, 0.108 mmol) prepared in step 2 was reacted, and then the reaction mixture was purified by column chromatography using methylene chloride solvent to obtain [2]benzoxepino[3,4-f]-1,3-benzodioxol-11(6H)-one (28.8 mg, 98%).
¹H NMR (500 MHz, CDCl₃) δ 7.94 (d, *J* = 7.7 Hz, 1H), 7.68 (s, 1H), 7.54 (t, *J =* 7.2 Hz, 1H), 7.47 (t, *J =* 7.6 Hz, 1H), 7.33 (d, *J =* 7.4 Hz, 1H), 6.50 (s, 1H), 6.01 (s, 2H), 5.16 (s, 2H)

### <Example 3> Preparation of 8,9-difluoro[2]benzoxepino[3,4-f]-1,3-benzodioxol-11(6H)-one (HN2208)

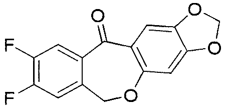

### Step 1: Preparation of methyl 4,5-difluoro-2-methylbenzoate

According to the preparation method of step 1 of General Preparation Method 1, 4,5-difluoro-2-methylbenzoic acid(100 mg, 0.581 mmol) and iodomethane(0.043 mL, 0.697 mmol) were allowed to react, and then the reaction mixture was purified by column chromatography using 5% ethyl acetate solvent in n-hexane to obtain methyl 4,5-difluoro-2-methylbenzoate(109 mg, 99%).

### Step 2: Preparation of methyl 2-bromomethyl-4,5-difluorobenzoate

According to the preparation method of step 2 of General Preparation Method 1, methyl 4,5-difluoro-2-methylbenzoate(100 mg, 0.537 mmol) prepared in step 1 and N-bromosuccinimide(104.6 mg, 0.590 mmol) were allowed to react, and then the reaction mixture was purified by column chromatography using 5% ethyl acetate solvent in n-hexane to obtain methyl 2-bromomethyl-4,5-difluorobenzoate(114.3 mg, 80%).

### Step 3: Preparation of methyl 2-[(1,3-benzodioxol-5-yloxy)methyl]-4,5-difluorobenzoate

According to the preparation method of step 1 of General Preparation Method 2, methyl 2-bromomethyl-4,5-difluorobenzoate(100 mg, 0.377 mmol) and sesamol(62.4 mg, 0.452 mmol) were allowed to react, and then the reaction mixture was purified by column chromatography using 10% ethyl acetate solvent in n-hexane to obtain methyl 2-[(1,3-benzodioxol-5-yloxy)methyl]-4,5-difluorobenzoate (54.5 mg, 45%).

### Step 4: Preparation of 2-[(1,3-benzodioxol-5-yloxy)methyl]-4,5-difluorobenzoic acid

According to the preparation method of step 2 of General Preparation Method 2, methyl 2-[(1,3-benzodioxol-5-yloxy)methyl]-4,5-difluorobenzoate (30 mg, 0.093 mmol) prepared in step 3 was reacted to obtain 2-[(1,3-benzodioxol-5-yloxy)methyl]-4,5-difluorobenzoic acid (20.8 mg, 73%).

### Step 5: Preparation of 8,9-difluoro[2]benzoxepino[3,4-f]-1,3-benzodioxol-11(6H)-one

According to the preparation method of step 3 of General Preparation Method 2, 2-[(1,3-benzodioxol-5-yloxy)methyl]-4,5-difluorobenzoic acid(40 mg, 0.129 mmol) prepared in step 4 was reacted, and then the reaction mixture was purified by column chromatography using methylene chloride solvent to obtain 8,9-difluoro[2]benzoxepino[3,4-f]-1,3-benzodioxol-11(6H)-one (21.3 mg, 57%).
¹H NMR (500 MHz, CDCl₃) δ 7.82 (dd, *J =* 10.8, 8.0 Hz, 1H), 7.65 (s, 1H), 7.16 (dd, *J =* 9.5, 7.2 Hz, 1H), 6.50 (s, 1H), 6.03 (s, 2H), 5.09 (s, 2H)

### <Example 4> Preparation of 8-(dimethylamino)-9-fluoro[2]benzoxepino[3,4-f]-1,3-benzodioxol-11(6H)-one (HN2209)

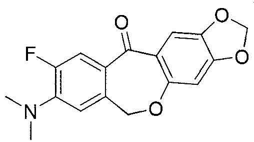

According to the preparation method of General Preparation Method 5, 8,9-difluoro[2]benzoxepino[3,4-f]-1,3-benzodioxol-11(6H)-one(20 mg, 0.068 mmol) of Example 3 and dimethylamine(0.1 mL) were allowed to react, and then the reaction mixture was purified by column chromatography using methylene chloride solvent to obtain 8-(dimethylamino)-9-fluoro[2]benzoxepino[3,4-f]-1,3-benzodioxol-11(6H)-one(18.4 mg, 86%).
¹H NMR (500 MHz, DMSO-d₆) δ 7.57 (d, *J =* 15.4 Hz, 1H), 7.52 (s, 1H), 6.97 (d, *J =* 8.9 Hz, 1H), 6.71 (s, 1H), 6.13 (s, 2H), 5.18 (s, 2H), 3.00 (d, *J =* 1.3 Hz, 6H)

### <Example 5> Preparation of 8-(morpholino)-9-fluoro[2]benzoxepino[3,4-f]-1,3-benzodioxol-11(6H)-one (HN2210)

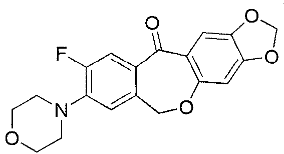

According to the preparation method of General Preparation Method 5, 8,9-difluoro[2]benzoxepino[3,4-f]-1,3-benzodioxol-11(6H)-one(20 mg, 0.068 mmol) of Example 3 and morpholine(0.03 mL, 0.340 mmol) were allowed to react, and then the reaction mixture was purified by column chromatography using methylene chloride solvent to obtain 8-(morpholino)-9-fluoro[2]benzoxepino[3,4-f]-1,3-benzodioxol-11(6H)-one(24.3 mg, 99%).
¹H NMR (500 MHz, DMSO-d₆) δ 7.59 (d, *J =* 14.4 Hz, 1H), 7.51 (s, 1H), 7.18 (d, *J* = 8.5 Hz, 1H), 6.71 (s, 1H), 6.13 (s, 2H), 5.21 (s, 2H), 3.79 - 3.74 (m, 4H), 3.23 - 3.18 (m, 4H)

### <Example 6> Preparation of 8-(piperidinyl)-9-fluoro[2]benzoxepino[3,4-f]-1,3-benzodioxol-11(6H)-one (HN2211)

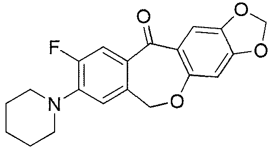

According to the preparation method of General Preparation Method 5, 8,9-difluoro[2]benzoxepino[3,4-f]-1,3-benzodioxol-11(6H)-one(20 mg, 0.068 mmol) of Example 3 and piperidine(0.03 mL, 0.34 mmol) were allowed to react, and then the reaction mixture was purified by column chromatography using methylene chloride solvent to obtain 8-(piperidinyl)-9-fluoro[2]benzoxepino[3,4-f]-1,3-benzodioxol-11(6H)-one(9.0 mg, 37%).
¹H NMR (500 MHz, MeOD) δ 7.64 (s, 1H), 7.61 (s, 1H), 7.01 (d, *J* = 8.3 Hz, 1H), 6.57 (s, 1H), 6.06 (s, 2H), 5.15 (s, 2H), 3.26 (s, 2H), 3.18 - 3.15 (m, 8H)

### <Example 7> Preparation of 8-(phenylpiperazine-1-yl)-9-fluoro[2]benzoxepino[3,4-f]-1,3-benzodioxol-11(6H)-one (HN2212)

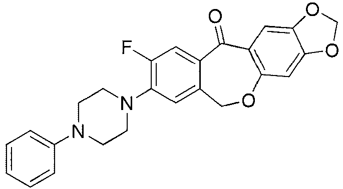

According to the preparation method of General Preparation Method 5, 8,9-difluoro[2]benzoxepino[3,4-f]-1,3-benzodioxol-11(6H)-one(30 mg, 0.103 mmol) of Example 3 and 1-phenylpiperidine(0.047 mL, 0.310 mmol) were allowed to react, and then the reaction mixture was purified by column chromatography using 5-9% ethyl acetate and 50% methylene chloride solvent in n-hexane to obtain 8-(phenylpiperazine-1-yl)-9-fluoro[2]benzoxepino[3,4-f]-1,3-benzodioxol-11(6H)-one(43.6 mg, 97%).
¹H NMR (500 MHz, DMSO-d₆) δ 7.62 (d, *J* = 14.3 Hz, 1H), 7.52 (s, 1H), 7.26 (dd, *J* = 16.1, 8.0 Hz, 3H), 7.03 (d, *J =* 8.1 Hz, 2H), 6.84 (s, 1H), 6.73 (s, 1H), 6.14 (s, 2H), 5.23 (s, 2H), 3.39 (d, *J* = 3.2 Hz, 4H)

### <Example 8> Preparation of 8-(benzylamino)-9-fluoro[2]benzoxepino[3,4-f]-1,3-benzodioxol-11(6H)-one (HN2213)

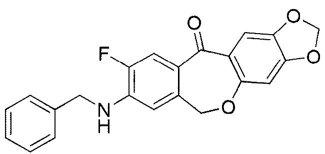

According to the preparation method of General Preparation Method 5, 8,9-difluoro[2]benzoxepino[3,4-f]-1,3-benzodioxol-11(6H)-one(30 mg, 0.103 mmol) of Example 3 and benzylamine(0.056 mL, 0.517 mmol) were allowed to react, and then the reaction mixture was purified by column chromatography using 5% ethyl acetate and 50% methylene chloride in n-hexane to obtain 8-(benzylamino)-9-fluoro[2]benzoxepino[3,4-f]-1,3-benzodioxol-11(6H)-one(32.2 mg, 82%).
¹H NMR (500 MHz, DMSO-d₆) δ 7.58 (d, *J =* 13.1 Hz, 1H), 7.52 (s, 1H), 7.40 (d, *J =* 7.0 Hz, 2H), 7.35 (t, *J* = 7.7 Hz, 2H), 7.26 (s, 1H), 6.73 (d, *J* = 8.3 Hz, 1H), 6.66 (s, 1H), 6.12 (s, 2H), 5.06 (s, 2H), 4.49 (d, *J* = 6.3 Hz, 2H)

### <Example 9> Preparation of 9-(4-chlorophenyl)[2]benzoxepino[3,4-f]-1,3-benzodioxol-11(6H)-one (HN2214)

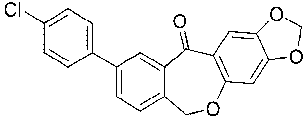

### Step 1: Preparation of methyl 5-bromo-2-methylbenzoate

According to the preparation method of step 1 of General Preparation Method 1, 5-bromo-2-methylbenzoic acid(200 mg, 0.930 mmol) and iodomethane(0.07 mL, 1.116 mmol) were allowed to react, and then the reaction mixture was purified by column chromatography using 5% ethyl acetate solvent in n-hexane to obtain methyl 5-bromo-2-methylbenzoate(218.7 mg, 98%).

### Step 2: Preparation of methyl 2-bromomethyl-5-bromobenzoate

According to the preparation method of step 2 of General Preparation Method 1, methyl 5-bromo-2-methylbenzoate(100 mg, 0.436 mmol) prepared in step 1 and N-bromosuccinimide(85.5 mg, 0.480 mmol) were allowed to react, and then the reaction mixture was purified by column chromatography using 5% ethyl acetate solvent in n-hexane to obtain methyl 2-bromomethyl-5-bromobenzoate(120 mg, 90%).

### Step 3: Preparation of methyl 2-[(1,3-benzodioxol-5-yloxy)methyl]-5-bromobenzoate

According to the preparation method of step 1 of General Preparation Method 2, methyl 2-bromomethyl-5-bromobenzoate(120 mg, 0.389 mmol) prepared in step 2 and sesamol(64.5 mg, 0.467 mmol) were allowed to react, and then the reaction mixture was purified by column chromatography using 10% ethyl acetate solvent in n-hexane to obtain methyl 2-[(1,3-benzodioxol-5-yloxy)methyl]-5-bromobenzoate(55.2 mg, 63%).

### Step 4: Preparation of 2-[(1,3-benzodioxol-5-yloxy)methyl]-5-bromobenzoic acid

According to the preparation method of step 2 of General Preparation Method 2, methyl 2-[(1,3-benzodioxol-5-yloxy)methyl]-5-bromobenzoate(20 mg, 0.054 mmol) prepared in step 3 was reacted to obtain 2-[(1,3-benzodioxol-5-yloxy)methyl]-5-bromobenzoic acid(24.1 mg, 97%).

### Step 5: Preparation of 9-bromo[2]benzoxepino[3,4-f]-1,3-benzodioxol-11(6H)-one

According to the preparation method of step 3 of General Preparation Method 2, 2-[(1,3-benzodioxol-5-yloxy)methyl]-5-bromobenzoic acid(10 mg, 0.029 mmol) prepared in step 4 was reacted, and then the reaction mixture was purified by column chromatography using 25% ethyl acetate solvent in n-hexane to obtain 9-bromo[2]benzoxepino[3,4-f]-1,3-benzodioxol-11(6H)-one 22(9.3 mg, 80%).

### Step 6: Preparation of 9-(4-chlorophenyl)[2]benzoxepino[3,4-f]-1,3-benzodioxol-11 (6H)-one

According to the preparation method of General Preparation Method 6, 9-bromo[2]benzoxepino[3,4-f]-1,3-benzodioxol-11(6H)-one(30 mg, 0.09 mmol) prepared in step 5 and 4-chlorophenylboronic acid(21.1 mg, 0.135 mmol) were allowed to react, and then the reaction mixture was purified by column chromatography using 66% methylene chloride solvent in n-hexane. The purified compound was decanted with n-hexane to obtain 9-(4-chlorophenyl)[2]benzoxepino[3,4-f]-1,3-benzodioxol-11(6H)-one(31 mg, 94%).
¹H NMR (500 MHz, CDCl₃) δ 8.13 (d, *J* = 2.0 Hz, 1H), 7.72 (dd, *J* = 7.8, 2.0 Hz, 1H), 7.69 (s, 1H), 7.58 - 7.53 (m, 2H), 7.45 - 7.39 (m, 3H), 6.51 (s, 1H), 6.03 (s, 2H), 5.19 (s, 2H)

### <Example 10> Preparation of 9-(4-hydroxyphenyl)[2]benzoxepino[3,4-f]-1,3-benzodioxol-11(6H)-one (HN2215)

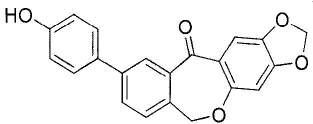

According to the preparation method of General Preparation Method 6, the compound **22** (35 mg, 0.105) prepared in the step 5 was reacted, and then the reaction mixture was purified by column chromatography using 5~20% ethyl acetate solvent in n-hexane to obtain 9-(4-hydroxyphenyl)[2]benzoxepino[3,4-f]-1,3-benzodioxol-11(6H)-one.
¹H NMR (500 MHz, DMSO-d₆) δ 9.67 (s, 1H), 7.94 (d, *J =* 2.0 Hz, 1H), 7.84 (dd, *J =* 7.8, 2.0 Hz, 1H), 7.57 (d, *J* = 7.9 Hz, 1H), 7.56 - 7.52 (m, 2H), 7.50 (s, 1H), 6.92 - 6.82 (m, 2H), 6.72 (s, 1H), 6.13 (s, 2H), 5.26 (s, 2H)

### <Example 11> Preparation of 9-(3-furanyl)[2]benzoxepino[3,4-f]-1,3-benzodioxol-11(6H)-one (HN2216)

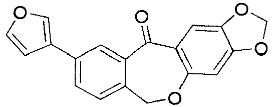

According to the preparation method of General Preparation Method 6, the compound **22** (30 mg, 0.09 mmol) prepared in step 5 of Example 9 and 3-furylboronic acid(15.1 mg, 0.135 mmol) were allowed to react, and then the reaction mixture was purified by column chromatography using 2.5% ethyl acetate and 50% methylene chloride solvent in n-hexane. The purified compound was decanted with n-hexane to obtain 9-(3-furanyl)[2]benzoxepino[3,4-f]-1,3-benzodioxol-11(6H)-one(27.7 mg, 96%).
¹H NMR (500 MHz, CDCl₃) δ 8.04 (d, *J* = 1.9 Hz, 1H), 7.80 (dd, *J* = 1.4, 0.9 Hz, 1H), 7.68 (s, 1H), 7.64 (dd, *J* = 7.7, 1.9 Hz, 1H), 7.49 (t, *J=* 1.7 Hz, 1H), 7.34 (d, *J =* 7.8 Hz, 1H), 6.75 (dd, *J* = 1.9, 0.9 Hz, 1H), 6.50 (s, 1H), 6.02 (s, 2H), 5.15 (s, 2H)

### <Example 12> Preparation of 9-(p-tolyl)[2]benzoxepino[3,4-f]-1,3-benzodioxol-11(6H)-one (HN2217)

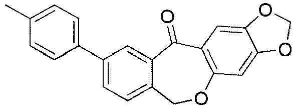

According to the preparation method of General Preparation Method 6, the compound **22** (30 mg, 0.09 mmol) prepared in step 5 of Example 9 and 4-methylphenylboronic acid(18.3 mg, 0.135 mmol) were allowed to react, and then the reaction mixture was purified by column chromatography using 66% methylene chloride solvent in n-hexane. The purified compound was decanted with n-hexane to obtain 9-(p-tolyl)[2]benzoxepino[3,4-f]-1,3-benzodioxol-11(6H)-one(24.3 mg, 78%).
¹H NMR (500 MHz, CDCl₃) δ 8.15 (d, *J =* 1.9 Hz, 1H), 7.74 (dd, *J* = 7.7, 1.9 Hz, 1H), 7.69 (s, 1H), 7.53 (d, *J =* 8.1 Hz, 2H), 7.39 (d, *J =* 7.8 Hz, 1H), 7.27 (s, 1H), 7.25 (s, 1H), 6.51 (s, 1H), 6.02 (s, 2H), 5.18 (s, 2H), 2.40 (s, 3H)

### <Example 13> Preparation of 9-(4-methoxyphenyl)[2]benzoxepino[3,4-f]-1,3-benzodioxol-11(6H)-one (HN2218)

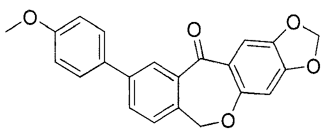

According to the preparation method of General Preparation Method 6, the compound **22** (30 mg, 0.09 mmol) prepared in step 5 of Example 9 and 4-methoxyphenylboronic acid(20.5 mg, 0.135 mmol) were allowed to react, and then the reaction mixture was purified by column chromatography using 3% ethyl acetate and 50% methylene chloride solvent in n-hexane to obtain 9-(4-methoxyphenyl)[2]benzoxepino[3,4-f]-1,3-benzodioxol-11(6H)-one (30.3 mg, 93%).
¹H NMR (500 MHz, CDCl₃) δ 8.12 (d, *J =* 2.0 Hz, 1H), 7.72 (dd, *J* = 7.8, 2.0 Hz, 1H), 7.69 (s, 1H), 7.60 - 7.54 (m, 2H), 7.38 (d, *J =* 7.8 Hz, 1H), 6.99 (d, *J =* 8.8 Hz, 2H), 6.51 (s, 1H), 6.02 (s, 2H), 5.18 (s, 2H), 3.86 (s, 3H)

### <Example 14> Preparation of 9-(2,4-dimethoxyphenyl)[2]benzoxepino[3,4-f]-1,3-benzodioxol-11(6H)-one (HN2219)

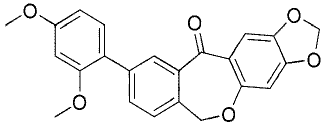

According to the preparation method of General Preparation Method 6, the compound **22** (35 mg, 0.105 mmol) prepared in step 5 of Example 9 and 2,4-dimethoxyphenylboronic acid(28.0 mg, 0.158 mmol) were allowed to react, and then the reaction mixture was purified by column chromatography using 5~33% ethyl acetate solvent in n-hexane to obtain 9-(2,4-dimethoxyphenyl)[2]benzoxepino[3,4-f]-1,3-benzodioxol-11(6H)-one.
¹H NMR (500 MHz, CDCl₃) δ 8.06 (d, *J* = 1.8 Hz, 1H), 7.69 (q, *J* = 1.9 Hz, 2H), 7.34 (d, *J* = 7.8 Hz, 1H), 7.28 (s, 1H), 6.59 - 6.55 (m, 2H), 6.50 (s, 1H), 6.01 (s, 2H), 5.17 (s, 2H), 3.84 (s, 3H), 3.80 (s, 3H)

### <Example 15> Preparation of 9-(3,4,5-trimethoxyphenyl)[2]benzoxepino[3,4-f]-1,3-benzodioxol-11(6H)-one (HN2220)

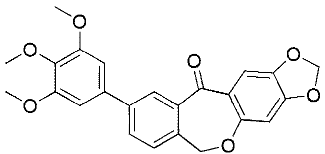

According to the preparation method of General Preparation Method 6, the compound 22 (30 mg, 0.09 mmol) prepared in step 5 of Example 9 and 3,4,5-trimethoxyphenylboronic acid(28.6 mg, 0.135 mmol) were allowed to react, and then the reaction mixture was purified by column chromatography using 12% ethyl acetate and 50% methylene chloride solvent in n-hexane to obtain 9-(3,4,5-trimethoxyphenyl)[2]benzoxepino[3,4-f]-1,3-benzodioxol-11(6H)-one(36.2 mg, 95%).
¹H NMR (500 MHz, CDCl₃) δ 8.12 (d, *J* = 1.9 Hz, 1H), 7.72 (dd, *J* = 7.7, 2.0 Hz, 1H), 7.69 (s, 1H), 7.40 (d, *J =* 7.8 Hz, 1H), 6.81 (s, 2H), 6.51 (s, 1H), 6.03 (s, 2H), 5.19 (s, 2H), 3.93 (s, 6H), 3.90 (s, 3H)

### <Example 16> Preparation of 8-(cyclohexylamino)-9-fluoro[2]benzoxepino[3,4-f]-1,3-benzodioxol-11(6H)-one (HN2221)

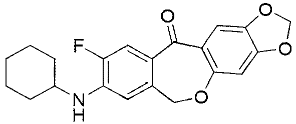

According to the preparation method of General Preparation Method 5, 8,9-difluoro[2]benzoxepino[3,4-f]-1,3-benzodioxol-11(6H)-one(20 mg, 0.068 mmol) of Example 3 and cyclohexylamine(0.04 mL, 0.340 mmol) were allowed to react, and then the reaction mixture was purified by column chromatography using 17% ethyl acetate solvent in n-hexane to obtain 8-(cyclohexylamino)-9-fluoro[2]benzoxepino[3,4-f]-1,3-benzodioxol-11(6H)-one(22.4 mg, 89%).
¹H NMR (500 MHz, DMSO-d₆) δ 7.56 (d, *J* = 13.3 Hz, 1H), 7.53 (s, 1H), 6.84 (d, *J* = 8.3 Hz, 1H), 6.69 (s, 1H), 6.12 (s, 2H), 5.15 (s, 2H), 1.94 (d, *J =* 11.3 Hz, 2H), 1.71 (dd, *J =* 55.1, 13.0 Hz, 4H), 1.41 - 1.29 (m, 4H)

### <Example 17> Preparation of 8-(4-benzylpiperidin-1-yl)-9-fluoro[2]benzoxepino[3,4-f]-1,3-benzodioxol-11(6H)-one (HN2222)

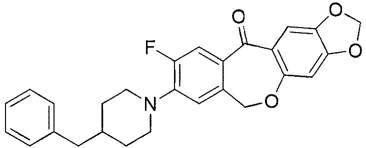

According to the preparation method of General Preparation Method 5, 8,9-difluoro[2]benzoxepino[3,4-f]-1,3-benzodioxol-11(6H)-one(20 mg, 0.068 mmol) of Example 3 and 4-benzylpiperidine(0.06 mL, 0.340 mmol) were allowed to react, and then the reaction mixture was purified by column chromatography using 17% ethyl acetate solvent in n-hexane to obtain 8-(4-benzylpiperidin-1-yl)-9-fluoro[2]benzoxepino[3,4-f]-1,3-benzodioxol-11(6H)-one(13.3 mg, 44%).
¹H NMR (500 MHz, DMSO-d₆) δ 7.56 (d, *J =* 14.5 Hz, 1H), 7.51 (s, 1H), 7.34 - 7.28 (m, 2H), 7.22 (d, *J* = 7.5 Hz, 3H), 7.13 (d, *J* = 8.6 Hz, 1H), 6.71 (s, 1H), 6.13 (s, 2H), 5.19 (s, 2H), 3.62 (d, *J* = 12.4 Hz, 2H), 2.80 (t, *J =* 11.4 Hz, 2H), 2.58 (d, *J* = 7.0 Hz, 2H), 1.77 - 1.72 (m, 1H), 1.69 (d, *J* = 13.6 Hz, 2H), 1.36 (dd, *J* = 20.9, 12.0 Hz, 3H)

### <Example 18> Preparation of 8-((4-methoxyphenethyl)amino)-9-fluoro[2]benzoxepino[3,4-f]-1,3-benzodioxol-11(6H)-one (HN2223)

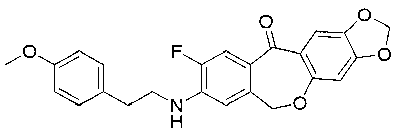

According to the preparation method of General Preparation Method 5, 8,9-difluoro[2]benzoxepino[3,4-f]-1,3-benzodioxol-11(6H)-one(20 mg, 0.068 mmol) of Example 3 and 1-(4-methoxyphenyl)ethylamine (0.05 mL, 0.340 mmol) were allowed to react, and then the reaction mixture was purified by column chromatography using 25% ethyl acetate solvent in n-hexane to obtain 8-((4-methoxyphenethyl)amino)-9-fluoro[2]benzoxepino[3,4-f]-1,3-benzodioxol-11(6H)-one(22.9 mg, 80%).
¹H NMR (500 MHz, DMSO-d₆) δ 7.56 (d, *J* = 13.2 Hz, 1H), 7.53 (s, 1H), 7.22 (d, *J* = 8.5 Hz, 2H), 6.88 (d, *J =* 8.6 Hz, 2H), 6.83 (d, *J =* 8.4 Hz, 1H), 6.69 (s, 1H), 6.12 (s, 2H), 5.15 (s, 2H), 3.73 (s, 3H), 3.43 - 3.38 (m, 2H), 2.87 - 2.82 (m, 2H)

### <Example 19> Preparation of 8-((2-(thiophen-2-yl)ethyl)amino)-9-fluoro[2]benzoxepino[3,4-f]-1,3-benzodioxol-11(6H)-one (HN2224)

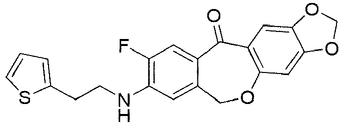

According to the preparation method of General Preparation Method 5, 8,9-difluoro[2]benzoxepino[3,4-f]-1,3-benzodioxol-11(6H)-one(20 mg, 0.068 mmol) of Example 3 and 2-thiopheneethylamine(0.04 mL, 0.340 mmol) were allowed to react, and then the reaction mixture was purified by column chromatography using 25% ethyl acetate solvent in n-hexane to obtain 8-((2-(thiophen-2-yl)ethyl)amino)-9-fluoro[2]benzoxepino[3,4-f]-1,3-benzodioxol-11(6H)-one(17.2 mg, 64%).
¹H NMR (500 MHz, DMSO-d₆) δ 7.56 (d, *J =* 13.1 Hz, 1H), 7.52 (s, 1H), 7.38 - 7.32 (m, 1H), 6.98 (d, *J* = 3.7 Hz, 2H), 6.84 (d, *J* = 8.4 Hz, 1H), 6.72 - 6.70 (m, 1H), 6.69 (s, 1H), 6.12 (s, 2H), 5.14 (s, 2H), 3.49 (dd, *J* = 13.2, 7.1 Hz, 2H), 3.14 (t, *J* = 7.2 Hz, 2H)

### <Example 20> Preparation of 7,8-difluoro[2]benzoxepino[3,4-f]-1,3-benzodioxol-11(6H)-one (HN2225)

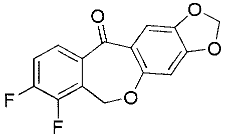

### Step 1: Preparation of methyl 3,4-difluoro-2-methylbenzoate

Methyl 3,4-difluoro-2-methylbenzoate (937.3 mg, 86%) was prepared in the same manner as in step 1 of Example 3, except that in step 1 of Example 3, 3,4-difluoro-2-methylbenzoic acid (1.0 g, 5.811 mmol) was used instead of 4,5-difluoro-2-methylbenzoic acid.

### Step 2: Preparation of methyl 2-bromomethyl-3,4-difluorobenzoate

Methyl 2-bromomethyl-3,4-difluorobenzoate(1.28 g, 97%) was prepared in the same manner as in step 2 of Example 3, except that in step 2 of Example 3, methyl 3,4-difluoro-2-methylbenzoate(930 mg, 4.995 mmol) prepared in step 1 was used instead of methyl 4,5-difluoro-2-methylbenzoate.

### Step 3: Preparation of methyl 2-[(1,3-benzodioxol-5-yloxy)methyl]-3,4-difluorobenzoate

Methyl 2-[(1,3-benzodioxol-5-yloxy)methyl]-3,4-difluorobenzoate(1.35 g, 87%) was prepared in the same manner as in step 3 of Example 3, except that in step 3 of Example 3, methyl 2-bromomethyl-3,4-difluorobenzoate(1.28 g, 4.844 mmol) prepared in step 2 was used instead of methyl 2-bromomethyl-4,5-difluorobenzoate.

### Step 4: Preparation of 2-[(1,3-benzodioxol-5-yloxy)methyl]-3,4-difluorobenzoic acid

According to the preparation method of step 2 of General Preparation Method 2, methyl 2-[(1,3-benzodioxol-5-yloxy)methyl]-3,4-difluorobenzoate(1.344 g, 4.171 mmol) prepared in step 3 was reacted to obtain 2-[(1,3-benzodioxol-5-yloxy)methyl]-3,4-difluorobenzoic acid(1.25 g, 97%).

### Step 5: Preparation of 7,8-difluoro[2]benzoxepino[3,4-f]-1,3-benzodioxol-11(6H)-one

7,8-Difluoro[2]benzoxepino[3,4-f]-1,3-benzodioxol-11(6H)-one(1.07 g, 91%) was prepared in the same manner as in step 5 of Example 3, except that in step 5 of Example 3, 2-[(1,3-benzodioxol-5-yloxy)methyl]-3,4-difluorobenzoic acid(1.245 g, 4.039 mmol) prepared in step 4 was used instead of 2-[(1,3-benzodioxol-5-yloxy)methyl]-4,5-difluorobenzoic acid.
¹H NMR (500 MHz, CDCl₃) δ 7.75 (ddd, *J* = 8.7, 4.8, 1.7 Hz, 1H), 7.64 (s, 1H), 7.27 -7.22 (m, 1H), 6.52 (s, 1H), 6.03 (s, 2H), 5.29 (d, *J=* 0.7 Hz, 2H)

### <Example 21> Preparation of 8-(dimethylamino)-7-fluoro[2]benzoxepino[3,4-f]-1,3-benzodioxol-11(6H)-one (HN2226)

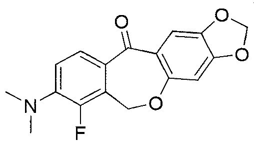

8-(Dimethylamino)-7-fluoro[2]benzoxepino[3,4-f]-1,3-benzodioxol-11(6H)-one(20 g, 92%) was prepared in the same manner as in Example 4, except that in Example 4, 7,8-difluoro[2]benzoxepino[3,4-f]-1,3-benzodioxol-11(6H)-one(20 mg, 0.069 mmol) of Example 20 was used instead of 8,9-difluoro[2]benzoxepino[3,4-f]-1,3-benzodioxol-11(6H)-one.
¹H NMR (500 MHz, DMSO-d₆) δ 7.63 (d, *J* = 8.8 Hz, 1H), 7.49 (s, 1H), 6.97 (t, *J* = 9.0 Hz, 1H), 6.69 (s, 1H), 6.11 (s, 2H), 5.27 (s, 2H), 2.96 (d, *J =* 1.5 Hz, 6H)

### <Example 22> Preparation of 8-(morpholino)-7-fluoro[2]benzoxepino[3,4-f]-1,3-benzodioxol-11(6H)-one (HN2227)

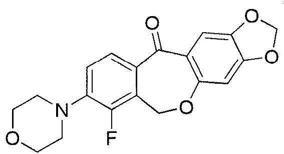

8-(Morpholino)-7-fluoro[2]benzoxepino[3,4-f]-1,3-benzodioxol-11(6H)-one(32.5 mg, 88%) was prepared in the same manner as in Example 5, except that in Example 5, 7,8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11(6H)-one.
¹H NMR (500 MHz, DMSO-d₆) δ 7.65 (d, *J =* 8.4 Hz, 1H), 7.48 (s, 1H), 7.12 (t, *J =* 8.6 Hz, 1H), 6.70 (s, 1H), 6.11 (s, 2H), 5.29 (s, 2H), 3.75 (t, *J =* 4.8 Hz, 4H), 3.16 (t, *J* = 4.8 Hz, 4H)

### <Example 23> Preparation of 8-(piperidinyl)-7-fluoro[2]benzoxepino[3,4-f]-1,3-benzodioxol-11(6H)-one (HN2228)

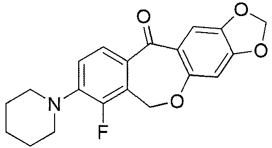

8-(Piperidinyl)-7-fluoro[2]benzoxepino[3,4-f]-1,3-benzodioxol-11(6H)-one(32.1 mg, 87%) was prepared in the same manner as in Example 6, except that in Example 6, 7,8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11(6H)-one.
¹H NMR (500 MHz, DMSO-d₆) δ 7.63 (d, *J =* 8.4 Hz, 1H), 7.48 (s, 1H), 7.10 (t, *J =* 8.8 Hz, 1H), 6.70(s, 1H), 6.11 (s, 2H), 5.28 (s, 2H), 3.14 (t, *J =* 5.3 Hz, 4H), 1.65 - 1.64(m, 4H), 1.58 - 1.57(m, 2H)

### <Example 24> Preparation of 8-(phenylpiperazine-1-yl)-7-fluoro[2]benzoxepino[3,4-f]-1,3-benzodioxol-11(6H)-one (HN2229)

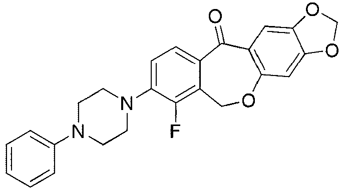

8-(Phenylpiperazine-1-yl)-7-fluoro[2]benzoxepino[3,4-f]-1,3-benzodioxol-11(6H)-one(37.5 mg, 83%) was prepared in the same manner as in Example 7, except that in Example 7, 7,8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11(6H)-one.
¹H NMR (500 MHz, DMSO-d₆) δ 7.67 (d, *J =* 8.8 Hz, 1H), 7.49 (s, 1H), 7.25 (dd, *J =* 7.3, 8.8 Hz, 2H), 7.18 (t, *J =* 8.8 Hz, 1H), 6.99 (d, *J =* 8.0 Hz, 2H), 6.81 (t, *J =* 7.3 Hz, 1H), 6.71 (s, 1H), 6.12 (s, 2H), 5.31 (s, 2H), 3.34 - 3.30 (m, 4H)

### <Example 25> Preparation of 8-(benzylamino)-7-fluoro[2]benzoxepino[3,4-f]-1,3-benzodioxol-11(6H)-one (HN2230)

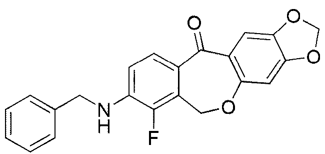

8-(Benzylamino)-7-fluoro[2]benzoxepino[3,4-f]-1,3-benzodioxol-11(6H)-one(30.2 mg, 77%) was prepared in the same manner as in Example 8, except that in Example 8, 7,8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11(6H)-one.
¹H NMR (500 MHz, DMSO-d₆) δ 7.54 (d, *J =* 8.4 Hz, 1H), 7.48 (s, 1H), 7.35 - 7.29 (m, 4H), 7.22 (d, *J =* 6.9 Hz, 2H), 6.66 (t, *J =* 8.8 Hz, 2H), 6.09 (s, 2H), 5.26 (s, 2H), 4.45 (d, *J =* 6.3Hz, 2H)

### <Example 26> Preparation of 8-(cyclohexylamino)-7-fluoro[2]benzoxepino[3,4-f]-1,3-benzodioxol-11(6H)-one (HN2231)

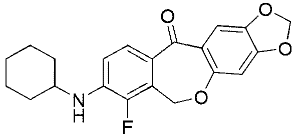

8-(Cyclohexylamino)-7-fluoro[2]benzoxepino[3,4-f]-1,3-benzodioxol-11(6H)-one(29.2 mg, 76%) was prepared in the same manner as in Example 16, except that in Example 16, 8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11(6H)-one.
¹H NMR (500 MHz, DMSO-d₆) δ 7.64 (d, *J =* 8.8 Hz, 1H), 7.50 (s, 1H), 6.83 (t, *J =* 8.8 Hz, 1H), 6.68 (s, 1H), 6.10 (s, 2H), 5.24 (s, 2H), 1.90 (d, *J =* 11.1 Hz, 2H), 1.72 (d, *J =* 12.6 Hz, 2H), 1.64 - 1.57 (m, 2H), 1.36 - 1.27 (m, 4H)

### <Example 27> Preparation of 8-(4-benzylpiperidin-1-yl)-7-fluoro[2]benzoxepino[3,4-f]-1,3-benzodioxol-11(6H)-one (HN2232)

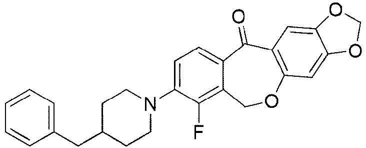

8-(4-Benzylpiperidin-1-yl)-7-fluoro[2]benzoxepino[3,4-f]-1,3-benzodioxol-11(6H)-one(39.1 mg, 84.9%) was prepared in the same manner as in Example 17, except that in Example 17, 7,8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11(6H)-one.
¹H NMR (500 MHz, DMSO-d₆) δ 7.61 (d, *J =* 8.8 Hz, 1H), 7.48 (s, 1H), 7.28 (t, *J =* 7.6 Hz, 2H), 7.18 (t, *J =* 7.6 Hz, 3H), 7.08 (t, *J =* 8.6 Hz, 1H), 6.69(s, 1H), 6.11 (s, 2H), 5.27 (s, 2H), 3.53 (d, *J =* 12.2 Hz, 2H), 2.75 (t, *J =* 11.3 Hz, 2H), 2.56 (d, *J =* 6.9 Hz, 2H), 1.75 - 1.71 (m, 1H), 1.67 (d, *J =* 13.4 Hz, 2H), 1.34 (ddd, J = 23.8, 11.7, 3.2 Hz, 2H)

### <Example 28> Preparation of 8-((4-methoxyphenethyl)amino)-7-fluoro[2]benzoxepino[3,4-f]-1,3-benzodioxol-11(6H)-one (HN2233)

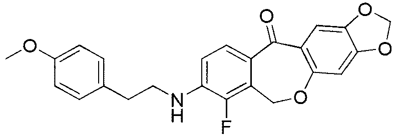

8-((4-Methoxyphenethyl)amino)-7-fluoro[2]benzoxepino[3,4-f]-1,3-benzodioxol-11(6H)-one(36.1 mg, 82.8%) was prepared in the same manner as in Example 18, except that in Example 18, 7,8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11(6H)-one.
¹H NMR (500 MHz, DMSO-d₆) δ 7.66 (d, *J =* 8.8 Hz, 1H), 7.51 (s, 1H), 7.18 (d, *J=* 8.4 Hz, 2H), 6.86 - 6.83 (m, 3H), 6.68 (s, 1H), 6.10 (s, 2H), 5.25 (s, 2H), 3.71 (s, 3H), 3.38 (dd, *J =* 14.4, 6.3 Hz, 2H), 2.80 (t, *J =* 7.6 Hz, 2H)

### <Example 29> Preparation of 8-((2-(thiophen-2-yl)ethyl)amino)-7-fluoro[2]benzoxepino[3,4-f]-1,3-benzodioxol-11(6H)-one (HN2234)

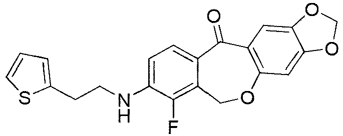

8-((2-(Thiophen-2-yl)ethyl)amino)-7-fluoro[2]benzoxepino[3,4-f]-1,3-benzodioxol-11(6H)-one(34.6 mg, 84%) was prepared in the same manner as in Example 19, except that in Example 19, 7,8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11(6H)-one.
¹H NMR (500 MHz, DMSO-d₆) δ 7.65 (d, *J =* 8.4 Hz, 1H), 7.51 (s, 1H), 7.32 (dd, *J =* 4.3, 1.8 Hz, 1H), 6.95 (t, *J =* 4.0 Hz, 2H), 6.84 (t, *J =* 8.6 Hz, 1H), 6.68(s, 1H), 6.61 (s, 1H), 6.10 (s, 2H), 5.25 (s, 2H), 3.47 (dd, *J =* 7,1, 13.2 Hz, 2H), 3.09 (t, *J =* 7.1 Hz, 2H)

### <Example 30> Preparation of 9-bromo-2,3-dimethoxydibenz[b,e]oxepin-11(6H)-one (HN2235)

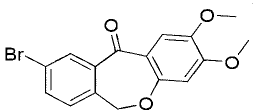

### Step 1: Preparation of methyl 2-[(3,4-dimethoxyphenoxy)methyl]-5-bromobenzoate

Methyl 5-bromo-2-(bromomethyl)benzoate (95.8 mg, 0.311 mmol) prepared in step 2 of Example 9 and 3,4-dimethoxyphenol(57.5 mg, 0.373 mmol) were allowed to react, and then the reaction mixture was purified by column chromatography using 25% ethyl acetate solvent in n-hexane to obtain methyl 2-[(3,4-dimethoxyphenoxy)methyl]-5-bromobenzoate(86.5 mg, 73%).

### Step 2: Preparation of 2-[(3,4-dimethoxyphenoxy)methyl]-5-bromobenzoic acid

According to the preparation method of step 2 of General Preparation Method 2, methyl 2-[(3,4-dimethoxyphenoxy)methyl]-5-bromobenzoate(40 mg, 0.105 mmol) prepared in step 1 was reacted to obtain 2-[(3,4-dimethoxyphenoxy)methyl]-5-bromobenzoic acid(36.5 mg, 95%).

### Step 3: Preparation of 9-bromo-2,3-dimethoxydibenz[b-e]oxepin-11(6H)-one

According to the preparation method of step 3 of General Preparation Method 2, 2-[(3,4-dimethoxyphenoxy)methyl]-5-bromobenzoic acid(37.7 mg, 0.103 mmol) prepared in step 2 was reacted, and then the reaction mixture was purified by column chromatography using 9% ethyl acetate and 50% methylene chloride solvent in n-hexane to obtain 9-bromo-2,3-dimethoxydibenz[b,e]oxepin-11(6H)-one(31.7 mg, 88%).
¹H NMR (500 MHz, CDCl₃) δ 8.07 (d, *J =* 2.1 Hz, 1H), 7.66 (dd, *J =* 7.3, 2.8 Hz, 2H), 7.24 (d, *J =* 8.0 Hz, 1H), 6.50 (s, 1H), 5.12 (s, 2H), 3.94 (s, 3H), 3.92 (s, 3H)

### <Example 31> Preparation of 9-(2,4-dimethoxyphenyl)-2,3-dimethoxydibenzo[b,e]oxepin-11(6H)-one (HN2236)

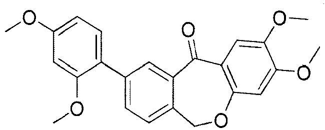

According to the preparation method of General Preparation Method 6, 9-bromo-2,3-dimethoxydibenz[b,e]oxepin-11(6H)-one(10 mg, 0.028 mmol) of Example 30 and 2,4-dimethoxyphenylboronic acid(7.8 mg, 0.043 mmol) were allowed to react, and then the reaction mixture was purified by column chromatography using 33% ethyl acetate solvent in n-hexane to obtain 9-(2,4-dimethoxyphenyl)-2,3-dimethoxydibenzo[b,e]oxepin-11(6H)-one(11.5 mg, 90%).
¹H NMR (500 MHz, CDCl₃) δ 8.06 (d, *J =* 1.6 Hz, 1H), 7.71 (s, 1H), 7.68 (dd, *J* = 7.7, 1.9 Hz, 1H), 7.35 (d, *J =* 7.6 Hz, 1H), 7.25 (d, *J =* 8.4 Hz, 1H), 6.57 - 6.54 (m, 2H), 6.50 (s, 1H), 5.17 (s, 2H), 3.92, (s, 3H), 3.90 (s, 3H), 3.84 (s, 3H), 3.78 (s, 3H)

### <Example 32> Preparation of 9-(3,4-dimethoxyphenyl)-2,3-dimethoxydibenzo[b,e]oxepin-11(6H)-one (HN2237)

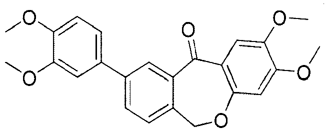

According to the preparation method of General Preparation Method 6, 9-bromo-2,3-dimethoxydibenz[b,e]oxepin-11(6H)-one(25 mg, 0.072 mmol) of Example 30 and 3,4-dimethoxyphenylboronic acid(19.5 mg, 0.107 mmol) were allowed to react, and then the reaction mixture was purified by column chromatography using 20~33% ethyl acetate solvent in n-hexane to obtain 9-(3,4-dimethoxyphenyl)-2,3-dimethoxydibenzo[b,e]oxepin-11(6H)-one(19.7 mg, 67%).
¹H NMR (500 MHz, CDCl₃) δ 8.12 (d, *J* = 1.9 Hz, 1H), 7.73 (d, *J* = 2.0 Hz, 1H), 7.71 (s, 1H), 7.39 (d, *J* = 8.1 Hz, 1H), 7.18 (dd, *J* = 8.4, 1.9 Hz, 1H), 7.12 (d, *J* = 2.3 Hz, 1H), 6.94 (d, *J* = 8.4 Hz, 1H), 6.51 (s, 1H), 5.18 (s, 2H), 3.93 (d, *J* = 3.1 Hz, 6H), 3.91 (d, *J* = 3.1 Hz, 6H)

### <Example 33> Preparation of 2,3-dimethoxy-9-(3-methoxyphenyl)dibenzo[b,e]oxepin-11(6H)-one (HN2238)

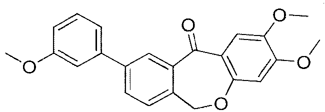

According to the preparation method of General Preparation Method 6, 9-bromo-2,3-dimethoxydibenz[b,e]oxepin-11(6H)-one(25 mg, 0.072 mmol) of Example 30 and 3-methoxyphenylboronic acid(16.3 mg, 0.107 mmol) were allowed to react, and then the reaction mixture was purified by column chromatography using 33% ethyl acetate solvent in n-hexane to obtain 2,3-dimethoxy-9-(3-methoxyphenyl)dibenzo[b,e]oxepin-11(6H)-one(24.5 mg, 91%).
¹H NMR (500 MHz, CDCl₃) δ 8.16 (d, *J* = 1.9 Hz, 1H), 7.74 (dd, *J* = 7.8 , 2.1 Hz, 1H), 7.71 (s, 1H), 7.41 (d, *J =* 7.6 Hz, 1H), 7.35 (t, *J =* 8.0 Hz, 1H), 7.20 (d, *J =* 8.0 Hz, 1H), 7.14 (t, *J =* 2.1 Hz, 1H), 6.91 (dd, *J =* 8.2, 2.5 Hz, 1H), 6.51 (s, 1H), 5.19 (s, 2H), 3.93 (s, 3H), 3.91 (s, 3H), 3.85 (s, 3H)

### <Example 34> Preparation of 9-(4-fluorophenyl)-2,3-dimethoxydibenzo[b,e]oxepin-11(6H)-one (HN2239)

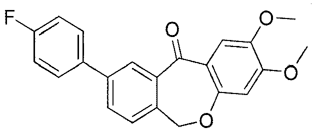

According to the preparation method of General Preparation Method 6, 9-bromo-2,3-dimethoxydibenz[b,e]oxepin-11(6H)-one(25 mg, 0.072 mmol) of Example 30 and 4-fluorophenylboronic acid(15.0 mg, 0.107 mmol) were allowed to react, and then the reaction mixture was purified by column chromatography using 6% ethyl acetate and 90% methylene chloride solvent in n-hexane to obtain 9-(4-fluorophenyl)-2,3-dimethoxydibenzo[b,e]oxepin-11(6H)-one(24 mg, 92%).
¹H NMR (500 MHz, CDCl₃) δ 8.11 (d, *J =* 1.9 Hz, 1H), 7.71 - 7.69 (m, 2H), 7.59 - 7.56 (m, 2H), 7.41 (d, *J =* 8.0 Hz, 1H), 7.13 (t, *J =* 8.8 Hz, 2H), 6.51 (s, 1H), 5.19 (s, 2H), 3.93 (s, 3H), 3.91 (s, 3H)

### <Example 35> Preparation of 8-(4-chlorophenyl)[2]benzoxepino[3,4-f]-1,3-benzodioxol-11(6H)-one (HN2240)

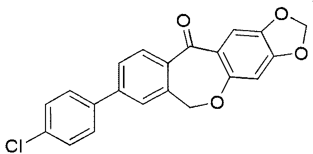

8-(4-Chlorophenyl)[2]benzoxepino[3,4-f]-1,3-benzodioxol-11(6H)-one was prepared in the same manner as in step 6 of Example 9, except that in step 6 of Example 9, 8-bromo[2]benzoxepino[3,4-f]-1,3-benzodioxol-11(6H)-one(35 mg, 0.105 mmol) of Example 76 was used instead of 9-bromo[2]benzoxepino[3,4-f]-1,3-benzodioxol-11(6H)-one.
¹H NMR (500 MHz, CDCl₃) δ 8.03 (d, *J =* 8.1 Hz, 1H), 7.70 (s, 1H), 7.64 (dd, *J* = 8.1, 1.9 Hz, 1H), 7.58 - 7.54 (m, 2H), 7.50 (d, *J =* 1.8 Hz, 1H), 7.47 - 7.41 (m, 2H), 6.51 (s, 1H), 6.02 (s, 2H), 5.20 (s, 2H)

### <Example 36> Preparation of 8-(p-tolyl)[2]benzoxepino[3,4-f]-1,3-benzodioxol-11(6H)-one (HN2241)

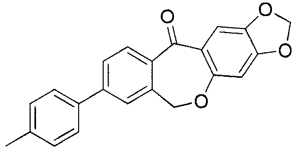

8-(p-Tolyl)[2]benzoxepino[3,4-f]-1,3-benzodioxol-11(6H)-one(26.0 mg, 72%) was prepared in the same manner as in Example 12, except that in Example 12, 8-bromo[2]benzoxepino[3,4-f]-1,3-benzodioxol-11(6H)-one(35 mg, 0.105 mmol) of Example 76 was used instead of 9-bromo[2]benzoxepino[3,4-f]-1,3-benzodioxol-11(6H)-one.
¹H NMR (500 MHz, CDCl₃) δ 8.03 (d, *J =* 8.1 Hz, 1H), 7.71 (s, 1H), 7.68 (dd, *J =* 8.1, 1.8 Hz, 1H), 7.56 - 7.53 (m, 2H), 7.52 (d, *J =* 1.8 Hz, 1H), 7.28 (d, *J =* 7.9 Hz, 2H), 6.52 (s, 1H), 6.02 (s, 2H), 5.21 (s, 2H), 2.41 (s, 3H)

### <Example 37> Preparation of 8-(4-methoxyphenyl)[2]benzoxepino[3,4-f]-1,3-benzodioxol-11(6H)-one (HN2242)

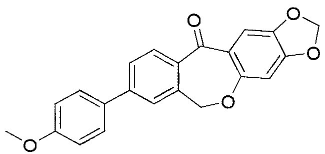

8-(4-Methoxyphenyl)[2]benzoxepino[3,4-f]-1,3-benzodioxol-11(6H)-one(29.5 mg, 78%) was prepared in the same manner as in Example 13, except that in Example 13, 8-bromo[2]benzoxepino[3,4-f]-1,3-benzodioxol-11(6H)-one(35 mg, 0.105 mmol) of Example 76 was used instead of 9-bromo[2]benzoxepino[3,4-f]-1,3-benzodioxol-11(6H)-one.
¹H NMR (500 MHz, CDCl₃) δ 8.02 (d, *J =* 8.1 Hz, 1H), 7.71 (s, 1H), 7.65 (dd, *J =* 8.1, 1.9 Hz, 1H), 7.61 - 7.56 (m, 2H), 7.50 (d, *J =* 1.7 Hz, 1H), 7.03 - 6.98 (m, 2H), 6.52 (s, 1H), 6.03 (s, 2H), 5.21 (s, 2H), 3.87 (s, 3H)

### <Example 38> Preparation of 8-(2,4-dimethoxyphenyl)[2]benzoxepino[3,4-f]-1,3-benzodioxol-11(6H)-one (HN2243)

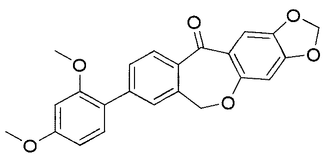

8-(2,4-Dimethoxyphenyl)[2]benzoxepino[3,4-f]-1,3-benzodioxol-11(6H)-one(25.6 mg, 63%) was prepared in the same manner as in Example 14, except that in Example 14, 8-bromo[2]benzoxepino[3,4-f]-1,3-benzodioxol-11(6H)-one(35 mg, 0.105 mmol) of Example 76 was used instead of 9-bromo[2]benzoxepino[3,4-f]-1,3-benzodioxol-11(6H)-one.
¹H NMR (400 MHz, CDCl₃) δ 7.98 (d, *J =* 8.3 Hz, 1H), 7.71 (s, 1H), 7.61 (d, *J =* 7.8 Hz, 1H), 7.47 (s, 1H), 7.29 (s, 1H), 6.59 (d, *J =* 9.7 Hz, 2H), 6.51 (s, 1H), 6.02 (s, 2H), 5.19 (s, 2H), 3.87 (s, 3H), 3.82 (s, 3H)

### <Example 39> Preparation of 8-(3,4,5-trimethoxyphenyl)[2]benzoxepino[3,4-f]-1,3-benzodioxol-11(6H)-one (HN2244)

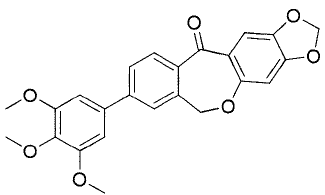

8-(3,4,5-Trimethoxyphenyl)[2]benzoxepino[3,4-f]-1,3-benzodioxol-11(6H)-one(22.5 mg, 51%) was prepared in the same manner as in Example 15, except that in Example 15, 8-bromo[2]benzoxepino[3,4-f]-1,3-benzodioxol-11(6H)-one(35 mg, 0.105 mmol) of Example 76 was used instead of 9-bromo[2]benzoxepino[3,4-f]-1,3-benzodioxol-11(6H)-one.
¹H NMR (400 MHz, CDCl₃) δ 8.03 (d, *J =* 8.1 Hz, 1H), 7.71 (s, 1H), 7.65 (d, *J =* 8.0 Hz, 1H), 7.50 (s, 1H), 6.81 (s, 2H), 6.53 (s, 1H), 6.04 (s, 2H), 5.23 (s, 2H), 3.95 (s, 6H), 3.91 (s, 3H)

### <Example 40> Preparation of 2,3-dimethoxy-9-(4-(trifluoromethyl)phenyl)dibenzo[b,e]oxepin-11(6H)-one (HN2245)

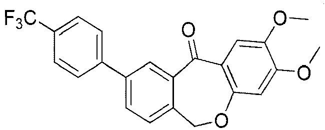

According to the preparation method of General Preparation Method 6, 9-bromo-2,3-dimethoxydibenz[b,e]oxepin-11(6H)-one(25 mg, 0.072 mmol) of Example 30 and 4-(trifluoromethyl)phenylboronic acid(20.4 mg, 0.107 mmol) were allowed to react, and then the reaction mixture was purified by column chromatography using 12% ethyl acetate and 30% methylene chloride solvent in n-hexane to obtain 2,3-dimethoxy-9-(4-(trifluoromethyl)phenyl)dibenzo[b,e]oxepin-11(6H)-one(26 mg, 88%).
¹H NMR (500 MHz, CDCl₃) δ 8.19 (d, *J =* 1.7 Hz, 1H), 7.78 (dd, *J =* 7.7, 1.8 Hz, 1H), 7.76 - 7.69 (m, 5H), 7.48 (d, *J =* 7.8 Hz, 1H), 6.54 (s, 1H), 5.22 (s, 2H), 3.96 (s, 3H), 3.93 (s, 3H)

### <Example 41> Preparation of N-(9-fluoro-11-oxo-6,11-dihydro-[1,3]dioxolo[4',5':4,5]benzo[1,2-b]benzo[e]oxepin-8-yl)methanesulfonamide (HN2301)

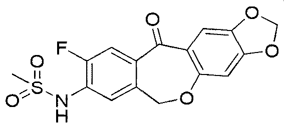

According to the preparation method of General Preparation Method 5, 8,9-difluoro[2]benzoxepino[3,4-f]-1,3-benzodioxol-11(6H)-one(30 mg, 0.103 mmol) of Example 3 and methanesulfonamide(19.6 mg, 0.206 mmol) were allowed to react, and then the reaction mixture was purified by column chromatography using 33% ethyl acetate solvent in n-hexane to obtain N-(9-fluoro-11-oxo-6,11-dihydro-[1,3]dioxolo[4',5':4,5]benzo[1,2-b]benzo[e]oxepin-8-yl)methanesulfonamide(12 mg, 31%).
¹H NMR (500 MHz, DMSO-d₆) δ 10.18 (s, 1H), 7.63 (d, *J =* 11.2 Hz, 1H), 7.56 (d, *J* = 7.4 Hz, 1H), 7.46 (s, 1H), 6.70 (s, 1H), 6.11 (s, 2H), 5.21 (s, 2H), 3.15 (s, 3H)

### <Example 42> Preparation of N-(7-fluoro-11-oxo-6,11-dihydro-[1,3]dioxolo[4',5':4,5]benzo[1,2-b]benzo[e]oxepin-8-yl)methanesulfonamide (HN2302)

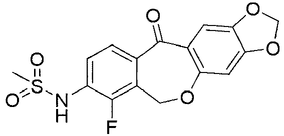

N-(7-fluoro-11-oxo-6,11-dihydro-[1,3]dioxolo[4',5':4,5]benzo[1,2-b]benzo[e]oxepin-8-yl)methanesulfonamide(12 mg, 31%) was prepared in the same manner as in Example 41, except that in Example 41, 7,8-difluoro[2]benzoxepino[3,4-f]-1,3-benzodioxol-11(6H)-one(30 mg, 0.103 mmol) of Example 20 was used instead of 8,9-difluoro[2]benzoxepino[3,4-f]-1,3-benzodioxol-11 (6H)-one.
¹H NMR (500 MHz, DMSO-d₆) δ 10.18 (s, 1H), 7.65 (d, *J =* 8.8 Hz, 1H), 7.54 (t, *J =* 8.1 Hz, 1H), 7.48 (s, 1H), 6.73 (s, 1H), 6.13 (s, 2H), 5.31 (s, 2H), 3.11 (s, 3H)

### <Example 43> Preparation of 2,3-dimethoxy-10,11-dihydro-5H-dibenzo[a,d][7]annulen-5-one (HN2303)

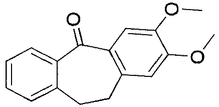

### Step 1: Preparation of methyl 2-(3,4-dimethoxyphenethyl)benzoate

According to the preparation method of step 1 of General Preparation Method 4, methyl 2-(bromomethyl)benzoate(200 mg, 0.873 mmol) and triphenylphosphine(251.9 mg, 0.959 mmol) were reacted, the resultant (2-(methoxycarbonyl)benzyl)triphenylphosphonium bromide (115.3 mg, 0.234 mmol) was reacted with 3,4-dimethoxybenzaldehyde (30 mg, 0.180 mmol), and then the next reaction was carried out without purification. The concentrated mixture was dissolved in 1.8 mL of MeOH, and a catalytic amount of Pd/C was added. The mixture was reacted by purging H₂ gas, and then purified by column chromatography using 16~20% ethyl acetate in n-hexane to obtain methyl 2-(3,4-dimethoxyphenethyl)benzoate(44.8 mg, 82%).

### Step 2: Preparation of 2-(3,4-dimethoxyphenethyl)benzoic acid

According to the preparation method of step 2 of General Preparation Method 4, 2-(3,4-dimethoxyphenethyl)benzoate (42.3 mg, 0.141 mmol) prepared in step 1 was reacted to obtain 2-(3,4-dimethoxyphenethyl)benzoic acid (39.6 mg, 98%).

### Step 3: Preparation of 2,3-dimethoxy-10,11-dihydro-5H-dibenzo[a,d][7]annulen-5-one

According to the preparation method of step 3 of General Preparation Method 4, 2-(3,4-dimethoxyphenethyl)benzoic acid(20 mg, 0.069 mmol) prepared in step 2 was reacted, and then the reaction mixture was purified by column chromatography using 9% ethyl acetate and 30% methylene chloride solvent in n-hexane to obtain 2,3-dimethoxy-10,11-dihydro-5H-dibenzo[a,d][7]annulen-5-one(16.2 mg, 86%).
¹H NMR (500 MHz, CDCl₃) δ 7.96 (dd, *J* = 7.8, 1.2 Hz, 1H), 7.76 (s, 1H), 7.42 (td, *J* = 7.4, 1.4 Hz, 1H), 7.33 (td, *J =* 7.7, 1.1 Hz, 1H), 7.22 (d, *J =* 7.3 Hz, 1H), 6.66 (s, 1H), 3.96 (s, 3H), 3.94 (s, 3H), 3.21 - 3.12 (m, 4H)

### <Example 44> Preparation of 9-bromo-2,3-dimethoxydibenzo[b,e]thiepin-11(6H)-one (HN2304)

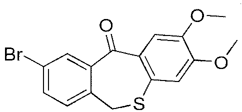

### Step 1: Preparation of methyl 5-bromo-2-(((3,4-dimethoxyphenyl)thio)methyl)benzoate

According to the preparation method of step 1 of General Preparation Method 2, methyl 5-bromo-2-(bromomethyl)benzoate (312 mg, 1.01 mmol) prepared in step 2 of Example 9 and 3,4-dimethoxythiophenol(161.2 mg, 0.947 mmol) were allowed to react, and then the reaction mixture was purified by column chromatography using 16~20% ethyl acetate in n-hexane to obtain methyl 5-bromo-2-(((3,4-dimethoxyphenyl)thio)methyl)benzoate(236.6 mg, 62%).

### Step 2: Preparation of 5-bromo-2-(((3,4-dimethoxyphenyl)thio)methyl)benzoic acid

According to the preparation method of step 2 of General Preparation Method 2, methyl 5-bromo-2-(((3,4-dimethoxyphenyl)thio)methyl)benzoate(236 mg, 0.594 mmol) prepared in step 1 was reacted to obtain 5-bromo-2-(((3,4-dimethoxyphenyl)thio)methyl)benzoic acid(223.2 mg, 98%).

### Step 5: Preparation of 9-bromo-2,3-dimethoxydibenzo[b-e]thiepin-11(6H)-one

According to the preparation method of step 3 of General Preparation Method 2, 5-bromo-2-(((3,4-dimethoxyphenyl)thio)methyl)benzoic acid(20 mg, 0.052 mmol) prepared in step 2 was reacted, and then the reaction mixture was purified by column chromatography using 5% ethyl acetate and 30% methylene chloride solvent in n-hexane to obtain 9-bromo-2,3-dimethoxydibenzo[b,e]thiepin-11(6H)-one(17.8 mg, 93%).
¹H NMR (500 MHz, CDCl₃) δ 7.82 (s, 1H), 7.78 (d, *J =* 2.1 Hz, 1H), 7.56 (dd, *J =* 8.1, 2.1 Hz, 1H), 7.09 (d, *J* = 8.1 Hz, 1H), 6.75 (s, 1H), 3.98 (s, 2H), 3.95 (s, 3H), 3.92 (s, 3H)

### <Example 45> Preparation of 2,3-dimethoxy-9-(3,4,5-trimethoxyphenyl)dibenzo[b,e]thiepin-11(6H)-one(HN2305)

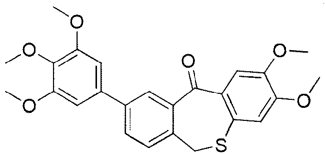

According to the preparation method of General Preparation Method 6, 9-bromo-2,3-dimethoxydibenzo[b,e]thiepin-11(6H)-one(30 mg, 0.082 mmol) of Example 44 and 3,4,5-trimethoxyphenylboronic acid(26.1 mg, 0.123 mmol) were allowed to react, and then the reaction mixture was purified by column chromatography using 17% ethyl acetate and 50% methylene chloride solvent in n-hexane and then decanted with n-hexane to obtain 2,3-dimethoxy-9-(3,4,5-trimethoxyphenyl)dibenzo[b,e]thiepin-11(6H)-one(22 mg, 59%).
¹H NMR (500 MHz, CDCl₃) δ 7.87 (s, 1H), 7.82 (d, *J =* 2.2 Hz, 1H), 7.63 (dd, *J =* 8.0, 1.9 Hz, 1H), 7.26 (d, *J =* 8.0 Hz, 1H), 6.75 (d, *J =* 4.6 Hz, 3H), 4.06 (s, 2H), 3.95 (s, 3H), 3.90 (s, 3H), 3.90 (s, 6H) 3.87 (s, 3H)

### <Example 46> Preparation of 2,3-dimethoxy-9-(4-methoxyphenyl)dibenzo[b,e]thiepin-11(6H)-one(HN2306)

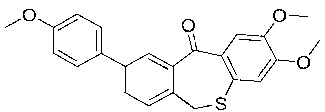

According to the preparation method of General Preparation Method 6, 9-bromo-2,3-dimethoxydibenzo[b,e]thiepin-11(6H)-one(30 mg, 0.082 mmol) of Example 44 and 4-methoxyphenylboronic acid(18.7 mg, 0.123 mmol) were allowed to react, and then the reaction mixture was purified by column chromatography using 6% ethyl acetate and 50% methylene chloride solvent in n-hexane and then decanted with n-hexane to obtain 2,3-dimethoxy-9-(4-methoxyphenyl)dibenzo[b,e]thiepin-11(6H)-one(31.3 mg, 97%).
¹H NMR (500 MHz, CDCl₃) δ 7.85 (s, 1H), 7.82 (d, *J =* 1.9 Hz, 1H), 7.62 (dd, *J* = 7.7, 1.9 Hz, 1H), 7.51 (d, *J =* 8.8 Hz, 2H), 7.24 (d, *J =* 8.0 Hz, 1H), 6.94 (d, *J =* 8.8 Hz, 2H), 6.75 (s, 1H), 4.05 (s, 2H), 3.94 (s, 3H), 3.90 (s,3H), 3.83 (s, 3H)

### <Example 47> Preparation of 2,3-dimethoxy-9-(p-tolyl)dibenzo[b,e]thiepin-11(6H)-one(HN2307)

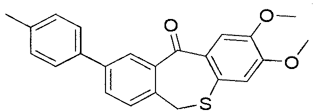

According to the preparation method of General Preparation Method 6, 9-bromo-2,3-dimethoxydibenzo[b,e]thiepin-11(6H)-one(30 mg, 0.082 mmol) of Example 44 and 4-methylphenylboronic acid(16.8 mg, 0.123 mmol) were allowed to react, and then the reaction mixture was purified by column chromatography using 5% ethyl acetate and 50% methylene chloride solvent in n-hexane to obtain 2,3-dimethoxy-9-(p-tolyl)dibenzo[b,e]thiepin-11(6H)-one(30 mg, 98%).
¹H NMR (500 MHz, CDCl₃) δ 7.86 (s, 1H), 7.85 (d, *J =* 1.9 Hz, 1H), 7.64 (dd, *J =* 8.0, 1.9 Hz, 1H), 7.47 (d, *J =* 8.0 Hz, 2H), 7.25 (s, 1H), 7.22 (d, *J =* 7.7 Hz, 2H), 6.75 (s, 1H), 4.05 (s, 2H), 3.94 (s, 3H), 3.90 (s,3H), 2.37 (s, 3H)

### <Example 48> Preparation of 9-(3-furanyl)-2,3-dimethoxydibenzo[b,e]thiepin-11(6H)-one(HN2308)

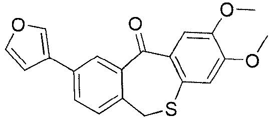

According to the preparation method of General Preparation Method 6, 9-bromo-2,3-dimethoxydibenzo[b,e]thiepin-11(6H)-one(30 mg, 0.082 mmol) of Example 44 and 3-furylboronic acid(13.8 mg, 0.123 mmol) were allowed to react, and then the reaction mixture was purified by column chromatography using 6% ethyl acetate and 50% methylene chloride solvent in n-hexane to obtain 9-(3-furanyl)-2,3-dimethoxydibenzo[b,e]thiepin-11(6H)-one(24.4 mg, 84%).
¹H NMR (500 MHz, CDCl₃) δ 7.85 (s, 1H), 7.74 (d, *J =* 1.9 Hz, 1H), 7.73 (s, 1H), 7.55 (dd, *J =* 8.0, 1.9 Hz, 1H), 7.45 (d, *J =* 1.7 Hz, 1H), 7.20 (d, *J =* 8.1 Hz, 1H), 6.75 (s, 1H), 6.69 (m, 1H), 4.02 (s, 2H), 3.95 (s, 3H), 3.90 (s,3H)

### <Example 49> Preparation of 9-(4-chlorophenyl)-2,3-dimethoxydibenzo[b,e]thiepin-11(6H)-one(HN2309)

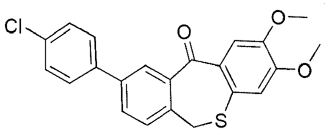

According to the preparation method of General Preparation Method 6, 9-bromo-2,3-dimethoxydibenzo[b,e]thiepin-11(6H)-one(30 mg, 0.082 mmol) of Example 44 and 4-chlorophenylboronic acid(19. 2 mg, 0.123 mmol) were allowed to react, and then the reaction mixture was purified by column chromatography using 5% ethyl acetate and 30% methylene chloride solvent in n-hexane and then decanted with n-hexane to obtain 9-(4-chlorophenyl)-2,3-dimethoxydibenzo[b,e]thiepin-11(6H)-one(29.1 mg, 89%).
¹H NMR (500 MHz, CDCl₃) δ 7.85 (s, 1H), 7.83 (d, *J =* 1.9 Hz, 1H), 7.62 (dd, *J* = 7.9, 2.1 Hz, 1H), 7.50 (m, 2H), 7.38 (m, 2H), 7.27 (d, *J =* 8.1 Hz, 1H), 6.75 (s, 1H), 4.02 (s, 2H), 3.95 (s, 3H), 3.90 (s,3H)

### <Example 50> Preparation of 10,11-dihydro-5H-benzo[4',5']cyclohepta[1',2':4,5]benzo[1,2-d][1,3]dioxol-5-one(HN2310)

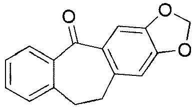

### Step 1: Preparation of methyl 2-(2-(benzo[d][1,3]dioxol-5-yl)ethyl)benzoate

(2-(Methoxycarbonyl)benzyl)triphenylphosphonium bromide(170.2 mg, 0.346 mmol) used in step 1 of Example 43 and piperonal(40 mg, 0.266 mmol) were allowed to react, and then the next reaction was carried out without purification. The concentrated mixture was dissolved in 3 mL of MeOH, and a catalytic amount of Pd/C was added. The mixture was reacted by purging H₂ gas, and then purified by column chromatography using 5% ethyl acetate in n-hexane to obtain methyl 2-(2-(benzo[d][1,3]dioxol-5-yl)ethyl)benzoate(68.3 mg, 90%).

### Step 2: Preparation of 2-(2-(benzo[d][1,3]dioxol-5-yl)ethyl)benzoic acid

According to the preparation method of step 2 of General Preparation Method 4, methyl 2-(2-(benzo[d][1,3]dioxol-5-yl)ethyl)benzoate(61.2 mg, 0.215 mmol) prepared in step 1 was reacted to obtain 2-(2-(benzo[d][1,3]dioxol-5-yl)ethyl)benzoic acid(56.7 mg, 97%).

### Step 3: Preparation of 10,11-dihydro-5H-benzo[4',5']cyclohepta[1',2':4,5]benzo[1,2-d][1,3]dioxol-5-one

According to the preparation method of step 3 of General Preparation Method 4, 2-(2-(benzo[d][1,3]dioxol-5-yl)ethyl)benzoic acid(50.3 mg, 0.186 mmol) prepared in step 2 was reacted, and then the reaction mixture was purified by column chromatography using 66% methylene chloride solvent in n-hexane to obtain 10,11-dihydro-5H-benzo[4',5']cyclohepta[1',2':4,5]benzo[1,2-d][1,3]dioxol-5-one(46.6 mg, 99%).
¹H NMR (500 MHz, CDCl₃) δ 7.98 (dd, *J =* 7.8 Hz, 1H), 7.61 (s, 1H), 7.42 (t, *J =* 7.4 Hz, 1H), 7.32 (t, *J =* 7.5 Hz, 1H), 7.20 (d, *J =* 7.5 Hz, 1H), 6.66 (s, 1H), 6.01 (s, 2H), 3.22 - 3.06 (m, 4H)

### <Example 51> Preparation of 2,3-difluoro-7,8-dimethoxy-10,11-dihydro-5H-dibenzo[a,d][7]annulen-5-one(HN2311)

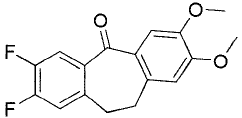

### Step 1: Preparation of methyl 4,5-difluoro-2-methylbenzoate

The process was carried out in the same manner as in the preparation method of step 1 of Example 3.

### Step 2: Preparation of methyl 2-bromomethyl-4,5-difluorobenzoate

The process was carried out in the same manner as in the preparation method of step 2 of Example 3.

### Step 3: Preparation of methyl 2-(3,4-dimethoxyphenethyl)-4,5-difluorobenzoate

According to the preparation method of step 1 of General Preparation Method 4, methyl 2-bromomethyl-4,5-difluorobenzoate(510.9 mg, 1.927 mmol) prepared in step 2 and triphenylphosphine(606.7 mg, 2.313 mmol) were allowed to react, and the resultant (4,5-difluoro-2-(methoxycarbonyl)benzyl)triphenylphosphonium bromide(885.2 mg, 0.234 mmol) was reacted with 3,4-dimethoxybenzaldehyde(214.5 mg, 1.291 mmol), and then the next reaction was carried out without purification. The concentrated mixture was dissolved in 13 mL of MeOH, and a catalytic amount of Pd/C was added. The mixture was reacted by purging H₂ gas, and then purified by column chromatography using 20% ethyl acetate in n-hexane to obtain methyl 2-(3,4-dimethoxyphenethyl)-4,5-difluorobenzoate(331.1 mg, 76%).

### Step 4: Preparation of 2-(3,4-dimethoxyphenethyl)-4,5-difluorobenzoic acid

According to the preparation method of step 2 of General Preparation Method 4, methyl 2-(3,4-dimethoxyphenethyl)-4,5-difluorobenzoate(318.4 mg, 0.946 mmol) prepared in step 3 was reacted to obtain 2-(3,4-dimethoxyphenethyl)-4,5-difluorobenzoic acid(303.3 mg, 99%).

### Step 5: Preparation of 2,3-difluoro-7,8-dimethoxy-10,11-dihydro-5H-dibenzo[a,d][7]annulen-5-one

According to the preparation method of step 3 of General Preparation Method 4, 2-(3,4-dimethoxyphenethyl)-4,5-difluorobenzoic acid(298.9 mg, 0.924 mmol) prepared in step 4 was reacted, and then the reaction mixture was purified by column chromatography using 66~100% methylene chloride solvent in n-hexane to obtain 2,3-difluoro-7,8-dimethoxy-10,11-dihydro-5H-dibenzo[a,d][7]annulen-5-one(275 mg, 97%).
¹H NMR (500 MHz, DMSO-d₆) δ 7.83 (dd, *J =* 8.3, 11.8 Hz, 1H), 7.58 (s, 1H), 7.48 (dd, *J* = 7.8, 11.2 Hz, 1H), 6.93 (s, 1H), 3.85 (s, 3H), 3.79 (s, 3H), 3.13 - 3.08 (m, 4H)

### <Example 52> Preparation of 8-(cyclopropylamino)-9-fluoro-[1,3]dioxolo[4',5':4,5]benzo[1,2-b]benzo[e]oxepin-11(6H)-one(HN2312)

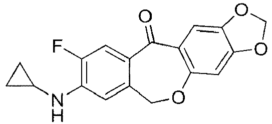

According to the preparation method of General Preparation Method 5, 8,9-difluoro[2]benzoxepino[3,4-f]-1,3-benzodioxol-11(6H)-one(25 mg, 0.086 mmol) of Example 3 and cyclopropylamine(0.03 mL, 0.43 mmol) were allowed to react, and then the reaction mixture was purified by column chromatography using 66-100% methylene chloride solvent in n-hexane to obtain 8-(cyclopropylamino)-9-fluoro-[1,3]dioxolo[4',5':4,5]benzo[1,2-b]benzo[e]oxepin-11(6H)-one(24.1 mg, 85%).
¹H NMR (500 MHz, DMSO-d₆) δ 7.53 (d, *J =* 13.0 Hz, 1H), 7.51 (s, 1H), 7.00 (d, *J =* 8.0 Hz, 1H), 6.67 (s, 1H), 6.10 (s, 2H), 5.16 (s, 2H), 2.50 - 2.48 (m, 1H), 0.80 - 0.76 (m, 2H), 0.55 - 0.52 (m, 2H)

### <Example 53> Preparation of 8-(cyclopentylamino)-9-fluoro-[1,3]dioxolo[4',5':4,5]benzo[1,2-b]benzo[e]oxepin-11(6H)-one(HN2313)

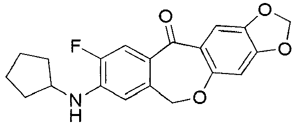

According to the preparation method of General Preparation Method 5, 8,9-difluoro[2]benzoxepino[3,4-f]-1,3-benzodioxol-11(6H)-one(25 mg, 0.086 mmol) of Example 3 and cyclopentylamine(0.042 mL, 0.43 mmol) were allowed to react, and then the reaction mixture was purified by column chromatography using 66-80% methylene chloride solvent in n-hexane and then decanted with n-hexane to obtain 8-(cyclopentylamino)-9-fluoro-[1,3]dioxolo[4',5':4,5]benzo[1,2-b]benzo[e]oxepin-11(6H)-one(24.7 mg, 80%).
¹H NMR (500 MHz, DMSO-d₆) δ 7.53 (d, *J =* 13.4 Hz, 1H), 7.51 (s, 1H), 6.79 (d, *J =* 8.4 Hz, 1H), 6.67 (s, 1H), 6.10 (s, 2H), 5.13 (s, 2H), 3.91 - 3.85 (m, 1H), 2.02 - 1.96 (m, 2H), 1.73 - 1.65 (m, 2H), 1.59 - 1.52 (m, 4H)

### <Example 54> Preparation of 2-(dimethylamino)-3-fluoro-7,8-dimethoxy-10,11-dihydro-5H-dibenzo[a,d][7]annulen-5-one(HN2314)

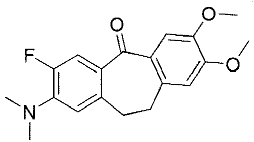

According to the preparation method of General Preparation Method 5, 2,3-difluoro-7,8-dimethoxy-10,11-dihydro-5H-dibenzo[a,d][7]annulen-5-one(25 mg, 0.082 mmol) of Example 51 and dimethylamine(0.1 mL) were allowed to react, and then the reaction mixture was purified by column chromatography using 50% methylene chloride and 3% ethyl acetate solvent in n-hexane to obtain 2-(dimethylamino)-3-fluoro-7,8-dimethoxy-10,11-dihydro-5H-dibenzo[a,d][7]annulen-5-one(24.1 mg, 89%).
¹H NMR (500 MHz, DMSO-d₆) δ 7.69 (d, *J =* 16.4 Hz, 1H), 7.58 (s, 1H), 6.90 (s, 1H), 6.73 (d, *J =* 9.2 Hz, 1H), 3.84 (s, 3H), 3.78 (s, 3H), 3.04 (s, 4H), 2.96 (d, *J =* 1.5 Hz, 6H)

### <Example 55> Preparation of 3-fluoro-7,8-dimethoxy-2-(piperidin-1-yl)-10,11-dihydro-5H-dibenzo[a,d] [7] annulen-5-one(HN2315)

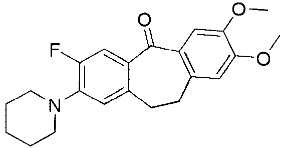

According to the preparation method of General Preparation Method 5, 2,3-difluoro-7,8-dimethoxy-10,11-dihydro-5H-dibenzo[a,d][7]annulen-5-one(25 mg, 0.082 mmol) of Example 51 and piperidine(0.04 mL, 0.41 mmol) were allowed to react, and then the reaction mixture was purified by column chromatography using 33~50% methylene chloride and 3% ethyl acetate solvent in n-hexane to obtain 3-fluoro-7,8-dimethoxy-2-(piperidin-1-yl)-10,11-dihydro-5H-dibenzo[a,d][7]annulen-5-one(29.4 mg, 97%).
¹H NMR (500 MHz, DMSO-d₆) δ 7.67 (d, *J =* 15.7 Hz, 1H), 7.57 (s, 1H), 6.91 (s, 1H), 6.88 (d, *J =* 8.8 Hz, 1H), 3.84 (s, 3H), 3.78 (s, 3H), 3.17 - 3.14 (m, 4H), 3.05 (s, 4H), 1.66 - 1.61 (m, 4H), 1.58 - 1.54 (m, 2H)

### <Example 56> Preparation of 3-fluoro-7,8-dimethoxy-2-(4-phenylpiperazin-1-yl)-10,11-dihydro-5H-dibenzo[a,d][7]annulen-5-one(HN2316)

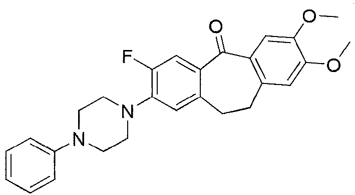

According to the preparation method of General Preparation Method 5, 2,3-difluoro-7,8-dimethoxy-10,11-dihydro-5H-dibenzo[a,d][7]annulen-5-one(25 mg, 0.082 mmol) of Example 51 and 1-phenylpiperazine(0.037 mL, 0.24 mmol) were allowed to react, and then the reaction mixture was purified by column chromatography using 25% ethyl acetate solvent in n-hexane to obtain 3-fluoro-7,8-dimethoxy-2-(4-phenylpiperazin-1-yl)-10,11-dihydro-5H-dibenzo[a,d][7]annulen-5-one(31.1 mg, 85%).
¹H NMR (500 MHz, DMSO-d₆) δ 7.71 (d, *J =* 15.3 Hz, 1H), 7.58 (s, 1H), 7.25 - 7.22 (m, 2H), 7.00 - 6.96 (m, 3H), 6.92 (s, 1H), 6.81 (t, *J =* 7.3 Hz, 1H), 3.85 (s, 3H), 3.79 (s, 3H), 3.36 - 3.35 (m, 4H), 3.30 - 3.28 (m, 4H), 3.08 (s, 4H)

### <Example 57> Preparation of 2-(benzylamino)-3-fluoro-7,8-dimethoxy-10,11-dihydro-5H-dibenzo[a,d][7]annulen-5-one(HN2317)

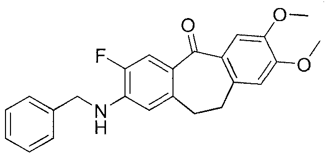

According to the preparation method of General Preparation Method 5, 2,3-difluoro-7,8-dimethoxy-10,11-dihydro-5H-dibenzo[a,d][7]annulen-5-one(25 mg, 0.082 mmol) of Example 51 and benzylamine (0.045 mL, 0.41 mmol) were allowed to react, and then the reaction mixture was purified by column chromatography using 25% ethyl acetate solvent in n-hexane and then decanted with n-hexane to obtain 2-(benzylamino)-3-fluoro-7,8-dimethoxy-10,11-dihydro-5H-dibenzo[a,d][7]annulen-5-one (21.9 mg, 68%).
¹H NMR (500 MHz, DMSO-d₆) δ 7.70 (d, *J =* 13.8 Hz, 1H), 7.56 (s, 1H), 7.37 - 7.31 (m, 4H), 7.23 (t, *J =* 7.3 Hz, 1H), 6.48 (d, *J =* 8.8 Hz, 1H), 6.85 (s, 1H), 4.44 (d, *J =* 5.4 Hz, 2H), 3.84 (s, 3H), 3.76 (s, 3H), 2.97 - 2.95 (m, 2H), 2.91 - 2.89 (m, 2H)

### <Example 58> Preparation of 8-(cyclopropylamino)-7-fluoro-[1,3]dioxolo[4',5':4,5]benzo[1,2-b]benzo[e]oxepin-11(6H)-one(HN2318)

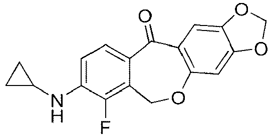

8-(Cyclopropylamino)-7-fluoro-[1,3]dioxolo[4',5':4,5]benzo[1,2-b]benzo[e]oxepin-11(6H)-one(21.8 mg, 77%) was prepared in the same manner as in Example 52, except that in Example 52, 7,8-difluoro[2]benzoxepino[3,4-f]-1,3-benzodioxol-11(6H)-one(25 mg, 0.086 mmol) of Example 20 was used instead of 8,9-difluoro[2]benzoxepino[3,4-f]-1,3-benzodioxol-11(6H)-one.
¹H NMR (500 MHz, DMSO-d₆) δ 7.69 (d, *J =* 8.8 Hz, 1H), 7.51 (s, 1H), 7.06 (d, *J =* 8.4 Hz, 1H), 6.68 (s, 1H), 6.10 (s, 2H), 5.24 (s, 2H), 2.49 - 2.47 (m, 1H), 0.78 - 0.74 (m, 2H), 0.52 - 0.49 (m, 2H)

### <Example 59> Preparation of 8-(cyclopentylamino)-7-fluoro-[1,3]dioxolo[4',5':4,5]benzo[1,2-b]benzo[e]oxepin-11(6H)-one(HN2319)

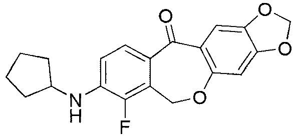

8-(Cyclopentylamino)-7-fluoro-[1,3]dioxolo[4',5':4,5]benzo[1,2-b]benzo[e]oxepin-11(6H)-one(22.3 mg, 73%) was prepared in the same manner as in Example 53, except that in Example 53, 7,8-difluoro[2]benzoxepino[3,4-f]-1,3-benzodioxol-11(6H)-one(25 mg, 0.086 mmol) of Example 20 was used instead of 8,9-difluoro[2]benzoxepino[3,4-f]-1,3-benzodioxol-11(6H)-one.
¹H NMR (500 MHz, DMSO-d₆) δ 7.65 (d, *J =* 8.8 Hz, 1H), 7.51 (s, 1H), 6.82 (t, *J =* 8.6 Hz, 1H), 6.68 (s, 1H), 6.10 (s, 2H), 5.25 (s, 2H), 3.89 - 3.83 (m, 1H), 1.99 - 1.93 (m, 2H), 1.69 - 1.64 (m, 2H), 1.59 - 1.54 (m, 4H)

### <Example 60> Preparation of 2-(cyclopropylamino)-3-fluoro-7,8-dimethoxy-10,11-dihydro-5H-dibenzo[a,d][7]annulen-5-one(HN2320)

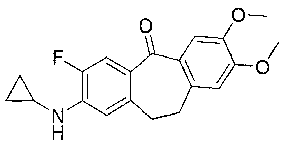

According to the preparation method of General Preparation Method 5, 2,3-difluoro-7,8-dimethoxy-10,11-dihydro-5H-dibenzo[a,d][7]annulen-5-one(25 mg, 0.082 mmol) of Example 51 and cyclopropylamine(0.028 mL, 0.41 mmol) were allowed to react, and then the reaction mixture was purified by column chromatography using 25% ethyl acetate solvent in n-hexane and then decanted with n-hexane to obtain 2-(cyclopropylamino)-3-fluoro-7,8-dimethoxy-10,11-dihydro-5H-dibenzo[a,d][7]annulen-5-one(19.2 mg, 68%).
¹H NMR (500 MHz, DMSO-d₆) δ 7.68 (d, *J =* 13.7 Hz, 1H), 7.58 (s, 1H), 6.89 (s, 1H), 6.81 (d, *J =* 8.8 Hz, 1H), 3.84 (s, 3H), 3.77 (s, 3H), 3.04 (s, 4H), 0.83 - 0.71 (m, 2H), 0.58 - 0.47 (m, 2H)

### <Example 61> Preparation of 2-(cyclopentylamino)-3-fluoro-7,8-dimethoxy-10,11-dihydro-5H-dibenzo[a,d][7]annulen-5-one(HN2321)

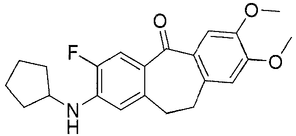

According to the preparation method of General Preparation Method 5, 2,3-difluoro-7,8-dimethoxy-10,11-dihydro-5H-dibenzo[a,d][7]annulen-5-one(25 mg, 0.082 mmol) of Example 51 and cyclopentylamine(0.04 mL, 0.41 mmol) were allowed to react, and then the reaction mixture was purified by column chromatography using 25% ethyl acetate solvent in n-hexane to obtain 2-(cyclopentylamino)-3-fluoro-7,8-dimethoxy-10,11-dihydro-5H-dibenzo[a,d][7]annulen-5-one(24.5 mg, 81%).
¹H NMR (500 MHz, DMSO-d₆) δ 7.68 (d, *J =* 14.1 Hz, 1H), 7.57 (s, 1H), 6.88 (s, 1H), 6.57 (d, *J =* 8.8 Hz, 1H), 3.89 - 3.85 (m, 1H), 3.83 (s, 3H), 3.77 (s, 3H), 3.02 (s, 4H), 2.00 - 1.93 (m, 2H), 1.73 - 1.50 (m, 6H)

### <Example 62> Preparation of 8-((2,4-dichlorophenethyl)amino)-9-fluoro-[1,3]dioxolo[4',5':4,5]benzo[1,2-b]benzo[e]oxepin-11(6H)-one(HN2322)

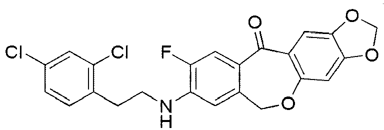

According to the preparation method of General Preparation Method 5, 8,9-difluoro[2]benzoxepino[3,4-f]-1,3-benzodioxol-11(6H)-one(25 mg, 0.086 mmol) of Example 3 and 2-(2,4-dichlorophenyl)ethylamine(0.032 mL, 0.215 mmol) were allowed to react, and then the reaction mixture was purified by column chromatography using 16~20% ethyl acetate solvent in n-hexane to obtain 8-((2,4-dichlorophenethyl)amino)-9-fluoro-[1,3]dioxolo[4',5':4,5]benzo[1,2-b]benzo[e]oxepin-11(6H)-one(35.0 mg, 88%).
¹H NMR (500 MHz, DMSO-d₆) δ 7.58 (d, *J =* 1.9 Hz, 1H), 7.54 (d, *J =* 13.0 Hz, 1H), 7.51 (s, 1H), 7.43 (d, *J =* 8.1 Hz, 1H), 7.37 (dd, *J =* 2.1, 8.2 Hz, 1H), 6.81 (d, *J* = 8.4 Hz, 1H), 6.67 (s, 1H), 6.10 (s, 2H), 5.11 (s, 2H), 3.47 - 3.43 (m, 2H), 3.01 (t, *J =* 7.3 Hz, 2H)

### <Example 63> Preparation of 9-fluoro-8-((2-methoxybenzyl)amino)-[1,3] dioxolo [4',5':4,5] benzo [1,2-b] benzo [e] oxepin-11 (6H)-one(HN2323)

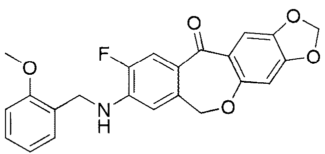

According to the preparation method of General Preparation Method 5, 8,9-difluoro[2]benzoxepino[3,4-f]-1,3-benzodioxol-11(6H)-one(25 mg, 0.086 mmol) of Example 3 and 2-methoxybenzylamine(0.056 mL, 0.43 mmol) were allowed to react, and then the reaction mixture was purified by column chromatography using 20% ethyl acetate solvent in n-hexane to obtain 9-fluoro-8-((2-methoxybenzyl)amino)-[1,3]dioxolo[4',5':4,5]benzo[1,2-b]benzo[e]oxepin-11(6H)-one(32.2 mg, 92%).
¹H NMR (500 MHz, DMSO-d₆) δ 7.56 (d, *J =* 13.4 Hz, 1H), 7.50 (s, 1H), 7.25 - 7.22 (m, 1H), 7.17 (d, *J =* 7.7 Hz, 1H), 7.02 - 6.98 (m, 2H), 6.88 (t, *J =* 6.9 Hz, 1H), 6.63 (s, 2H), 6.09 (s, 2H), 5.03 (s, 2H), 4.42 (d, *J =* 5.4 Hz, 2H), 3.86 (s, 3H)

### <Example 64> Preparation of 8,9-difluoro-2,3-dimethoxydibenzo[b,e]thiepin-11(6H)-one(HN2324)

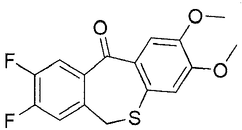

### Step 1: Preparation of methyl 4,5-difluoro-2-methylbenzoate

The process was carried out in the same manner as in the preparation method of step 1 of Example 3.

### Step 2: Preparation of methyl 2-bromomethyl-4,5-difluorobenzoate

The process was carried out in the same manner as in the preparation method of step 2 of Example 3.

### Step 3: Preparation of methyl 2-(((3,4-dimethoxyphenyl)thio)methyl)-4,5-difluorobenzoate

According to the preparation method of step 1 of General Preparation Method 3, methyl 2-bromomethyl-4,5-difluorobenzoate(500 mg, 1.886 mmol) prepared in step 2 and 3,4-dimethoxythiophenol(321.1 mg, 1.886 mmol) were allowed to react, and then the reaction mixture was purified by column chromatography using 16~20% ethyl acetate solvent in n-hexane to obtain methyl 2-(((3,4-dimethoxyphenyl)thio)methyl)-4,5-difluorobenzoate(602.6 mg, 90%).

### Step 4: Preparation of 2-(((3,4-dimethoxyphenyl)thio)methyl)-4,5-difluorobenzoic acid

According to the preparation method of step 2 of General Preparation Method 3, methyl 2-(((3,4-dimethoxyphenyl)thio)methyl)-4,5-difluorobenzoate(581.8 mg, 1.642 mmol) prepared in step 3 was reacted to obtain 2-(((3,4-dimethoxyphenyl)thio)methyl)-4,5-difluorobenzoic acid(527.3 mg, 94%).

### Step 5: Preparation of 8,9-difluoro-2,3-dimethoxydibenzo[b,e]thiepin-11(6H)-one

According to the preparation method of step 3 of General Preparation Method 3, 2-(((3,4-dimethoxyphenyl)thio)methyl)-4,5-difluorobenzoic acid(522.4 mg, 1.535 mmol) prepared in step 4 was reacted, and then the reaction mixture was purified by column chromatography using 50% methylene chloride and 1 % ethyl acetate solvent in n-hexane to obtain 8,9-difluoro-2,3-dimethoxydibenzo[b,e]thiepin-11(6H)-one(447.5 mg, 90%).
¹H NMR (500 MHz, CDCl₃) δ 7.83 (s, 1H), 7.54 (dd, *J =* 7.8, 10.9 Hz, 1H), 7.00 (dd, *=* 7.3, 9.9 Hz, 1H), 6.75 (s, 1H), 3.96 (s, 2H), 3.93 (s, 3H), 3.90 (s, 3H)

### <Example 65> Preparation of 9-fluoro-8-((3-methoxybenzyl)amino)-[1,3]dioxolo[4',5':4,5]benzo[1,2-b]benzo[e]oxepin-11(6H)-one(HN2325)

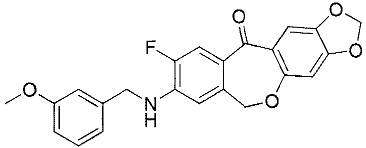

According to the preparation method of General Preparation Method 5, 8,9-difluoro[2]benzoxepino[3,4-f]-1,3-benzodioxol-11(6H)-one(25 mg, 0.086 mmol) of Example 3 and 3-methoxybenzylamine(0.057 mL, 0.43 mmol) were allowed to react, and then the reaction mixture was purified by column chromatography using 20% ethyl acetate solvent in n-hexane to obtain 9-fluoro-8-((3-methoxybenzyl)amino)-[1,3]dioxolo[4',5':4,5]benzo[1,2-b]benzo[e]oxepin-11(6H)-one(25.8 mg, 73%).
¹H NMR (500 MHz, DMSO-d₆) δ 7.55 (d, *J =* 13.0 Hz, 1H), 7.49 (s, 1H), 7.25 - 7.22 (m, 2H), 6.94 (d, *J =* 7.3 Hz, 2H), 6.81 - 6.79 (m, 1H), 6.70 (d, *J =* 8.4 Hz, 1H), 6.63 (s, 1H), 6.09 (s, 2H), 5.03 (s, 2H), 4.43 (d, *J =* 5.4 Hz, 2H), 3.72 (s, 3H)

### <Example 66> Preparation of 9-fluoro-8-((4-methoxybenzyl)amino)-[1,3] dioxolo [4',5':4,5] benzo [1,2-b] benzo [e] oxepin-11 (6H)-one(HN2326)

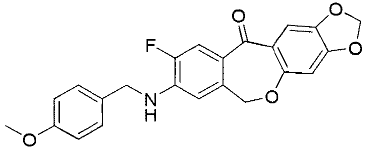

According to the preparation method of General Preparation Method 5, 8,9-difluoro[2]benzoxepino[3,4-f]-1,3-benzodioxol-11(6H)-one(25 mg, 0.086 mmol) of Example 3 and 4-methoxybenzylamine(0.056 mL, 0.43 mmol) were allowed to react, and then the reaction mixture was purified by column chromatography using 20% ethyl acetate solvent in n-hexane to obtain 9-fluoro-8-((4-methoxybenzyl)amino)-[1,3]dioxolo[4',5':4,5]benzo[1,2-b]benzo[e]oxepin-11(6H)-one(25.5 mg, 72%).
¹H NMR (500 MHz, DMSO-d₆) δ 7.54 (d, *J =* 13.4 Hz, 1H), 7.49 (s, 1H), 7.30 (d, *J =* 8.4 Hz, 2H), 7.18 (t, *J =* 5.5 Hz, 1H), 6.88 (d, *J =* 8.8 Hz, 2H), 6.71 (d, *J =* 8.4 Hz, 1H), 6.63 (s, 1H), 6.09 (s, 2H), 5.04 (s, 2H), 4.38 (d, *J =* 6.1 Hz, 2H), 3.71 (s, 3H)

### <Example 67> Preparation of 9-fluoro-8-((3-methoxyphenethyl)amino)-[1,3]dioxolo[4',5':4,5]benzo[1,2-b]benzo[e]oxepin-11(6H)-one(HN2327)

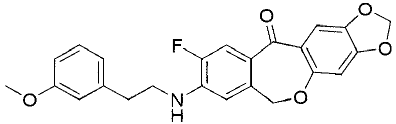

According to the preparation method of General Preparation Method 5, 8,9-difluoro[2]benzoxepino[3,4-f]-1,3-benzodioxol-11(6H)-one(25 mg, 0.086 mmol) of Example 3 and 2-(3-methoxyphenyl)ethylamine(0.062 mL, 0.43 mmol) were allowed to react, and then the reaction mixture was purified by column chromatography using 20% ethyl acetate solvent in n-hexane to obtain 9-fluoro-8-((3-methoxyphenethyl)amino)-[1,3]dioxolo[4',5':4,5]benzo[1,2-b]benzo[e]oxepin-11(6H)-one(28.7 mg, 79%).
¹H NMR (500 MHz, DMSO-d₆) δ 7.54 (d, *J =* 13.4 Hz, 1H), 7.51 (s, 1H), 7.21 (t, *J =* 8.0 Hz, 1H), 6.87 - 6.85 (m, 2H), 6.82 (d, *J =* 8.4 Hz, 1H), 6.79 - 6.76 (m, 1H), 6.67 (s, 1H), 6.59 (s, 1H), 6.10 (s, 2H), 5.13 (s, 2H), 3.73 (s, 3H), 3.46 - 3.40 (m, 2H), 2.87 (t, *J* = 7.6 Hz, 2H)

### <Example 68> Preparation of 8-((2,4-dimethoxybenzyl)amino)-9-fluoro-[1,3]dioxolo[4',5':4,5]benzo[1,2-b]benzo[e]oxepin-11(6H)-one(HN2328)

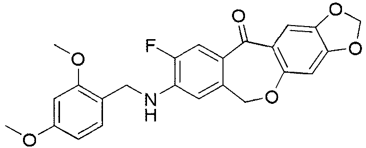

According to the preparation method of General Preparation Method 5, 8,9-difluoro[2]benzoxepino[3,4-f]-1,3-benzodioxol-11(6H)-one(25 mg, 0.086 mmol) of Example 3 and 2,4-dimethoxybenzylamine(0.065 mL, 0.43 mmol) were allowed to react, and then the reaction mixture was purified by column chromatography using 16~20% ethyl acetate solvent in n-hexane to obtain 8-((2,4-dimethoxybenzyl)amino)-9-fluoro-[1,3]dioxolo[4',5':4,5]benzo[1,2-b]benzo[e]oxepin-11(6H)-one(31.7 mg, 84%).
¹H NMR (500 MHz, DMSO-d₆) δ 7.55 (d, *J =* 13.4 Hz, 1H), 7.50 (s, 1H), 7.09 (d, *J =* 8.4 Hz, 1H), 6.90 (s, 1H), 6.64 - 6.63 (m, 2H), 6.57 (d, *J =* 2.3 Hz, 1H), 6.46 (dd, *J =* 8.4, 2.3 Hz, 1H), 6.09 (s, 2H), 5.04 (s, 2H), 4.33 (d, *J =* 5.7 Hz, 2H), 3.84 (s, 3H), 3.72 (s, 3H)

### <Example 69> Preparation of 8-((3,4-dimethoxybenzyl)amino)-9-fluoro-[1,3]dioxolo[4',5':4,5]benzo[1,2-b]benzo[e]oxepin-11(6H)-one(HN2329)

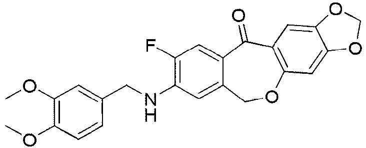

According to the preparation method of General Preparation Method 5, 8,9-difluoro[2]benzoxepino[3,4-f]-1,3-benzodioxol-11(6H)-one(25 mg, 0.086 mmol) of Example 3 and 3,4-dimethoxybenzylamine(0.039 mL, 0.258 mmol) were allowed to react, and then the reaction mixture was purified by column chromatography using 20~25% ethyl acetate solvent in n-hexane to obtain 8-((3,4-dimethoxybenzyl)amino)-9-fluoro-[1,3]dioxolo[4',5':4,5]benzo[1,2-b]benzo[e]oxepin-11(6H)-one(29.1 mg, 77%).
¹H NMR (500 MHz, DMSO-d₆) δ 7.54 (d, *J =* 13.0 Hz, 1H), 7.49 (s, 1H), 7.16 (t, *J =* 6.1 Hz, 1H), 7.03 (s,1H), 6.91 - 6.87 (m, 2H), 6.73 (d, *J =* 8.4 Hz, 1H), 6.63 (s, 1H), 6.09 (s, 2H), 5.05 (s, 2H), 4.37 (d, *J =* 5.7 Hz, 2H), 3.73 (s, 3H), 3.70 (s, 3H)

### <Example 70> Preparation of 8-Bromo[2]benzoxepino[3,4-f]-1,3-benzodioxol-11(6H)-one(HN2336)

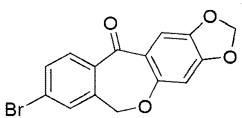

### Step 1: Preparation of methyl 2-((benzo[d][1,3]dioxol-5-yloxy)methyl)-4-bromobenzoate

According to the preparation method of step 1 of General Preparation Method 2, methyl 4-bromo-2-(bromomethyl)benzoate (100 mg, 0.325 mmol) and sesamol(53.8 mg, 0.389 mmol) were allowed to react, and then the reaction mixture was purified by column chromatography using 33% methylene chloride solvent in n-hexane to obtain methyl 2-((benzo[d][1,3]dioxol-5-yloxy)methyl)-4-bromobenzoate(82.0 mg, 69%).

### Step 2: Preparation of 2-((benzo[d][1,3]dioxol-5-yloxy)methyl)-4-bromobenzoic acid

According to the preparation method of step 2 of General Preparation Method 2, methyl 2-((benzo[d][1,3]dioxol-5-yloxy)methyl)-4-bromobenzoate(76.8 mg, 0.210 mmol) prepared in step 1 was reacted to obtain 2-((benzo[d][1,3]dioxol-5-yloxy)methyl)-4-bromobenzoic acid (62.4 mg, 84%).

### Step 3: Preparation of 8-bromo[2]benzoxepino[3,4-f]-1,3-benzodioxol-11(6H)-one

According to the preparation method of step 3 of General Preparation Method 2, 2-((benzo[d][1,3]dioxol-5-yloxy)methyl)-4-bromobenzoic acid(57.8 mg, 0.165 mmol) prepared in step 2 was reacted, and then the reaction mixture was purified by column chromatography using 50% methylene chloride solvent in n-hexane to obtain 8-bromo[2]benzoxepino[3,4-f]-1,3-benzodioxol-11(6H)-one(51.6 mg, 94%).
¹H NMR (500 MHz, CDCl₃) δ 7.83 (d, *J =* 8.3 Hz, 1H), 7.66 (s, 1H), 7.60 (dd, *J* = 8.3, 1.3 Hz, 1H), 7.50 (s, 1H), 6.50 (s, 1H), 6.02 (s, 2H), 5.10 (s, 2H)

### <Example 71> Preparation of 2,3-dimethoxy-5-tosyl-5,6-dihydro-11H-dibenzo[b,e]azepin-11-one(HN2337)

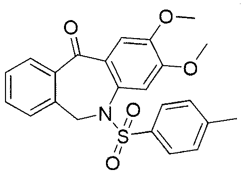

### Step 1: Preparation of methyl 4,5-dimethoxy-2-((4-methylphenyl)sulfonamido)benzoate

According to the preparation method of step 1 of General Preparation Method 7, methyl 2-Amino-4,5-dimethoxybenzoate(50 mg, 0.236 mmol), p-TsCl (54.2 mg, 0.284 mmol) and pyridine (0.06 mL, 0.710 mmol) were allowed to react, and then the reaction mixture was purified by column chromatography using 5% ethyl acetate and 50% methylene chloride solvent in n-hexane to obtain methyl 4,5-dimethoxy-2-((4-methylphenyl)sulfonamido)benzoate(85 mg, 98%).

### Step 2: Preparation of methyl 2-((N-benzyl-4-methylphenyl)sulfonamido)-4,5-dimethoxybenzoate

According to the preparation method of step 2 of General Preparation Method 7, 4,5-dimethoxy-2-((4-methylphenyl)sulfonamido)benzoate(45 mg, 0.123 mmol) prepared in step 1 and benzyl bromide(0.03 mL, 0.246 mmol) were allowed to react, and then the reaction mixture was purified by column chromatography using 25% ethyl acetate solvent in n-hexane to obtain methyl 2-((N-benzyl-4-methylphenyl)sulfonamido)-4,5-dimethoxybenzoate(54.5 mg, 97%).

### Step 3: Preparation of 2-((N-benzyl-4-methylphenyl)sulfonamido)-4,5-dimethoxybenzoic acid

According to the preparation method of step 3 of General Preparation Method 7, methyl 2-((N-benzyl-4-methylphenyl)sulfonamido)-4,5-dimethoxybenzoate(54.5 mg, 0.119 mmol) was reacted to obtain 2-((N-benzyl-4-methylphenyl)sulfonamido)-4,5-dimethoxybenzoic acid(52.8 mg, 100%).

### Step 4: Preparation of 2,3-dimethoxy-5-tosyl-5,6-dihydro-11H-dibenzo[b,e]azepin-11-one

According to the preparation method of step 4 of General Preparation Method 7, 2-((N-benzyl-4-methylphenyl)sulfonamido)-4,5-dimethoxybenzoic acid(27.7 mg, 0.063 mmol) prepared in step 3 was reacted, and then the reaction mixture was purified by column chromatography using 40% ethyl acetate solvent in n-hexane to obtain 2,3-dimethoxy-5-tosyl-5,6-dihydro-11H-dibenzo[b,e]azepin-11-one(5.8 mg, 22%).
¹H NMR (500 MHz, DMSO-d₆) δ 7.63 (s, 1H), 7.62 - 7.57 (m, 2H), 7.48 (d, *J =* 7.4 Hz, 1H), 7.37 (t, *J =* 7.5 Hz, 1H), 7.21 (s, 1H), 6.89 (d, *J =* 8.1 Hz, 2H), 6.69 (d, *J =* 8.2 Hz, 2H), 5.09 (s, 2H), 3.92 (s, 3H), 3.86 (s, 3H), 2.22 (s, 3H)

The compounds synthesized in Examples 1 to 71 are shown in Table 1 below:

**[Table 1]**

| | Structure | Name |
|---|---|---|
| Example 1 **(HN2203)** | 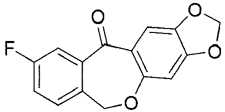 | 9-fluoro[2]benzoxepino[3,4-f]-1,3-benzodioxol-11(6H)-one |
| Example 2 **(HN2204)** | 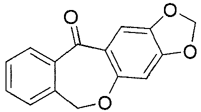 | [2]benzoxepino[3,4-f]-1,3-benzodioxol-11(6H)-one |
| Example 3 **(HN2208)** | 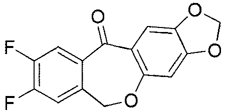 | 8,9-difluoro[2]benzoxepino[3,4-f]-1,3-benzodioxol-11(6H)-one |
| Example 4 **(HN2209)** | 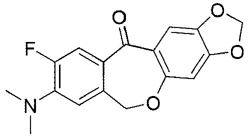 | 8-(dimethylamino)-9-fluoro[2]benzoxepino[3,4-f]-1,3-benzodioxol-11(6H)-one |
| Example 5 **(HN2210)** | 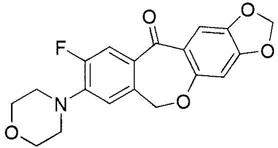 | 8-(morpholino)-9-fluoro[2]benzoxepino[3,4-f]-1,3-benzodioxol-11(6H)-one |
| Example 6 **(HN2211)** | 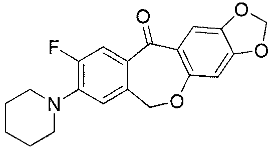 | 8-(piperidinyl)-9-fluoro[2]benzoxepino[3,4-f]-1,3-benzodioxol-11(6H)-one |
| Example 7 **(HN2212)** | 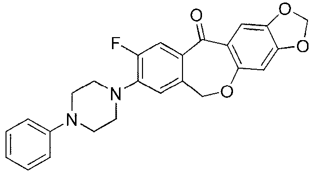 | 8-(phenylpiperazine-1-yl)-9-fluoro[2]benzoxepino[3,4-f]-1,3-benzodioxol-11(6H)-one |
| Example 8 **(HN2213)** | 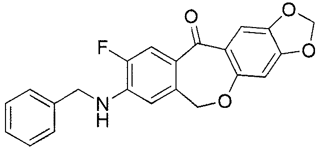 | 8-(benzylamino)-9-fluoro[2]benzoxepino[3,4-f]-1,3-benzodioxol-11(6H)-one |
| Example 9 **(HN2214)** | 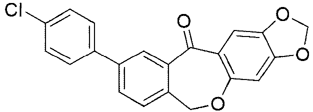 | 9-(4-chlorophenyl)[2]benzoxepino[3,4-f]-1,3-benzodioxol-11(6H)-one |
| Example 10 **(HN2215)** | 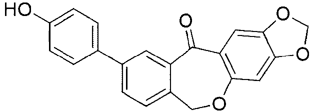 | 9-(4-hydroxyphenyl)[2]benzoxepino[3,4-f]-1,3-benzodioxol-11(6H)-one |
| Example 11 **(HN2216)** | 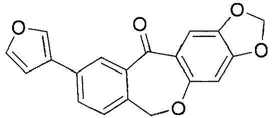 | 9-(3-furanyl)[2]benzoxepino[3,4-f]-1,3-benzodioxol-11(6H)-one |
| Example 12 **(HN2217)** | 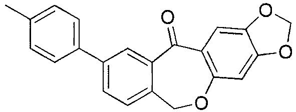 | 9-(p-tolyl)[2]benzoxepino[3,4-f]-1,3-benzodioxol-11(6H)-one |
| Example 13 **(HN2218)** | 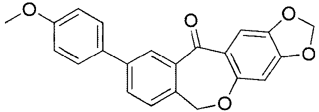 | 9-(4-methoxyphenyl)[2]benzoxepino[3,4-f]-1,3-benzodioxol-11(6H)-one |
| Example 14 **(HN2219)** | 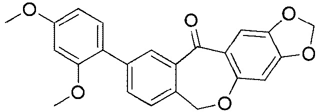 | 9-(2,4-dimethoxyphenyl)[2]benzoxepino[3,4-f]-1,3-benzodioxol-11(6H)-one |
| Example 15 **(HN2220)** | 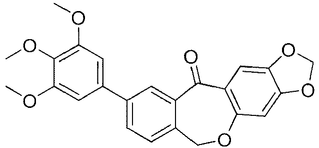 | 9-(3,4,5-trimethoxyphenyl)[2]benzoxepino[3,4-f]-1,3-benzodioxol-11(6H)-one |
| Example 16 **(HN2221)** | 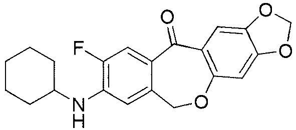 | 8-(cyclohexylamino)-9-fluoro[2]benzoxepino[3,4-f]-1,3-benzodioxol-11(6H)-one |
| Example 17 **(HN2222)** | 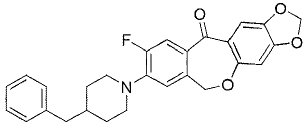 | 8-(4-benzylpiperidin-1-yl)-9-fluoro[2]benzoxepino[3,4-f]-1,3-benzodioxol-11(6H)-one |
| Example 18 **(HN2223)** | 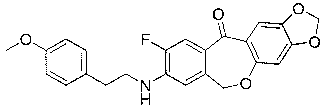 | 8-((4-methoxyphenethyl)amino)-9-fluoro[2]benzoxepino[3,4-f]-1,3-benzodioxol-11(6H)-one |
| Example 19 **(HN2224)** | 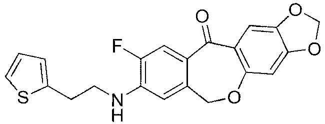 | 8-((2-(thiophen-2-yl)ethyl)amino)-9-fluoro[2]benzoxepino[3,4-f]-1,3-benzodioxol-11(6H)-one |
| Example 20 **(HN2225)** | 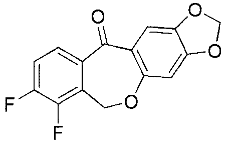 | 7, 8-difluoro[2]benzoxepino[3,4-f]-1,3-benzodioxol-11(6H)-one |
| Example 21 **(HN2226)** | 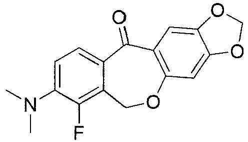 | 8-(dimethylamino)-7-fluoro[2]benzoxepino[3,4-f]-1,3-benzodioxol-11(6H)-one |
| Example 22 **(HN2227)** | 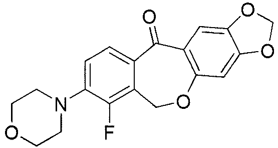 | 8-(morpholino)-7-fluoro[2]benzoxepino[3,4-f]-1,3-benzodioxol-11(6H)-one |
| Example 23 **(HN2228)** | 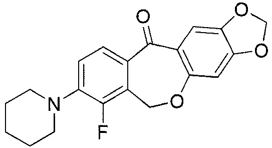 | 8-(piperidinyl)-7-fluoro[2]benzoxepino[3,4-f]-1,3-benzodioxol-11(6H)-one |
| Example 24 **(HN2229)** | 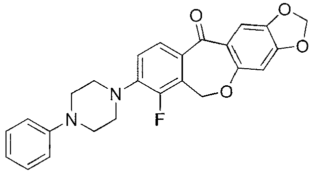 | 8-(phenylpiperazine-1-yl)-7-fluoro[2]benzoxepino[3,4-f]-1,3-benzodioxol-11(6H)-one |
| Example 25 **(HN2230)** | 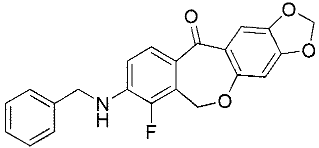 | 8-(benzylamino)-7-fluoro[2]benzoxepino[3,4-f]-1,3-benzodioxol-11(6H)-one |
| Example 26 **(HN2231)** | 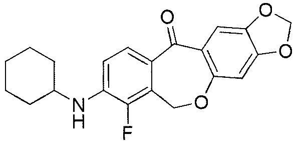 | 8-(cyclohexylamino)-7-fluoro[2]benzoxepino[3,4-f]-1,3-benzodioxol-11(6H)-one |
| Example 27 **(HN2232)** | 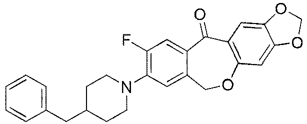 | 8-(4-benzylpiperidin-1-yl)-7-fluoro[2]benzoxepino[3,4-f]-1,3-benzodioxol-11(6H)-one |
| Example 28 **(HN2233)** | 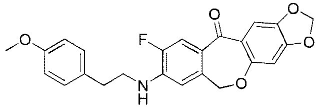 | 8-((4-methoxyphenethyl)amino)-7-fluoro[2]benzoxepino[3,4-f]-1,3-benzodioxol-11(6H)-one |
| Example 29 **(HN2234)** | 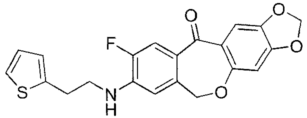 | 8-((2-(thiophen-2-yl)ethyl)amino)-7-fluoro[2]benzoxepino[3,4-f]-1,3-benzodioxol-11(6H)-one |
| Example 30 **(HN2235)** | 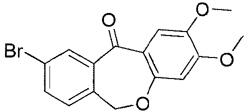 | 9-bromo-2,3-dimethoxydibenz[b,e]oxepin-11(6H)-one |
| Example 31 **(HN2236)** | 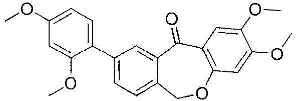 | 9-(2,4-dimethoxyphenyl)-2,3-dimethoxydibenzo[b,e]oxepin-11(6H)-one |
| Example 32 **(HN2237)** | 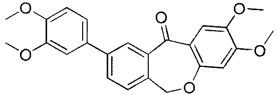 | 9-(3, 4-dimethoxyphenyl)-2,3-dimethoxydibenzo[b,e]oxepin-11(6H)-one |
| Example 33 **(HN2238)** | 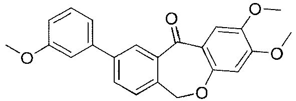 | 2,3-dimethoxy-9-(3-methoxyphenyl)dibenzo[b,e]oxepin-11(6H)-one |
| Example 34 **(HN2239)** | 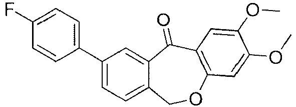 | 9-(4-fluorophenyl)-2,3-dimethoxydibenzo[b,e]oxepin-11(6H)-one |
| Example 35 **(HN2240)** | 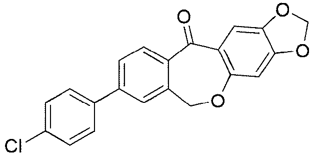 | 8-(4-chlorophenyl)[2]benzoxepino[3,4-f]-1,3-benzodioxol-11(6H)-one |
| Example 36 **(HN2241)** | 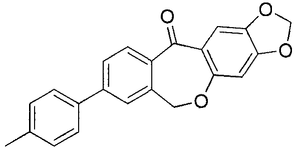 | 8-(p-tolyl)[2]benzoxepino[3,4-f]-1,3-benzodioxol-11(6H)-one |
| Example 37 **(HN2242)** | 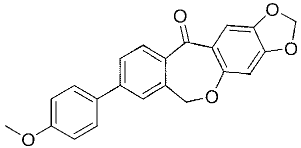 | 8-(4-methoxyphenyl)[2]benzoxepino[3,4-f]-1,3-benzodioxol-11(6H)-one |
| Example 38 **(HN2243)** | 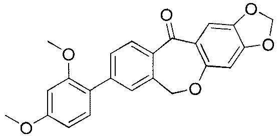 | 8-(2,4-dimethoxyphenyl)[2]benzoxepino[3,4-f]-1,3-benzodioxol-11(6H)-one |
| Example 39 **(HN2244)** | 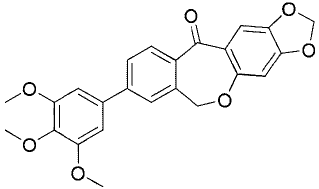 | 8-(3,4,5-trimethoxyphenyl)[2]benzoxepino[3,4-f]-1,3-benzodioxol-11(6H)-one |
| Example 40 **(HN2245)** | 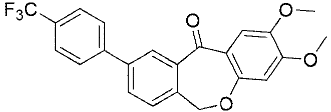 | 2,3-dimethoxy-9-(4-(trifluoromethyl)phenyl)dibenzo[b,e]oxepin-11(6H)-one |
| Example 41 **(HN2301)** | 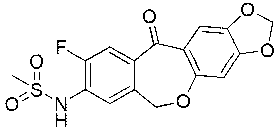 | N-(9-fluoro-11-oxo-6,11-dihydro-[1,3]dioxolo[4',5': 4,5]benzo[1,2-b]benzo[e]oxepin-8-yl)methanesulfonamide |
| Example 42 **(HN2302)** | 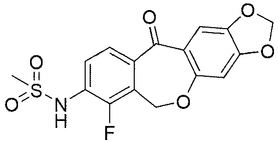 | N-(7-fluoro-11-oxo-6,11-dihydro-[1,3]dioxolo[4',5': 4,5]benzo[1,2-b]benzo[e]oxepin-8-yl)methanesulfonamide |
| Example 43 **(HN2303)** | 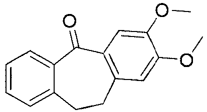 | 2,3-dimethoxy-10,11-dihydro-5H-dibenzo[a,d][7]annulen-5-one |
| Example 44 **(HN2304)** | 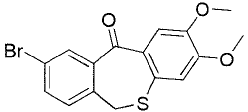 | 9-bromo-2,3-dimethoxydibenzo[b,e]thiepin-11(6H)-one |
| Example 45 **(HN2305)** | 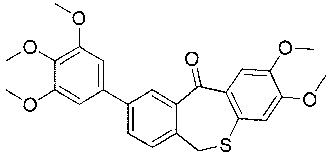 | 2,3-dimethoxy-9-(3,4,5-trimethoxyphenyl)dibenzo[b,e]thiepin-11(6H)-one |
| Example 46 **(HN2306)** | 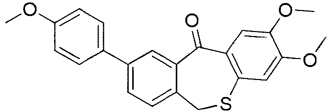 | 2,3-dimethoxy-9-(4-methoxyphenyl)dibenzo[b,e]thiepin-11(6H)-one |
| Example 47 **(HN2307)** | 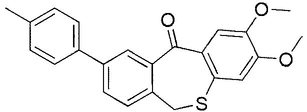 | 2,3-dimethoxy-9-(p-tolyl)dibenzo[b,e]thiepin-11(6H)-one |
| Example 48 **(HN2308)** | 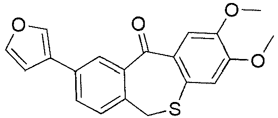 | 9-(3-furanyl)-2,3-dimethoxydibenzo[b,e]thiepin-11(6H)-one |
| Example 49 **(HN2309)** | 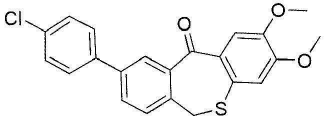 | 9-(4-chlorophenyl)-2,3-dimethoxydibenzo[b,e]thiepin-11(6H)-one |
| Example 50 (**HN2310**) | 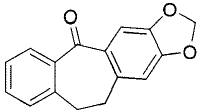 | 10,11-dihydro-5H-benzo[4',5']cyclohepta[1',2':4,5]benzo[1,2-d][1,3]dioxol-5-one |
| Example 51 (**HN2311**) | 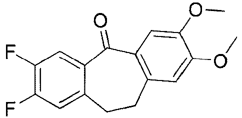 | 2,3-difluoro-7,8-dimethoxy-10,11-dihydro-5H-dibenzo[a,d][7]annulen-5-one |
| Example 52 **(HN2312)** | 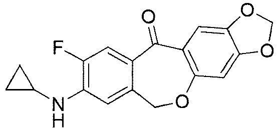 | 8-(cyclopropylamino)-9-fluoro-[1,3]dioxolo[4',5':4,5]benzo[1,2-b]benzo[e]oxepin-11(6H)-one |
| Example 53 **(HN2313)** | 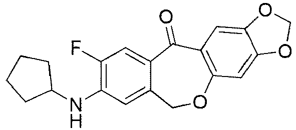 | 8-(cyclopentylamino)-9-fluoro-[1,3]dioxolo[4',5':4,5]benzo[1,2-b]benzo[e]oxepin-11(6H)-one |
| Example 54 **(HN2314)** | 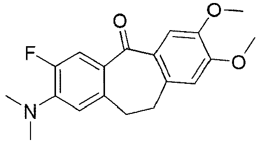 | 2-(dimethylamino)-3-fluoro-7,8-dimethoxy-10,11-dihydro-5H-dibenzo[a,d][7]annulen-5-one |
| Example 55 **(HN2315)** | 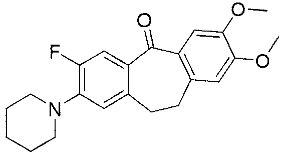 | 3-fluoro-7,8-dimethoxy-2-(piperidin-1-yl)-10,11-dihydro-5H-dibenzo[a,d][7]annulen-5-one |
| Example 56 (**HN2316**) | 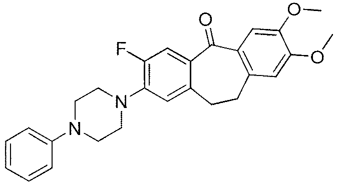 | 3-fluoro-7,8-dimethoxy-2-(4-phenylpiperazin-1-yl)-10,11-dihydro-5H-dibenzo[a,d][7]annulen-5-one |
| Example 57 **(HN2317)** | 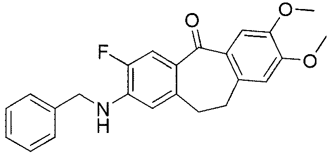 | 2-(benzylamino)-3-fluoro-7,8-dimethoxy-10,11-dihydro-5H-dibenzo[a,d][7]annulen-5-one |
| Example 58 **(HN2318)** | 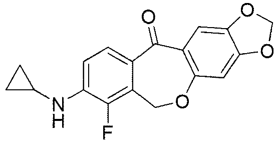 | 8-(cyclopropylamino)-7-fluoro-[1,3]dioxolo[4',5':4,5]benzo[1,2-b]benzo[e]oxepin-11(6H)-one |
| Example 59 **(HN2319)** | 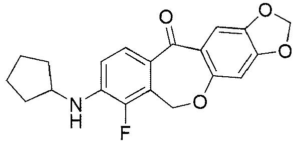 | 8-(cyclopentylamino)-7-fluoro-[1,3]dioxolo[4',5':4,5]benzo[1,2-b]benzo[e]oxepin-11(6H)-one |
| Example 60 **(HN2320)** | 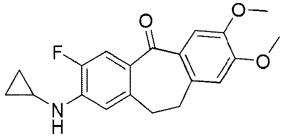 | 2-(cyclopropylamino)-3-fluoro-7,8-dimethoxy-10,11-dihydro-5H-dibenzo[a,d][7]annulen-5-one |
| Example 61 **(HN2321)** | 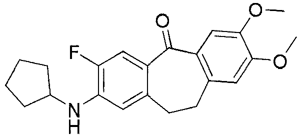 | 2-(cyclopentylamino)-3-fluoro-7,8-dimethoxy-10,11-dihydro-5H-dibenzo[a,d][7]annulen-5-one |
| Example 62 **(HN2322)** | 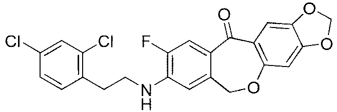 | 8-((2,4-dichlorophenethyl)amino)-9-fluoro-[1,3]dioxolo[4',5':4,5]benzo[1,2-b]benzo[e]oxepin-11(6H)-one |
| Example 63 **(HN2323)** | 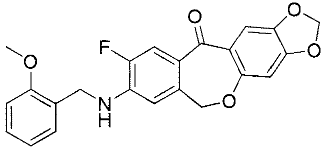 | 9-fluoro-8-((2-methoxybenzyl)amino)-[1,3]dioxolo[4',5':4,5]benzo[1,2-b]benzo[e]oxepin-11(6H)-one |
| Example 64 **(HN2324)** | 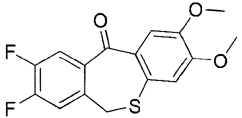 | 8,9-difluoro-2,3-dimethoxydibenzo[b,e]thiepin-11(6H)-one |
| Example 65 **(HN2325)** | 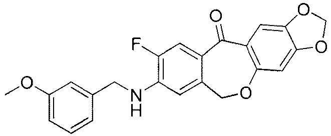 | 9-fluoro-8-((3-methoxybenzyl)amino)-[1,3]dioxolo[4',5':4,5]benzo[1,2-b]benzo[e]oxepin-11(6H)-one |
| Example 66 **(HN2326)** | 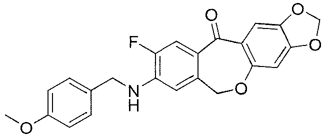 | 9-fluoro-8-((4-methoxybenzyl)amino)-[1,3]dioxolo[4',5':4,5]benzo[1,2-b]benzo[e]oxepin-11(6H)-one |
| Example 67 **(HN2327)** | 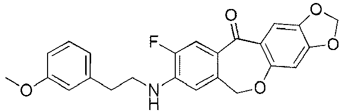 | 9-fluoro-8-((3-methoxyphenethyl)amino)-[1,3]dioxolo[4',5':4,5]benzo[1,2-b]benzo[e]oxepin-11(6H)-one |
| Example 68 **(HN2328)** | 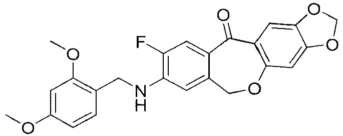 | 8-((2,4-dimethoxybenzyl)amino)-9-fluoro-[1,3]dioxolo[4',5':4,5]benzo[1,2-b]benzo[e]oxepin-11(6H)-one |
| Example 69 **(HN2329)** | 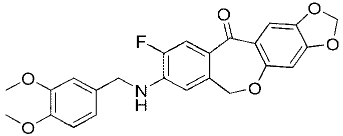 | 8-((3,4-dimethoxybenzyl)amino)-9-fluoro-[1,3]dioxolo[4',5':4,5]benzo[1,2-b]benzo[e]oxepin-11(6H)-one |
| Example 70 **(HN2336)** | 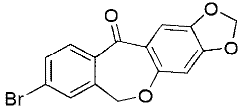 | 8-Bromo[2]benzoxepino[3,4-f]-1,3-benzodioxol-11(6H)-one |
| Example 71 **(HN2337)** | 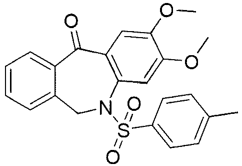 | 2,3-dimethoxy-5-tosyl-5,6-dihydro-11H-dibenzo[b,e]azepin-11-one |

### Experimental Example: Evaluation of in vitro efficacy of compounds

### Experimental Example 1. Evaluation of mTORC1 inhibitory activity and mTORC2 inhibitory activity in the mTOR signaling pathway of HeLa cells

In order to evaluate the mTORC1 inhibitory activity and mTORC2 inhibitory activity of the compounds synthesized in Examples 1 to 70, the following experiments were conducted in HeLa cells. As the experimental target compounds, 70 types of compounds: HN2203, HN2204, HN2205, HN2208, HN2209, HN2210, HN2211, HN2212, HN2213, HN2214, HN2215, HN2216, HN2217, HN2218, HN2220, HN2221, HN2222, HN2223, HN2224, HN2225, HN2226, HN2227, HN2228, HN2229, HN2230, HN2231, HN2232, HN2233, HN2234, HN2235, HN2236, HN2237, HN2238, HN2239, HN2240, HN2241, HN2242, HN2243, HN2244, HN2245, HN2301, HN2302, HN2303, HN2304, HN2305, HN2306, HN2307, HN2308, HN2309, HN2310, HN2311, HN2312, HN2313, HN2314, HN2315, HN2316, HN2317, HN2318, HN2319, HN2320, HN2321, HN2322, HN2323, HN2324, HN2325, HN2326, HN2327, HN2328, HN2329, and HN2336 listed in Table 1 were used.

Specifically, HeLa cells were cultured in a 5% CO₂ incubator at 37°C in DMEM (Dulbecco's Modified Eagles Media) medium supplemented with 10% FBS, penicillin (100 unit/ml), and streptomycin (100 µg/ml). Cultivation was started by seeding 4×10⁵ cells in a 100 mm culture dish, and subculture was carried out every 3 days.

Human cell line HeLa cells were seeded in a 60 mm culture dish at a density of 2 x 10⁵ cells/plate, and cultured for 24 hours. Then, HeLa cells were treated with 70 types of compounds at a concentration of 40 µM each for 9 hours. HeLa cells were washed twice with cold 1x PBS, and then lysed with 100 µl of RIPA buffer (50 mM Tris-HCl (pH7.4), 0.25% sodium deoxycholate, 50 mM NaF, 1% NP-40, 150 mM NaCl, 1 mM β-glycerophosphate, 1 mM PMSF, 1 mM NaVO4, 1 µg/ml leupeptin, 1 µg/ml pepstatin A, 10 nM calyculin A, 10 µg/ml aprotinin, 1 mM EDTA) to hemolyze the cell membrane. Then, the lysates were centrifuged at 12,000 rpm at 4°C for 10 minutes to collect the supernatant, which is cell lysate. Protein concentration in the cell lysate was determined using the BCA protein assay kit (Thermo Fisher Scientific), and equal amounts of protein were separated by SDS-PAGE.

Western blot analysis was performed by the following method. Proteins separated by SDS-PAGE were transferred to a PVDF membrane via electroblotting, and the PVDF membrane was blocked in TBS-T buffer (20 mM Tris, 137 mM NaCl, 0.1% Tween 20) containing 5% skim milk for 1 hour with gentle shaking. After washing the membrane three times for 5 minutes each with TBS-T buffer, it was incubated with primary antibodies diluted in TBS-T buffer containing 5% skim milk or 5% BSA overnight at 4°C. Subseqently, the membrane was washed three times for 5 minutes each with TBS-T buffer, and then incubated with secondary antibodies diluted in TBS-T buffer containing 5% skim milk at room temperature for 30 minutes. The membrane was washed five times for 5 minutes each with TBS-T buffer containing 5% skim milk, and then the protein bands bound by the secondary antibody were detected using a chemiluminescence method. The primary antibodies used were Akt (cell signaling, #4691), pAkt(S473) (Cell signaling, #4060), p70S6K (Cell signaling, #S6198), pp70S6K(T389) (cell signaling, #9205S), LC3-II (MBL, #PM036), and the secondary antibodies used were HRP conjugated anti-rabbit (Jackson immunoresearch laboratory, #711-035-152) and HRP conjugated anti-mouse IgG (Jackson immunoresearch laboratory, #515-035-072).

As an mTORC2 activity indicator in HeLa cells, the level of Akt Ser473 phosphorylation was selected as a marker, and the phosphor-Akt (Ser473) signal obtained from Western blot analysis was normalized to the pan-Akt signal, and the data were expressed as a percentage relative to the control group treated with DMSO only, which is a vehicle.

As an mTORC1 activity indicator in HeLa cells, the level of p70S6K Thr389 phosphorylation was selected as a marker, and the level of phosphor-p70S6K (Thr389) signal measured by Western blot analysis was normalized to a level of pan-p70S6K signal, and then expressed as a percentage of the control group treated with only DMSO, which is a vehicle.

LC3-II formation was selected as an indicator of autophagy in HeLa cells, and the LC3-II signal obtained from Western blot analysis was normalized to the pan-Akt signal and the data were expressed as a percentage relative to the control group treated with DMSO only, which is a vehicle.

The results of the above experiments are shown in Table 2 below.

**[Table 2]**

| **Compound** | **pSer473-AKT/AKT (% control)** | **pThr389-S6K/S6K (% control)** | **LC3-II (% control)** |
|---|---|---|---|
| DMSO | 100 | 100 | 100 |
| HN2203 | 28 | 179 | 119 |
| HN2209 | 54 | 191 | 124 |
| HN2210 | 43 | 107 | 108 |
| HN2211 | 118 | 77 | 122 |
| HN2213 | 51 | 114 | 67 |
| HN2214 | 67 | 99 | 154 |
| HN2216 | 64 | 121 | 99 |
| HN2217 | 38 | 224 | 149 |
| HN2221 | 77 | 93 | 119 |
| HN2222 | 69 | 95 | 89 |
| HN2225 | 66 | 100 | 100 |
| HN2231 | 100 | 49 | 122 |
| HN2232 | 48 | 136 | 64 |
| HN2233 | 74 | 107 | 121 |
| HN2301 | 54 | 81 | 96 |
| HN2302 | 73 | 122 | 108 |
| HN2304 | 44 | 204 | ND |
| HN2305 | 47 | 64 | ND |
| HN2306 | 64 | 257 | ND |
| HN2307 | 72 | 106 | ND |
| HN2308 | 44 | 95 | ND |
| HN2309 | 66 | 106 | ND |
| HN2313 | 80 | 54 | 162 |
| HN2315 | 28 | 223 | 620 |
| HN2316 | 20 | 38 | 256 |
| HN2317 | 107 | 69 | ND |
| HN2318 | 42 | 109 | 227 |
| HN2319 | 79 | 78 | 157 |
| HN2321 | 140 | 33 | 160 |
| HN2323 | 72 | 60 | 180 |
| HN2325 | 87 | 44 | 249 |
| HN2326 | 69 | 153 | 404 |
| HN2328 | 78 | 86 | 340 |
| HN2329 | 36 | 57 | 386 |
| - Compound concentration: 40µM, compound treatment time: 9 hours | | | |
| - ND: not determined | | | |

As shown in Table 2 as a result, after treating with 29 types of compounds: HN2203, HN2209, HN2210, HN2213, HN2214, HN2216, HN2217, HN2221, HN2222, HN2225, HN2232, HN2233, HN2301, HN2302, HN2304, HN2305, HN2306, HN2307, HN2308, HN2309, HN2313, HN2315, HN2316, HN2318, HN2319, HN2323, HN2326, HN2328 and HN2329 at a concentration of 40 µM for 9 hours, more than 20% reduction in Akt Ser473 phosphorylation, an indicator of mTORC2 activity, was observed compared to the control group, and after treating with 10 types of compounds: HN2211, HN2231, HN2305, HN2313, HN2316, HN2317, HN2319, HN2321, HN2325 and HN2329 at a concentration of 40 µM for 9 hours, more than 20% reduction in p70S6K Thr389 phosphorylation, an indicator of mTORC1 activity, was observed compared to the control group.

Specifically, HN2210 reduced AKT Ser473 phosphorylation by 57% with no effect on S6K Thr389 phosphorylation, and increased LC3-II by 22%. HN2213 reduced AKT Ser473 phosphorylation by 49%, but increased S6K Thr389 phosphorylation by 14%, and decreased LC3-II by 33%. HN2216 reduced AKT Ser473 phosphorylation by 36% but increased S6K Thr389 phosphorylation by 21%. HN2308 reduced AKT Ser473 phosphorylation by 56% with no effect on S6K Thr389 phosphorylation. HN2313 reduced AKT Ser473 phosphorylation by 20% and decreased S6K Thr389 phosphorylation by 46%, and increased LC3-II by 62%. HN2323 reduced AKT Ser473 phosphorylation by 28% and decreased S6K Thr389 phosphorylation by 40%, and increased LC3-II by 80%. In particular, 29 types of compounds: HN2203, HN2209, HN2210, HN2213, HN2214, HN2216, HN2217, HN2221, HN2222, HN2225, HN2232, HN2233, HN2301, HN2302, HN2304, HN2306, HN2307, HN2308, HN2309, HN2315, HN2318, HN2326 and HN2328 were confirmed to selectively inhibit mTORC2.

From the above results, it can be understood that the 35 types of compounds, as well as compounds from Examples 1 to 70 sharing the same core structure or having similar structure, can inhibit the activity of mTORC1 and/or mTORC2 in HeLa cells.

### Experimental Example 2. Evaluation of mTORC1 inhibitory activity and mTORC2 inhibitory activity in the mTOR signaling pathway in cerebral cells

The following experiments were conducted to evaluate time-dependent inhibition of mTORC2 and mTORC1 activities, as well as LC-II activation in primary cultured cerebral cells. As the experimental target compounds, 16 type of compounds: HN2209, HN2210, HN2213, HN2216, HN2304, HN2308, HN2309, HN2313, HN2316, HN2318, HN2319, HN2323, HN2325, HN2328, HN2329, and HN2337, which demonstrated excellent mTORC2 inhibitory activity or mTORC1 inhibitory activity and low toxicity in Experimental Example 1, were selected for evaluation.

Specifically, primary neural cells were cultured from the cerebral cortex of E16 embryos of SD mice with a gestational age 16-day (Orient Bio). The extracted brain was separated into the left and right hemispheres, the meninges were removed, and the brains were treated with trypsin in Ca²⁺/Mg²⁺-free HBSS solution. After centrifugation to remove the supernatant, the tissue was gently triturated in neurobasal media to dissociate the cells, and remaining tissue chunks that did not dissociate were filtered through a 40 µm nylon mesh. The cells were seeded at a density of 19,000 cells/cm² onto 35 mm culture dishes coated with 50 µg/ml poly-D-Lysine (Sigma, P0899) and 1 µg/ml laminin (Invitrogen, 23017-015), and then maintained in neurobasal complete media supplemented with B27 (Gibco, 17504-044) in a 5% CO₂ incubator.

The mTORC2 inhibitory activity of the compounds was found to be stronger in primary cultured cerebral cells compared to HeLa cells. Therefore, the evaluation of mTORC2 inhibitory activity in primary cultured cerebral cells was conducted using the same method as in Experimental Example 1, except that each compound was treated at concentrations of 500 nM or 100 nM for 24 hours. The time-dependent changes in Akt Ser473 phosphorylation, Akt Thr308 phosphorylation, and LC3-II levels were measured by Western blot analysis.

The results of the above experiments are shown in FIGS. 1a to 1f and Table 3 below.

**[Table 3]**

| Evaluation of inhibitory activity on mTOR signaling in primary cultured cerebral neurons | | | | |
|---|---|---|---|---|
| **Compound** | **pSer473-AKT/AKT (% control)** | **pThr389-S6K/S6K (% control)** | **LC3-II (% control)** | **Conc. (nM)** |
| DMSO | 100 | 100 | 100 | - |
| HN2210 | 33 | 23 | 348 | 500 |
| HN2213 | 38 | 137 | 336 | 500 |
| HN2304 | 82 | 504 | 82 | 500 |
| HN2308 | 46 | 104 | 119 | 100 |
| HN2313 | 34 | 141 | 86 | 100 |
| HN2323 | 48 | 128 | 135 | 100 |
| - Compound treatment time for cerebral neurons: 24 hours | | | | |

As shown in FIGS. 1a to 1f and Table 3 as a result, 6 types of compounds: HN2210, HN2213, HN2304, HN2308, HN2313 and HN2323 were confirmed to reduce Akt Ser473 phosphorylation, an indicator mTORC2 activity, and HN2210 also reduced p70S6K Thr389 phosphorylation, an indicator of mTORC1 activity.

Specifically, HN2210 reduced AKT Ser473 phosphorylation by approximately 50% from 30 minutes to 6 hours, reaching a 67% reduction at 24 hours. S6K Thr389 phosphorylation increased by 49% at 3 hours but decreased by 77% at 24 hours. LC3-II were markedly increased. HN2213 reduced AKT Ser473 phosphorylation by 62% at 24 hours, but increased S6K Thr389 phosphorylation by 37% in a time-dependent manner. LC3-II was significantly increased. HN2304 significantly increased AKT Ser473 phosphorylation from 1 to 12 hours, then decreased by 17% at 24 hours. Conversely, S6K Thr389 phosphorylation markedly increased from 6 to 24 hours. LC3-II was not affected. HN2308 increased AKT Ser473 phosphorylation significantly from 1 to 12 hours, followed by a 54% decrease at 24 hours, with no effect on S6K Thr389 phosphorylation and LC3-II. HN2313 increased AKT Ser473 phosphorylation significantly from 1 hour to 12 hours and then decreased it by 66% at 24 hours. On the other hand, S6K Thr389 phosphorylation increased 2~3 fold from 1 to 6 hours. LC3-II was not affected. HN2323 increased AKT Ser473 phosphorylation significantly from 1 to 12 hours and then decreased it by 52% at 24 hours, with no effect on S6K Thr389 phosphorylation and LC3-II.

From the above results, it was confirmed that all compounds of HN2213, HN2304, HN2308, HN2313 and HN2323 significantly decreased Akt Ser473 phosphorylation, an indicator of mTORC2 activity, while they did not decrease or only slightly decrease p70S6K Thr389 phosphorylation, an indicator of mTORC1 activity. Therefore, it was confirmed that compounds of HN2213, HN2304, HN2308, HN2313 and HN2323 can be used as selective mTORC2 inhibitors.

### Experimental Example 3. Evaluation of the inhibitory efficacy on mGluR1/5-dependent mTORC2 activation in dendritic spines of primary cultured hippocampal neurons

### Experimental Example 3-1. Evaluation of the inhibitory efficacy on mGluR1/5-dependent mTORC2 activation in hippocampal neurons

When mGluR1/5 is activated by treating primary cultured and differentiated hippocampal neurons (DIV21) with DHPG, mTORC2 is activated in the dendritic spines of neurons. In order to evaluate the inhibitory effect of the compounds on mTORC2 activated in a mGluR1/5-dependent manner in the dendritic spines of hippocampal neurons, the following experiments were conducted. The 14 compounds with excellent mTORC2 inhibitory activity in Experimental Example 1 were selected and used as the experimental target compounds.

Specifically, primary cultured and differentiated hippocampal neurons (DIV21) were pretreated with HN2209, HN2210, HN2213, HN2216, HN2304, HN2305, HN2308, HN2309, HN2313, HN2319, HN2323, HN2325, HN2328, or HN2329 at a concentration of 500 nM for 2 hours, followed by treatment with DHPG for 10 minutes to induce mTORC2 activation. After the neurons were stained using immunostaining with the p-AKT (S473) antibody and PSD95 antibody, the stained puncta were quantified, and the results are shown in FIGS. 2a to 2l and Table 4.

**[Table 4]**

| Evaluation of inhibitory efficacy on mGluR1/5-dependent mTORC2 activation in dendritic spines of hippocampal neurons | |
|---|---|
| **Compound** | **pAKT(S473)+PSD95+ puncta per 20µm (% inhibition of DHPG induction)** |
| HN2209 | 98 |
| HN2210 | 117 |
| HN2213 | 113 |
| HN2216 | 102 |
| HN2304 | 105 |
| HN2305 | 96 |
| HN2308 | 101 |
| HN2309 | 124 |
| HN2313 | 119 |
| HN2323 | 82 |
| HN2325 | 113 |
| HN2329 | 68 |
| - the concentration of the compound: 500nM | |
| - % inhibition of DHPG induction = [(DHPG-compound)/(DHPG-CTL)]*100 | |

As shown in FIGS. 2a to 2l and Table 4 as a result, twelve types of compounds: HN2209, HN2210, HN2213, HN2216, HN2304, HN2305, HN2308, HN2309, HN2313, HN2323, HN2325 and HN2329 were found to reduce the increase in p-AKT(S473)+PSD95+ puncta induced by DHPG by 68~124%.

### Experimental Example 3-2. Evaluation of the IC₅₀ of compounds that inhibit mGluR1/5-dependent mTORC2 activation in dendritic spines of hippocampal neurons

In order to evaluate the IC₅₀ of the five types of compounds: HN2210, HN2213, HN2221, HN2308 and HN2313 inhibiting mGluR1/5-dependent mTORC2 activation in dendritic spines of primary cultured hippocampal neurons, the following experiments were conducted.

Specifically, primary cultured and differentiated hippocampal neurons (DIV21) were pretreated with HN2210, HN2213, HN2221, HN2308, and HN2313 at concentrations of 25, 50, and 100 nM for 2 hours, followed by treatment with with DHPG for 10 minutes to induce mTORC2 activation. After the neurons were stained using immunostaining with the p-AKT (S473) antibody and PSD95 antibody, the stained puncta were quantified, and the results are shown in FIGS. 3a to 3e and Table 5 below.

**[Table 5]**

| Evaluation of the IC₅₀ of compounds that inhibit mGluR1/5-dependent mTORC2 activation in dendritic spines of hippocampal neurons | | |
|---|---|---|
| **Compound** | **Concentration (nM)** | **pAKT(S473)+PSD95+ puncta per 20µm (% inhibition of DHPG induction)** |
| HN2210 | 25 | 78 |
| | 50 | 82 |
| | 100 | 87 |
| HN2213 | 25 | 93 |
| | 50 | 89 |
| | 100 | 85 |
| HN2221 | 25 | 75 |
| | 50 | 71 |
| | 100 | 83 |
| HN2308 | 25 | 97 |
| | 50 | 68 |
| | 100 | 78 |
| HN2313 | 25 | 66 |
| | 50 | 81 |
| | 100 | 86 |

As shown in FIGS. 3a to 3e and Table 5 as a result, HN2210, HN2213, HN2221, HN2308 and HN2313 were found to inhibit mTORC2 activity by 50% or more at a concentration of 25 nM, and therefore, the IC₅₀ of HN2210, HN2213, HN2221, HN2308 and HN2313 is 50 nM or less.

### Experimental Example 4. Evaluation of the inhibitory activity on the binding between mTOR protein and mLST8 protein

mLST8 binds to the mTOR C-terminal domain (mTOR(CD), a.a. 2179-2549). After binding to mTOR, mLST8 interacts with mSin1 and Rictor to complete the formation of the mTORC2 complex, serving as a scaffold. In order to evaluate the inhibitory activity of the compounds synthesized in Examples 1 to 70 on the binding between mLST8 protein and mTOR(CD) protein, the following mTOR(CD)-mLST8 binding assay was performed. Four types of compounds: HN2210, HN2213, HN2221 and HN2313 were used as the experimental target compounds.

Specifically, mTOR(CD) protein was overexpressed in *E. coli,* labeled with biotin, and then partially purified. mLST8 protein was overexpressed in insect Sf9 cells to obtain mLST8 extract. mTOR(CD)-biotin and mLST8 extract were incubated at 4°C to induce binding between mTOR(CD)-biotin and mLST8 proteins, and then the mTOR(CD)-biotin and mLST8 complex were co-precipitated using avidin-agarose beads. The relative amounts of precipitated mTOR(CD)-biotin and mLST8 proteins were measured by Western blot analysis, as shown in FIG. 4. Four types of compounds were added during the incubation process of the mTOR(CD)-biotin and mLST8 extract.

As shown in FIG. 4 as a result, HN2210 and HN2213 began to inhibit the binding between mTOR(CD) and mLST8 proteins by 24% and 19% at a concentration of 4 µM, respectively, and by 36% and 30% at a concentration of 40 µM, respectively. HN2313 inhibited the binding between mTOR(CD) and mLST8 proteins by 34% at a concentration of 40 µM. On the other hand, HN2221 did not significantly inhibit the binding between mTOR(CD) and mLST8 proteins at concentrations of 0.04 to 40 µM.

From the above experimental results, it can be seen that 3 types of compounds: HN2210, HN2213 and HN2313, as well as the compounds of Examples 1 to 70 that share the same core structure or a similar core structure, are capable of directly inhibiting the binding between mTOR and mLST8, thereby preventing the formation of the mTORC2 complex.

### Evaluation of the in vivo efficacy of compounds

### Experimental example 4. Evaluation of the in vivo efficacy of the compounds of Chemical Formula 1 in 5xFAD Alzheimer's disease model mice

In order to evaluate the efficacy of HN2210, HN2213, and HN2313 on Alzheimer's disease-related symptoms, specifically regarding 1) memory and cognitive impairment, 2) amyloid plaques, 3) neurofibrillary tangles (NFTs), and 4) brain inflammation, an in vivo efficacy assessment of HN2210, HN2213 and HN2313 was conducted using 5xFAD Alzheimer's disease model mice. The 5xFAD mice were purchased from Jackson Lab, USA (Cat #034840-JAX) and crossed once with C57bl6 mice to produce F1 heterozygous offspring (Het). The compound was administered via intraperitoneal injection to 6-month-old 5xFAD Het mice, followed by behavioral testing. The normal control mice, which were littermates of the 5xFAD Het mice, were aged to 6 months and received DMSO injection at the same volume.

### Experimental Example 4-1. Confirmation of the improvement effect on short-term memory ability through the Y-type maze test

In order to assess the short-term memory improvement effect of HN2210, HN2213, and HN2313 compounds in an Alzheimer's disease model, the Y-type maze test was conducted according to the following method using 5xFAD-DMSO mice without compound treatment, 5xFAD mice treated with donepezil, rapamycin, HN2210, HN2213, or HN2313 compounds, as well as normal mice used as controls. Donepezil was administered at 1 mg/kg, and rapamycin, HN2210, HN2213 and HN2313 at 5 mg/kg, once daily via intraperitoneal for 2 weeks.

Specifically, the Y-maze test was performed using a Y-shaped structure maze made of black acrylic with each arm at a 120° angle, and light and a camera were installed on the ceiling, and we measured the number of entries for white mice to freely enter each arm while the surroundings were covered with a curtain. This procedure was used to analyze short-term memory. In order to adapt mice to the environment, they were acclimated to the experimental space for 30 minutes. The mice were placed at one end of the maze and allowed to move freely for 12 minutes. The test was conducted with observation periods divided into 8, 10, and 12 minutes. When all four paws of a mice entered an arm, it was considered as fully entered, and the number of entries into each arm without repetition was recorded. The number of entries into each of the three different arms was divided by the total number of entries into all arms minus 2, and expressed as a percentage. All data were statistically analyzed using ANOVA test, and the results are shown in FIG. 5.

As shown in FIG. 5 as a result, in the Y-type maze test, the 5xFAD-DMSO mice showed a reduction in the short-term memory indicator, % alternation, to approximately 75% of the level observed in the normal control mice. In the 5xFAD mice in which HN2210, HN2213 or HN2313 were administered at a dose of 5 mg/kg for 14 days, the % alternation increased by 1.25-fold, 1.3-fold and 1.2-fold compared to 5xFAD-DMSO mice, reaching 93%, 97%, and 90%, respectively, of the normal control level. In contrast, treatment with donepezil (1 mg/kg) or rapamycin (5 mg/kg) for 2 weeks did not result in significant increase. The above experimental results indicate that short-term memory was nearly restored to the normal control level in the 5xFAD mice administered with HN2210, HN2213 or HN2313.

### Experimental Example 4-2. Confirmation of the improvement effect on spatial perceptive ability though the Morris water maze test

In order to assess the spatial perceptive ability improvement effect of HN2210, HN2213, and HN2313 compounds in an Alzheimer's disease model, the Morris water maze (MWM) test was conducted according to the following method using 5xFAD-DMSO mice without compound treatment, 5xFAD mice treated with donepezil, rapamycin, HN2210, HN2213, or HN2313 compounds, as well as normal mice used as controls.

Specifically, a 50 cm deep water tank filled with water (25.0 ± 1.0°C) was divided into four equal quadrants, and a platform (12 cm diameter) was placed 3 mm below the water surface in the center of one quadrant. The training test consisted of a 1-minute free swimming practice without the platform foothold on the first day and a four days of training, during which the animal used environmental cues to locate the hidden platform foothold, with four trials per day. The following day, the platform was removed, and the time the experimental animal spent in the target quadrant was measured (probe trial). Videos were recorded and analyzed using the Ethovision video tracking system (Noldus Information Technology, Wageningen, Netherlands). The data included the time spent swimming in the target quadrant and the number of crossings over the virtual platform's previous position. The measured experimental results are shown in FIGS. 6a and 6b.

As shown in FIGS. 6a and 6b as a result, in the Morris water maze test, the number of target crossings by 5xFAD-DMSO mice decreased to approximately 15% of the level observed in normal control mice, and in the 5xFAD mice in which HN2210, HN2213, or HN2313 was administered at a dose of 5 mg/kg for 14 days, the number of target crossings increased by 3.96-fold, 4-fold, and 5.5-fold compared to 5xFAD-DMSO mice, reaching approximately 59%, 60% and 82% of the normal control levels, respectively. Additionally, the time spent in the target quadrant by 5xFAD-DMSO mice was reduced to about 55% of that in normal controls. In the 5xFAD mice in which HN2210, HN2213, or HN2313 was administered at a dose of 5 mg/kg for 14 days, this time increased by 1.74-fold, 1.91-fold, and 1.53-fold compared to 5xFAD-DMSO mice, reaching approximately 96%, 105% and 84% of the normal control levels, respectively. In contrast, treatment with donepezil (1 mg/kg) or rapamycin (5 mg/kg) for 2 weeks did not result in significant increases in either in the number of target crossings or the time spent in the target quadrant. The above experimental results indicate that spatial perceptive ability was nearly restored to the normal control level in 5xFAD mice administered with HN2210, HN2213, or HN2313.

### Experimental Example 4-3. Confirmation of the improvement effect on long-term memory ability though the passive avoidance test

In order to assess the long-term memory ability improvement effect of HN2210, HN2213, and HN2313 compounds in an Alzheimer's disease model, the passive avoidance test was conducted according to the following method using 5xFAD-DMSO mice without compound treatment, 5xFAD mice treated with donepezil, rapamycin, HN2210, HN2213, or HN2313 compounds, as well as normal mice used as controls.

Specifically, to mice trained to avoid bright light three times daily, an electric foot shock (1 mA, 300 g) was applied for 3 seconds in a dark room at the same time on the next day. After 24 hours, the mice were placed again in the same room, and the avoidance response by light, that is, the time taken to move from the bright room to the dark room was measured. At 24 hours after habituation training, the mice were placed back into the bright room, and the latency time for entering the dark room was recorded over a period of 720 seconds. The measured test results are shown in FIG. 7.

As shown in FIG. 7 as a result, in the passive avoidance test, the latency time of 5xFAD-DMSO mice decreased to approximately 16% of that observed in normal control mice, and in the 5xFAD mice in which HN2210, HN2213 or HN2313 was administered at a dose of 5 mg/kg for 14 days, the latency time increased by 5.16-fold, 4.52-fold, and 5.86-fold compared to 5xFAD-DMSO mice, reaching approximately 84%, 74%, and 96% of the normal control levels, respectively. On the other hand, treatment with donepezil (1 mg/kg) or rapamycin (5 mg/kg) for 2 weeks resulted in reaching only about 38% and 36% of the normal control levels, respectively. The above experimental results indicate that long-term memory was nearly restored to the normal control level in 5xFAD mice administered with HN2210, HN2213, or HN2313.

From the results of the Experimental Examples 4-1, 4-2 and 4-3, it was confirmed that memory and cognitive functions, which were impaired in 5xFAD mice compared to the normal group, were restored to the level of the normal group by following HN2210, HN2213, or HN2313 administration.

### Experimental Example 4-4. Confirmation of dose-dependent improvement in memory and cognitive functions

To confirm the dose-dependent improvement in memory and cognitive functions of HN2210 in an Alzheimer's disease model, the Y-maze test, Morris water maze test, and passive avoidance test were conducted according to the following method using 5xFAD-DMSO mice without compound treatment, 5xFAD mice treated with HN2210 at a dose of 0.2 mg/kg, 1 mg/kg, and 5 mg/kg, as well as normal mice used as controls.

As shown in FIG. 8a as a result, in the Y-maze test, the % alternation, a short-term memory indicator, of 5xFAD-DMSO mice decreased to approximately 70% of the level observed in normal control mice, and in the 5xFAD mice in which HN2210 was administered at doses of 0.2 mg/kg, 1 mg/kg, and 5 mg/kg for 14 days, the % alternation increased by 1.21-fold, 1.17-fold, and 1.32-fold compared to 5xFAD-DMSO mice, reaching approximately 85%, 82%, and 93% of the normal control levels, respectively.

As shown in FIG. 8b, in a Morris water maze test, the number of the target crossings by 5xFAD-DMSO mice decreased to approximately 15% of that in normal control mice, and in 5xFAD mice in which HN2210 was administered at doses of 0.2 mg/kg, 1 mg/kg, and 5 mg/kg for 14 days, the number of the target crossings increased by 2.02-fold, 4.27-fold, and 5.84-fold compared to 5xFAD-DMSO mice, reaching approximately 30%, 63%, and 86% of the normal control levels, respectively. Additionally, as shown in FIG. 8c, the time spent swimming in the target quadrant by 5xFAD-DMSO mice was decreased to about 41% of that in the normal controls, and in 5xFAD mice in which HN2210 was administered at doses of 0.2 mg/kg, 1 mg/kg, and 5 mg/kg for 14 days, the time spent swimming in the target quadrant increased by 2.14-fold, 2.07-fold, and 2.34-fold compared to 5xFAD-DMSO mice, reaching approximately 87%, 85%, and 96% of the normal control levels, respectively.

In addition, as shown in FIG. 8d, in the passive avoidance test, the latency time in 5xFAD-DMSO mice decreased to approximately 19% of that in normal control, and in 5xFAD mice in which HN2210 was administered at doses of 0.2 mg/kg, 1 mg/kg and 5 mg/kg for 14 days, the latency times increased by 2.97-fold, 4.39-fold and 5.16-fold compared to 5xFAD-DMSO mice, reaching approximately 56%, 83%, and 97% of the normal control levels.

From the results of the Experimental Example 4-4, it was confirmed that the memory and cognitive functions, which were impaired in 5xFAD mice compared to the normal group, were dose-dependently improved to the level of the normal controls following administration of HN2210.

### Experimental Example 4-5. Confirmation of amyloid plaque removal effect in the brain of 5xFAD mouse

In order to assess the amyloid plaque removal effect of HN2210, HN2213, and HN2313 compounds in the brains of Alzheimer's disease model mice, the following test was conducted using 5xFAD-DMSO mice without compound treatment, 5xFAD mice treated with HN2210, HN2213, or HN2313 compounds, and normal mice as controls.

Specifically, brain slices sectioned in the coronal plane were selected for every fifth slice from the anterior to the posterior parts of the brain and stained with the 6E10 antibody or thioflavin-S. The 6E10 antibody stains both Aβ and amyloid plaques, while thioflavin-S specifically stains amyloid plaques. DNA was co-stained with DAPI. The dentate gyrus (DG) region of the hippocampus, where amyloid plaques tend to accumulate heavily, was examined using a confocal scanning microscope, and the images are shown in FIGS. 9a to 9c.

As shown in FIGS. 9a and 9b as a result, the 6E10 signals in the dentate gyrus of 5xFAD mice treated with HN2210, HN2213, or HN2313 at a dose of 5 mg/kg for 14 days were reduced by 53%, 29%, and 38%, respectively, compared to the 5xFAD-DMSO group.

Additionally, as shown in FIGS. 9a and 9c, the thioflavin-S signals in the dentate gyrus of 5xFAD mice treated with HN2210, HN2213, or HN2313 at a dose of 5 mg/kg for 14 days were reduced by 38%, 28%, and 35%, respectively, compared to the 5xFAD-DMSO group.

### Experimental Example 4-6. Confirmation of tau aggregate removal effect in the brain of 5xFAD mice

In order to assess the neurofibrillary tangle removal effect of HN2210, HN2213, and HN2313 compounds in the brains of Alzheimer's disease model mice, the following test was conducted using 5xFAD-DMSO mice without compound treatment, 5xFAD mice treated with HN2210, HN2213, or HN2313 compounds, and normal mice as controls.

Specifically, AT8 antibody stains hyperphosphorylated Tau protein that constitutes tau aggregates. Therefore, tau aggregates formed in the hippocampus of 5xFAD mouse can be measured using immunostaining with the AT8 antibody. Brain slices were triple-stained with the AT8 antibody, MAP2 which is a neuronal marker, and DAPI to label nuclei, and the dentate gyrus region of the hippocampus was observed under a confocal scanning microscope. The results are shown in FIGS. 10a and 10b.

As shown in FIGS. 10a and 10b as a result, the AT8 antibody signals in the dentate gyrus of 5xFAD mice treated with HN2210, HN2213, or HN2313 at a dose of 5 mg/kg for 14 days were reduced by 61%, 59%, and 49%, respectively, compared to the 5xFAD-DMSO group.

### Experimental Example 4-7. Confirmation of anti-inflammatory effect in the brain of 5xFAD mice

In order to assess the anti-inflammatory effect of HN2210, HN2213, and HN2313 compounds in the brains of Alzheimer's disease model mice, the following test was conducted using 5xFAD-DMSO mice without compound treatment, 5xFAD mice treated with HN2210, HN2213, or HN2313 compounds, and normal mice as controls. Specifically, brain inflammation is mediated by the activation of microglia, which can be stained using the IBA-1 antibody. Therefore, inflammation in the hippocampus of 5xFAD mouse can be assessed using immunostaining with the IBA-1 antibody. Brain slices were double-stained with the IBA-1 antibody and DAPI to label nuclei, and the dentate gyrus region of the hippocampus was observed under a confocal scanning microscope. The results are shown in FIGS. 11a and 11b.

As shown in FIGS. 11a and 11b as a result, the IBA-1 antibody signals in the dentate gyrus of 5xFAD mice treated with HN2210, HN2213, or HN2313 at a dose of 5 mg/kg for 14 days were reduced by 50%, 24%, and 50%, respectively, compared to the 5xFAD-DMSO group.

From the results of the Experimental Examples 4-5, 4-6, and 4-7, it was confirmed that HN2210, HN2213 and HN2313 reduce amyloid plaques and neurofibrillary tangles, as well as suppress brain inflammation when they were administered to the 5xFAD Alzheimer's disease model mice.

From the above description, those skilled in the art will understand that the present invention can be implemented in other specific forms without changing its technical idea or essential features. As such, the described embodiments are to be considered in all respects only as illustrative and not restrictive. The scope of the present invention should be interpreted as including all modifications or variations derived from the meaning and scope of the patent claims described below rather than the aforementioned detailed description and their equivalent concepts.

## Claims

1. A compound represented by the following Chemical Formula 1, or a pharmaceutically acceptable salt thereof. 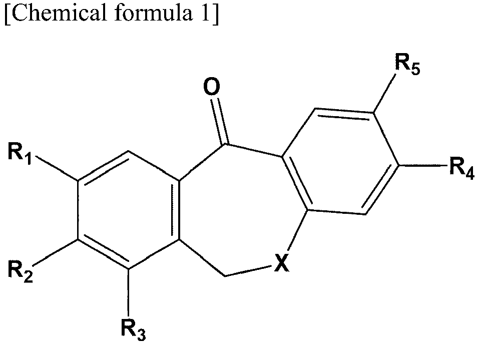
wherein in Chemical Formula 1,
the "X" is O, S, or CH₂,
the "R₁" is hydrogen, halo (F, Br, Cl, or I), a substituted or unsubstituted C₆₋₁₀ aryl, or a substituted or unsubstituted 4 to 12-membered heteroaryl containing one or more heteroatoms selected from the group consisting of N, O, and S,
the "R₂" is hydrogen, halo (F, Br, Cl, or I), a substituted or unsubstituted C₆₋₁₀ aryl, a substituted or unsubstituted C₁₋₆ amino, a substituted or unsubstituted 4 to 12-membered heterocycloalkyl, or an unsubstituted benzylamino,
the "R₃" is hydrogen or halo (F, Br, Cl, or I),
the "R₄" and "R₅" are each independently an unsubstituted C₁₋₆ alkoxy, or "R₄" and "R₅" together with two oxygen atoms to which they are attached may form -O(CH)ₙO-(wherein n is 1 or 2),
the substituted aryl or the substituted heteroaryl may be substituted with one or more substituents selected from the group consisting of hydroxy(OH), halo(F, Br, Cl, or I), a C₁₋₁₀ linear or branched alkyl which is substituted or unsubstituted with 1 to 3 halo, and an unsubstituted C₁₋₅ linear or branched alkoxy,
the substituted amino may be substituted with one or more substituents selected from the group consisting of a C₁₋₅ linear or branched alkyl, an unsubstituted C₃₋₈ cycloalkyl, a C₁₋₅ linear alkyl which is substituted with a phenyl group substituted with a C₁₋₅ linear or branched alkoxy, a C₁₋₅ linear alkyl which is substituted with an unsubstituted thiophenyl group, a C₃₋₈ cycloalkyl and a sulfonyl which is substituted with a C₁₋₅ linear alkyl,
the substituted heterocycloalkyl may be substituted with an unsubstituted C₆₋₁₀ aryl, or an unsubstituted C₆₋₁₀ aryl C₁₋₆ alkyl, and
the compound of Chemical Formula 1 does not include the following compounds:
a) 2,3-dimethoxydibenz[b,e]oxepin-11(6H)-one; and
b) 9-bromo[2]benzoxepino[3,4-f]-1,3-benzodioxol-11(6H)-one.

2. The compound or a pharmaceutically acceptable salt thereof according to claim 1, wherein:
the "R₁" is hydrogen, fluoro, bromo, a substituted or unsubstituted C₆₋₁₀ aryl, or an unsubstituted C₄₋₆ heteroaryl containing O as a hetero atom,
the substituted aryl of "R₁" is substituted with one substituent selected from the group consisting of hydroxy, fluoro, chloro, trifluoro, methyl, and 1 to 3 methoxy groups,
the "R₂" is hydrogen, fluoro, a substituted C₆₋₁₀ aryl, a substituted or unsubstituted C₁₋₆ amino, a substituted or unsubstituted 6-membered heterocycloalkyl, or an unsubstituted benzylamino,
the substituted aryl of "R₂" is substituted with one substituent selected from the group consisting of chloro, methyl, and 1 to 3 methoxy groups,
the substituted alkylamino is substituted with one substituent selected from the group consisting of two methyl, cyclohexyl, phenethyl, thiophenylethyl, and methylsulfonyl, and
the heterocycloalkyl is substituted with an unsubstituted aryl substituent.

3. The compound or a pharmaceutically acceptable salt thereof according to claim 1 or 2, wherein the "R₄" and "R₅" are each independently methoxy, or "R₄" and "R₅" together with two oxygen atoms to which they are attached form -O(CH)₂O-.

4. The compound or a pharmaceutically acceptable salt thereof according to claim 1, wherein the "R₁" is one selected from the group consisting of hydrogen, fluoro, bromo, 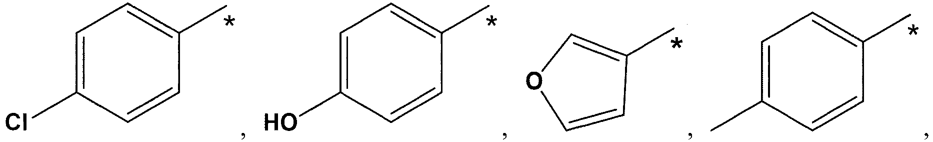 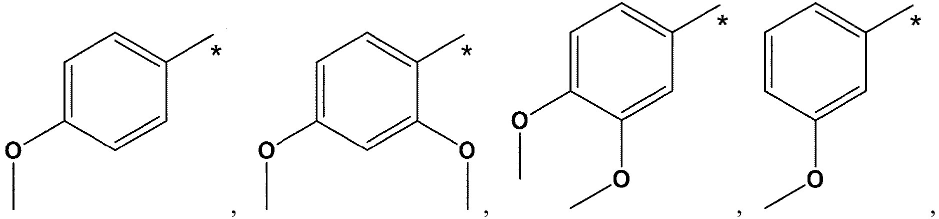 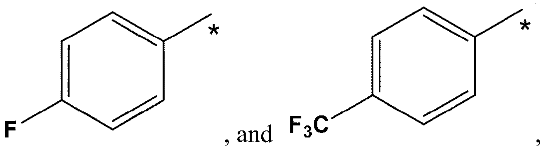 and
the "R₂" is one selected from the group consisting of hydrogen(H), fluoro(F), 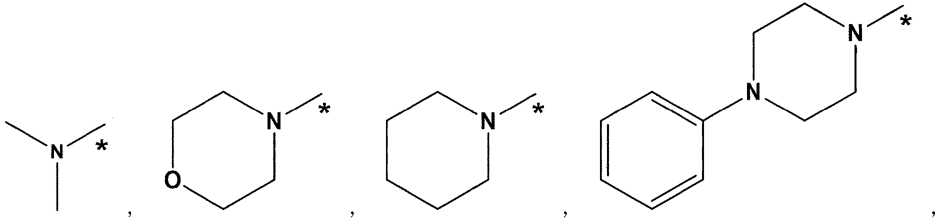 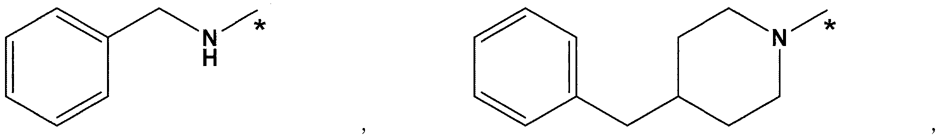 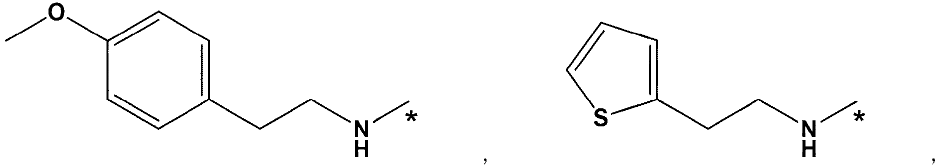 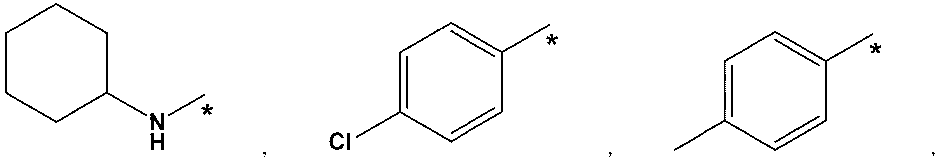 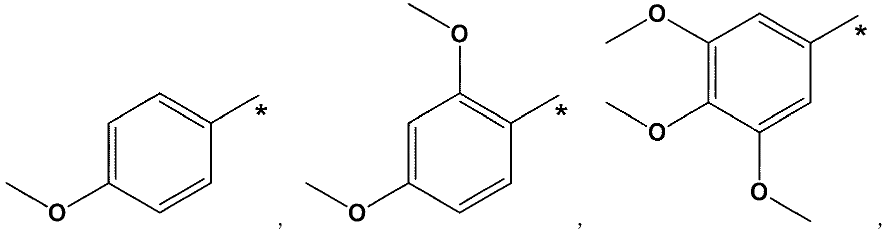 and

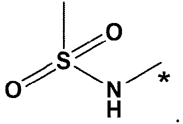

5. The compound or a pharmaceutically acceptable salt thereof according to claim 1, wherein the compound of Chemical Formula 1 is selected from the group consisting of the following compounds:
1) 9-fluoro[2]benzoxepino[3,4-f]-1,3-benzodioxol-11(6H)-one;
2) [2]benzoxepino[3,4-f]-1,3-benzodioxol-11(6H)-one;
3) 8,9-difluoro[2]benzoxepino[3,4-f]-1,3-benzodioxol-1 1(6H)-one;
4) 8-(dimethylamino)-9-fluoro[2]benzoxepino[3,4-f]-1,3-benzodioxol-11(6H)-one;
5) 8-(morpholino)-9-fluoro[2]benzoxepino[3,4-f]-1,3-benzodioxol-11(6H)-one;
6) 8-(piperidinyl)-9-fluoro[2]benzoxepino[3,4-f]-1,3-benzodioxol-11(6H)-one;
7) 8-(phenylpiperazine-1-yl)-9-fluoro[2]benzoxepino[3,4-f]-1,3-benzodioxol-11(6H)-one;
8) 8-(benzylamino)-9-fluoro[2]benzoxepino[3,4-f]-1,3-benzodioxol-11(6H)-one;
9) 9-(4-chlorophenyl)[2]benzoxepino[3,4-f]-1,3-benzodioxol-11(6H)-one;
10) 9-(4-hydroxyphenyl)[2]benzoxepino[3,4-f]-1,3-benzodioxol-11(6H)-one;
11) 9-(3-furanyl)[2]benzoxepino[3,4-f]-1,3-benzodioxol-11(6H)-one;
12) 9-(p-tolyl)[2]benzoxepino[3,4-f]-1,3-benzodioxol-11(6H)-one;
13) 9-(4-methoxyphenyl)[2]benzoxepino[3,4-f]-1,3-benzodioxol-11(6H)-one;
14) 9-(2,4-dimethoxyphenyl)[2]benzoxepino[3,4-f]-1,3-benzodioxol-11(6H)-one;
15) 9-(3,4,5-trimethoxyphenyl)[2]benzoxepino[3,4-f]-1,3-benzodioxol-11(6H)-one;
16) 8-(cyclohexylamino)-9-fluoro[2]benzoxepino[3,4-f]-1,3-benzodioxol-11(6H)-one;
17) 8-(4-benzylpiperidin-1-yl)-9-fluoro[2]benzoxepino[3,4-f]-1,3-benzodioxol-11(6H)-one;
18) 8-((4-methoxyphenethyl)amino)-9-fluoro[2]benzoxepino[3,4-f]-1,3-benzodioxol-11(6H)-one;
19) 8-((2-(thiophen-2-yl)ethyl)amino)-9-fluoro[2]benzoxepino[3,4-f]-1,3-benzodioxol-116H)-one;
20) 7, 8-difluoro[2]benzoxepino[3,4-f]-1,3-benzodioxol-11(6H)-one;
21) 8-(dimethylamino)-7-fluoro[2]benzoxepino[3,4-f]-1,3-benzodioxol-11(6H)-one;
22) 8-(morpholino)-7-fluoro[2]benzoxepino[3,4-f]-1,3-benzodioxol-11(6H)-one;
23) 8-(piperidinyl)-7-fluoro[2]benzoxepino[3,4-f]-1,3-benzodioxol-11(6H)-one;
24) 8-(phenylpiperazine-1-yl)-7-fluoro[2]benzoxepino[3,4-f]-1,3-benzodioxol-11(6H)-one;
25) 8-(benzylamino)-7-fluoro[2]benzoxepino[3,4-f]-1,3-benzodioxol-11(6H)-one;
26) 8-(cyclohexylamino)-7-fluoro[2]benzoxepino[3,4-f]-1,3-benzodioxol-11(6H)-one;
27) 8-(4-benzylpiperidin-1-yl)-7-fluoro[2]benzoxepino[3,4-f]-1,3-benzodioxol-11(6H)-one;
28) 8-((4-methoxyphenethyl)amino)-7-fluoro[2]benzoxepino[3,4-f]-1,3-benzodioxol-11(6H)-one;
29) 8-((2-(thiophen-2-yl)ethyl)amino)-7-fluoro[2]benzoxepino[3,4-f]-1,3-benzodioxol-11(6H)-one;
30) 9-bromo-2,3-dimethoxydibenz[b,e]oxepin-11(6H)-one;
31) 9-(2,4-dimethoxyphenyl)-2,3-dimethoxydibenzo[b,e]oxepin-11(6H)-one;
32) 9-(3, 4-dimethoxyphenyl)-2,3-dimethoxydibenzo[b,e]oxepin-11(6H)-one;
33) 2,3-dimethoxy-9-(3-methoxyphenyl)dibenzo[b,e]oxepin-11(6H)-one;
34) 9-(4-fluorophenyl)-2,3-dimethoxydibenzo[b,e]oxepin-11(6H)-one;
35) 8-(4-chlorophenyl)[2]benzoxepino[3,4-f]-1,3-benzodioxol-11(6H)-one;
36) 8-(p-tolyl)[2]benzoxepino[3,4-f]-1,3-benzodioxol-11(6H)-one;
37) 8-(4-methoxyphenyl)[2]benzoxepino[3,4-f]-1,3-benzodioxol-11(6H)-one;
38) 8-(2,4-dimethoxyphenyl)[2]benzoxepino[3,4-f]-1,3-benzodioxol-11(6H)-one;
39) 8-(3,4,5-trimethoxyphenyl)[2]benzoxepino[3,4-f]-1,3-benzodioxol-11(6H)-one;
40) 2,3-dimethoxy-9-(4-(trifluoromethyl)phenyl)dibenzo[b,e]oxepin-11(6H)-one;
41) N-(9-fluoro-11-oxo-6,11-dihydro-[1,3]dioxolo[4',5': 4,5]benzo[1,2-b]benzo[e]oxepin-8-yl)methanesulfonamide;
42) N-(7-fluoro-11-oxo-6,11-dihydro-[1,3]dioxolo[4',5': 4,5]benzo[1,2-b]benzo[e]oxepin-8-yl)methanesulfonamide;
43) 2,3-dimethoxy-10,11-dihydro-5H-dibenzo[a,d][7]annulen-5-one;
44) 9-bromo-2,3-dimethoxydibenzo[b,e]thiepin-11(6H)-one
45) 2,3-dimethoxy-9-(3,4,5-trimethoxyphenyl)dibenzo[b,e]thiepin-11(6H)-one **(HN2305);**
46) 2,3-dimethoxy-9-(4-methoxyphenyl)dibenzo[b,e]thiepin-11(6H)-one **(HN2306);**
47) 2,3-dimethoxy-9-(p-tolyl)dibenzo[b,e]thiepin-11(6H)-one **(HN2307);**
48) 9-(3-furanyl)-{2,3-dimethoxydibenzo[b,e]thiepin-11(6H)-one **(HN2308);**
49) 9-(4-chlorophenyl)-2,3-dimethoxydibenzo[b,e]thiepin-11(6H)-one **(HN2309);**
50) 10,11-dihydro-5H-benzo[4',5']cyclohepta[1',2':4,5]benzo[1,2-d][1,3]dioxol-5-one **(HN2310);**
51) 2,3-difluoro-7,8-dimethoxy-10,11-dihydro-5H-dibenzo[a,d][7]annulen-5-one **(HN2311);**
52) 8-(cyclopropylamino)-9-fluoro-[1,3]dioxolo[4',5':4,5]benzo[1,2-b]benzo[e]oxepin-11(6H)-one **(HN2312);**
53) 8-(cyclopentylamino)-9-fluoro-[1,3]dioxolo[4',5':4,5]benzo[1,2-b]benzo[e]oxepin-11(6H)-one **(HN2313);**
54) 2-(dimethylamino)-3-fluoro-7,8-dimethoxy-10,11-dihydro-5H-dibenzo[a,d][7]annulen-5-one **(HN2314);**
55) 3-fluoro-7,8-dimethoxy-2-(piperidin-1-yl)-10,11-dihydro-5H-dibenzo[a,d][7]annulen-5-one **(HN2315);**
56) 3-fluoro-7,8-dimethoxy-2-(4-phenylpiperazin-1-yl)-10,11-dihydro-5H-dibenzo[a,d][7]annulen-5-one **(HN2316);**
57) 2-(benzylamino)-3-fluoro-7,8-dimethoxy-10,11-dihydro-5H-dibenzo[a,d][7]annulen-5-one **(HN2317);**
58) 8-(cyclopropylamino)-7-fluoro-[1,3]dioxolo[4',5':4,5]benzo[1,2-b]benzo[e]oxepin-11(6H)-one **(HN2318);**
59) 8-(cyclopentylamino)-7-fluoro-[1,3]dioxolo[4',5':4,5]benzo[1,2-b]benzo[e]oxepin-11(6H)-one **(HN2319);**
60) 2-(cyclopropylamino)-3-fluoro-7,8-dimethoxy-10,11-dihydro-5H-dibenzo[a,d][7]annulen-5-one **(HN2320);**
61) 2-(cyclopentylamino)-3-fluoro-7,8-dimethoxy-10,11-dihydro-5H-dibenzo[a,d][7]annulen-5-one **(HN2321);**
62) 8-((2,4-dichlorophenethyl)amino)-9-fluoro-[1,3]dioxolo[4',5':4,5]benzo[1,2-b]benzo[e]oxepin-11(6H)-one **(HN2322);**
63) 9-fluoro-8-((2-methoxybenzyl)amino)-[1,3]dioxolo[4',5':4,5]benzo[1,2-b]benzo[e]oxepin-11(6H)-one **(HN2323);**
64) 8,9-difluoro-2,3-dimethoxydibenzo[b,e]thiepin-11(6H)-one **(HN2324);**
65) 9-fluoro-8-((3-methoxybenzyl)amino)-[1,3]dioxolo[4',5':4,5]benzo[1,2-b]benzo[e]oxepin-11(6H)-one **(HN2325);**
66) 9-fluoro-8-((4-methoxybenzyl)amino)-[1,3]dioxolo[4',5':4,5]benzo[1,2-b]benzo[e]oxepin-11(6H)-one **(HN2326);**
67) 9-fluoro-8-((3-methoxyphenethyl)amino)-[1,3]dioxolo[4',5':4,5]benzo[1,2-b]benzo[e]oxepin-11(6H)-one **(HN2327);**
68) 8-((2,4-dimethoxybenzyl)amino)-9-fluoro-[1,3]dioxolo[4',5':4,5]benzo[1,2-b]benzo[e]oxepin-11(6H)-one **(HN2328);**
69) 8-((3,4-dimethoxybenzyl)amino)-9-fluoro-[1,3]dioxolo[4',5':4,5]benzo[1,2-b]benzo[e]oxepin-11(6H)-one **(HN2329);** and
70) 8-Bromo[2]benzoxepino[3,4-f]-1,3-benzodioxol-11(6H)-one.

6. The compound or a pharmaceutically acceptable salt thereof according to claim 1, wherein the compound inhibits the activity of mTORC1 (mammalian Target of rapamycin complex 1) or mTORC2 (mammalian Target of rapamycin complex 2).

7. A pharmaceutical composition for prevention or treatment of neurodegenerative diseases, comprising the compound or a pharmaceutically acceptable salt thereof of claim 1 as an active ingredient.

8. The pharmaceutical composition according to claim 7, wherein the neurodegenerative disease is at least one selected from the group consisting of Alzheimer's disease, senile dementia, Lewy body dementia, frontotemporal dementia, mild cognitive impairment, Parkinson's disease, Pick's disease, Huntington's disease, spinocerebellar atrophy, epilepsy, and stroke.

9. The pharmaceutical composition according to claim 7, wherein the composition selectively inhibits mTORC2 activity.
